# EUROPEAN PATENT APPLICATION

(11) **EP 3 569 713 A1**
(43) Date of publication of application: **20.11.2019**
(21) Application number: 18172658.9
(22) Date of filing: 16.05.2018
(51) Int. Cl.: C12P 19/00, C12N 1/21

(54) **USE OF GLYCOSIDASES IN THE PRODUCTION OF OLIGOSACCHARIDES**

(71) Applicant: Jennewein Biotechnologie GmbH, 53619 Rheinbreitbach (DE)
(72) Inventor: The designation of the inventor has not yet been filed

(57) **Abstract**

Disclosed is a method for the production of a desired oligosaccharide using a genetically-engineered microbial host cell, and said genetically-engineered microbial host cell which has been genetically engineered to express a heterologous glycosidase which is able to intracellularly degrade metabolic saccharide byproducts that are generated during the intracellular biosynthesis of the desired oligosaccharide.

## Description

The present invention relates to the production of oligosaccharides by microbial fermentation. More specifically, the present invention concerns the use of glycosidases to improve the production of desired oligosaccharides by microbial fermentation.

### Background

Human milk contains a unique mixture of different oligosaccharides called Human Milk Oligosaccharides (HMOs). More than 150 structurally different oligosaccharides were identified in human milk thus far. With very few exceptions, the HMOs are characterized by a lactose moiety at their reducing end, and many HMOs contain a fucose residue and/or an *N*-acetylneuraminic acid residue at their non-reducing end. Generally, the monosaccharide residues of HMOs are derived from D-glucose, D-galactose, *N-*acetylglucosamine, L-fucose and *N*-acetylneuraminic acid. The importance of HMOs for infant nutrition is directly linked to their unique biological activities including protection of the neonate from pathogens, supporting development of the infant's immune system and cognitive abilities. Therefore, there is a strong interest in preparing HMOs in a commercial scale.

Besides chemical synthesis of individual HMOs, considerable progress has been made in the development of producing HMOs by microbial fermentation using genetically-modified microorganisms which overexpress a heterologous glycosyltransferase. Upon cultivation of such microorganisms in a medium and under conditions permissive for the microorganism to express said heterologous glycosyltransferase, a HMO can be produced by said microorganism and recovered from the culture medium or cell lysate.

However, glycosyltransferases often possess enzymatic side activities such that their overexpression for producing a desired oligosaccharide typically leads to by-products which are undesired. Typically, these by-products are oligosaccharides too, but have to be removed from the preparation of the desired oligosaccharide for the product's commercial use. However, removing such by-products from the desired oligosaccharide is difficult and cumbersome. One approach of removing such by-products involves the use of glycosidases that are either exogenously added to a reaction mixture/cell medium containing desired and undesired oligosaccharides or produced by a genetically engineered microorganism upon induction at a specific point of time at the end of the fermentation process for producing the desired oligosaccharide.

International Publication No. WO 2015/032412 A1 concerns the use of fucose and discloses a method wherein a genetically-modified cell expressing a heterologous fucosyltransferase is cultivated in the presence of lactose to produce and secrete a mixture of 2'-fucosyllactose (2'-FL) and difucosyllactose (DFL) into an extracellular space of the culture medium in high yield. The saccharides are separated and subjected to hydrolysis by an acid or by a fucosidase to produce fucose in high yields.

International Publication No. WO 2104/090261 A1 discloses a method to form a mixture containing at least one of 2'-FL and 3-fucosyllactose (3-FL), wherein DFL is subjected to partial hydrolysis, e.g. enzymatic hydrolysis or acid hydrolysis. In the enzymatic hydrolysis, DFL is exposed to a fucosidase that can release one of the fucose residues from DFL. DFL (10 mM) was incubated with the 1,2-α-L-fucosidase from *Xanthomonas manihotis* at 37 °C in an incubation buffer, and hydrolysis of DFL was followed by HPLC. After 18 hours, DFL was partially hydrolyzed to 3-FL and fucose. No lactose was detected.

European Patent Application No. EP 2 845 905 A1 concerns the production of oligosaccharides and discloses the use of one or more glycosidases in the process for the production and/or purification of an oligosaccharide. The process comprises a) cultivating a host microorganism suitable for the production of a desired oligosaccharide under conditions and in a medium permissive for the production of said desired oligosaccharide, whereby the oligosaccharide and, where applicable, biosynthetic saccharide intermediates and/or side products are produced; b) using a glycosidase in the medium the host microorganism is cultivated in, in order to degrade biosynthetic saccharide intermediates and/or saccharide side products and/or unused saccharide substrates; and c) recovering the desired oligosaccharide. In an embodiment, said glycosidase is endogenously produced in the host microorganism, wherein the glycosidase is a glycosidase that is not naturally occurring in the host microorganism, and wherein the expression of said glycosidase in said host microorganism is inducible such that the expression can be initiated after a sufficient and/or essentially maximum amount of desired oligosaccharide has been produced during cultivation of the host microorganism.

In summary, prior art discloses the use of glycosidases to remove undesired oligosaccharides from a mixture of desired and undesired oligosaccharides by hydrolysis of the undesired oligosaccharides in a reaction mixture / cell medium. However, these approaches comprise biosynthesis of the undesired oligosaccharides by the microorganism including the use of substrates and energy, and these approaches require removal of the degradation products of the undesired oligosaccharides from the desired oligosaccharide.

It was therefore an object of the present invention to provide a method for the production of a desired oligosaccharide by microbial fermentation without concomitant production / accumulation of undesired saccharide by-products, i.e. undesired oligosaccharides, in the cell medium containing the microorganism to be fermented.

The object is solved by providing a genetically-engineered microbial host cell being able to produce a desired oligosaccharide, wherein said microbial host cell expresses a heterologous glycosidase which is able to degrade metabolic by-products intracellularly that are generated during the intracellular biosynthesis of the desired oligosaccharide, thus preventing the formation of a mixture of desired and undesired saccharides in the culture medium. Said degradation products may then be utilized by the microbial host cell's metabolism, for example for the biosynthesis of the desired oligosaccharide.

Table 1 provides a comprehensive overview of desired oligosaccharides and conceivable precursors that are added for and/or undesired saccharide by-products that are generated during the production of the desired oligosaccharide.

**Table 1: Overview of desired oligosaccharides and conceivable precursors that are added for and/or undesired saccharide by-products that are generated during the production of the desired oligosaccharide.**

| **Desired oligosaccharides** | **Conceivable precursors added to and/or by-products generated during production of the desired oligosaccharides** |
|---|---|
| **fucosylated trisaccharides:** | L-fucose |
| | glucose |
| | galactose |
| 2'-fucosyllactose (2'-FL) or 3-fucosyllactose (3-FL) | lactose |
| | fucosylated galactose |
| | fucosylated glucose |
| | 3-fucosyllactose |
| | 2'-fucosyllactose |
| | difucosyllactose |
| **sialylated trisaccharides:** | *N*-acetylglucosamine |
| | *N*-acetylmannosamine |
| 3'-sialyllactose (3'-SL) | *N*-acetylneuraminic acid |
| or | glucose |
| 6'-sialyllactose (6'-SL) | galactose |
| | lactose |
| | sialylated galactose |
| | sialylated glucose |
| | sialylated *N*-acetylglucosamine |
| | sialylated *N*-acetylmannosamine |
| | 6'-sialyllactose |
| | 3'-sialyllactose |
| | 3-sialyllactose |
| | 6-sialyllactose |
| | disialyllactose |
| | KDO-lactose |
| ***N*-acetylglucosaminylated trisaccharides:** | glucose |
| | galactose |
| | lactose |
| lacto-*N*-triose II (LNT-II) | *N*-acetylglucosaminylated galactose |
| | galactosylated lactose |
| | glucosylated lactose |
| **galactosylated** | glucose |
| **tetrasaccharides:** | galactose |
| | *N*-acetylglucosamine |
| lacto-*N*-tetraose (LNT) | lactose |
| or | glucosylated galactose |
| lacto-*N*-*neo*tetraose (LN*n*T) | galactosylated galactose |
| | *N*-acetylglucosaminylated galactose |
| | galactosylated lactose |
| | glucosylated lactose |
| | lacto-*N*-triose II |
| | lacto-*N*-*neo*tetraose |
| | lacto-*N*-tetraose |
| | galactosylated lacto-*N*-tetraose |
| | glucosylated lacto-*N*-tetraose |
| | *N*-acetylglucosaminylated lacto-*N*-tetraose |
| | galactosylated lacto-*N*-*neo*tetraose |
| | glucosylated lacto-*N*-*neo*tetraose |
| | *N*-acetylglucosaminylated lacto-*N*-*neo*tetraose |
| | *para*-lacto-*N*-hexaose |
| | *para*-lacto-*N*-*neo*hexaose |
| **di-fucosylated tetrasaccharides:** | L-fucose |
| | glucose |
| | galactose |
| 2'3-difucosyllactose (DFL) | fucosylated galactose |
| | fucosylated glucose |
| | lactose |
| | 3-fucosyllactose |
| | 2'-fucosyllactose |
| **fucosylated** | L-fucose |
| **pentasaccharides:** | glucose |
| | galactose |
| lacto-*N*-fucopentaose I | *N*-acetylglucosamine |
| (LNFP-I) | lactose |
| or | fucosylated galactose |
| lacto-*N*-fucopentaose Ii | fucosylated glucose |
| (LNFP-II) | fucosylated *N*-acetylglucosamine |
| or | glucosylated galactose |
| lacto-*N*-fucopentaose III | galactosylated galactose |
| (LNFP-III) | *N*-acetylglucosaminylated galactose |
| or | galactosylated lactose |
| lacto-*N*-fucopentaose V | glucosylated lactose |
| (LNFP-V) | lacto-*N*-triose II |
| or | 3-fucosyllactose |
| lacto-*N*-fucopentaose VI | 2'-fucosyllactose |
| (LNFP-VI) | fucosylated lacto-*N*-triose II |
| or | difucosyllactose |
| lacto-*N*-*neo*fucopentaose | lacto-*N*-*neo*tetraose |
| (LNnFP) | lacto-*N*-tetraose |
| | galactosylated lacto-*N*-tetraose |
| | glucosylated lacto-*N*-tetraose |
| | *N*-acetylglucosaminylated lacto-*N*-tetraose |
| | galactosylated lacto-*N*-*neo*tetraose |
| | glucosylated lacto-*N*-*neo*tetraose |
| | *N*-acetylglucosaminylated lacto-*N*-*neo*tetraose |
| | *para*-lacto-*N*-hexaose |
| | *para*-lacto-*N*-*neo*hexaose |
| | lacto-*N*-fucopentaose I |
| | lacto-*N*-fucopentaose II |
| | lacto-*N*-fucopentaose IIII |
| | lacto-*N*-fucopentaose V |
| | lacto-*N*-fucopentaose VI |
| | lacto-*N*-*neo*fucopentaose |
| | mono-fucosylation of LNT or LN*n*T not leading to LNFP-I/-II/-III/-V/-VI or LN*n*FP di-fucosylation of LNT or LN*n*T |
| **sialylated** | *N*-acetylglucosamine |
| **pentasaccharides:** | *N*-acetylmannosamine |
| | *N*-acetylneuraminic acid |
| sialyllacto-*N*-tetraose a | glucose |
| (LST-a) | galactose |
| or | lactose |
| sialyllacto-*N*-tetraose b | sialylated galactose |
| (LST-b) | sialylated glucose |
| or | sialylated *N*-acetylglucosamine |
| sialyllacto-*N*-tetraose c | sialylated *N*-acetylmannosamine |
| (LST-c) | 6'-sialyllactose |
| | 3'-sialyllactose |
| | 3-sialyllactose |
| | 6-sialyllactose |
| | disialyllactose |
| | KDO-lactose |
| | lacto-*N*-triose II |
| | galactosylated lactose |
| | glucosylated lactose |
| | sialylated lacto-*N*-triose II |
| | lacto-*N*-tetraose |
| | lacto-*N*-*neo*tetraose |
| | KDO-lacto-*N*-tetraose |
| | KDO-lacto-*N*-*neo*tetraose |
| | galactosylated lacto-*N*-tetraose |
| | glucosylated lacto-*N*-tetraose |
| | *N*-acetylglucosaminylated lacto-*N*-tetraose |
| | galactosylated lacto-*N*-*neo*tetraose |
| | glucosylated lacto-*N*-*neo*tetraose |
| | *N*-acetylglucosaminylated lacto-*N*-*neo*tetraose |
| | *para*-lacto-*N*-hexaose |
| | *para*-lacto-*N*-*neo*hexaose |
| | sialyllacto-*N*-tetraose a |
| | sialyllacto-*N*-tetraose b |
| | sialyllacto-*N*-tetraose c |
| | di-sialylated lacto-*N*-tetraose |
| | di-sialylated lacto-*N*-*neo*tetraose |
| | mono-sialylation of LNT or LNnT not leading to LST -a/-b/-c |
| **di-fucosylated hexasaccharides:** | L-fucose |
| | glucose |
| | galactose |
| lacto-*N*-difucohexaose I (LNDFH-I) | *N*-acetylglucosamine |
| | lactose |
| or | fucosylated galactose |
| lacto-*N*-difucohexaose II | fucosylated glucose |
| (LNDFH-II) | fucosylated *N*-acetylglucosamine |
| | glucosylated galactose |
| | galactosylated galactose |
| | *N*-acetylglucosaminylated galactose |
| | galactosylated lactose |
| | glucosylated lactose |
| | lacto-*N*-triose II |
| | 3-fucosyllactose |
| | 2'-fucosyllactose |
| | fucosylated lacto-*N*-triose II |
| | difucosyllactose |
| | lacto-*N*-tetraose |
| | galactosylated lacto-*N*-tetraose |
| | glucosylated lacto-*N*-tetraose |
| | *N*-acetylglucosaminylated lacto-*N*-tetraose |
| | *para*-lacto-*N*-hexaose |
| | lacto-*N*-fucopentaose I |
| | lacto-*N*-fucopentaose II |
| | lacto-*N*-fucopentaose V |
| | di-fucosylation of LNT not leading to LNDFH-I/-II |
| **sialylated** | *N*-acetylglucosamine |
| **hexasaccharides:** | *N*-acetylmannosamine |
| | *N*-acetylneuraminic acid |
| disialyllacto-*N*-tetraose | glucose |
| (DSLNT) | galactose |
| | lactose |
| | sialylated galactose |
| | sialylated glucose |
| | sialylated *N*-acetylglucosamine |
| | sialylated *N*-acetylmannosamine |
| | 6'-sialyllactose |
| | 3'-sialyllactose |
| | 3-sialyllactose |
| | 6-sialyllactose |
| | disialyllactose |
| | KDO-lactose |
| | lacto-*N*-triose II |
| | galactosylated lactose |
| | glucosylated lactose |
| | sialylated lacto-*N*-triose II |
| | lacto-*N*-tetraose |
| | KDO-lacto-*N*-tetraose |
| | galactosylated lacto-*N*-tetraose |
| | glucosylated lacto-*N*-tetraose |
| | *N*-acetylglucosaminylated lacto-*N*-tetraose |
| | *para*-lacto-*N*-hexaose |
| | sialyllacto-*N*-tetraose a |
| | sialyllacto-*N*-tetraose b |
| | mono-sialylation of LNT or LNnT not leading to LST -a/-b/-c |
| | di-sialylation of lacto-*N*-tetraose not leading to DSLNT |

### Summary

In a first aspect, disclosed is a method for the production of a desired oligosaccharide using a genetically-engineered microbial host cell that is able to produce the desired oligosaccharide, said microbial host cell expresses a heterologous glycosidase which is able to intracellularly degrade metabolic saccharide by-products that are generated during the intracellular biosynthesis of the desired oligosaccharide.

In a second aspect, disclosed is a genetically-engineered microbial host cell for the production of a desired oligosaccharide, wherein said microbial host cell is able to produce the desired oligosaccharide, and wherein said microbial host cell has been genetically-engineered to express a heterologous glycosidase which is able to intracellularly degrade metabolic saccharide by-products that are generated during the intracellular biosynthesis of the desired oligosaccharide.

In a third aspect, disclosed is the use of the genetically-engineered microbial host cell according to the second aspect for the production of a desired oligosaccharide.

In a fourth aspect, disclosed are oligosaccharides, i.e. desired oligosaccharides, that are produced by the method according to the first aspect and/or by using the genetically-engineered microbial host cell according to the second aspect.

In a fifth aspect, disclosed is the use of the desired oligosaccharides according to the fourth aspect for the production of a nutritional composition.

In a sixth aspect, disclosed are nutritional compositions containing a desired oligosaccharide according to the fourth aspect.

### Brief description of the drawings

- FIG.: 1 shows a schematic representation of an embodiment of a microbial host cell expressing a heterologous glycosidase (e.g. an alpha-1,3-fucosidase) that is able to degrade metabolic saccharide by-products (e.g. 3-fucosyllactose and 2'3-difucosyllactose) that are generated during the intracellular biosynthesis of the desired oligosaccharide (2'-fucosyllactose), and wherein the microbial host cell is able to recycle the degradation products (e.g. fucose and lactose) resulting from the enzymatic activity of said glycosidase for the production of the desired oligosaccharide.

### Detailed description

According to the first aspect, provided is a method for the production of a desired oligosaccharide using a genetically-engineered microbial host cell, the method comprises the steps of:
(i) providing a genetically-engineered microbial host cell that is able to produce the desired oligosaccharide, wherein the microbial host cell has been genetically engineered to express a heterologous glycosidase which is able to intracellularly degrade metabolic saccharide by-products that are generated during the intracellular biosynthesis of the desired oligosaccharide, and wherein the microbial host cell is able to recycle degradation products resulting from the enzymatic activity of said glycosidase;
(ii) cultivating the genetically-engineered microbial host cell under conditions and in a medium permissive for the production of the desired oligosaccharide, thereby producing the desired oligosaccharide; and
(iii) optionally, recovering the desired oligosaccharide.

The term "desired" as used herein with respect to oligosaccharides refers to an oligosaccharide that is intended to be produced by the microbial host cell. The term "desired" is used to distinguish the oligosaccharide to be produced on purpose from other oligosaccharides the microbial host cell may produce. Said other oligosaccharides are considered to be "undesired", regardless of whether or not these other oligosaccharides have a biological function, are involved in the biosynthesis of other cell compounds such as glycolipids, glycoproteins or polysaccharides, or are metabolic saccharide products that are generated during the intracellular biosynthesis of the desired oligosaccharide either due to subsidiary (undesired) enzymatic activities of one or more of the enzymes involved in the biosynthesis of the desired oligosaccharide, or due to the enzymatic activity of one or more enzymes which are not directly involved in the biosynthesis of the desired oligosaccharide but use an oligosaccharide as substrate which is generated as an intermediate in the metabolic pathway leading to the desired oligosaccharide.

The term "oligosaccharide" as used herein refers to a saccharide molecule consiting of three to twenty monosaccharide residues, wherein each of said monosaccharide residues in bound to at least one other of said monosaccharide units by a glycosidic linkage. The oligosaccharide may be a linear chain of monosaccharide residues or a branched chain of monosaccharide residues.

In an additional and/or alternative embodiment, the desired oligosaccharide is a human milk oligosaccharide (HMO).

In an additional and/or alternative embodiment, the desired oligosaccharide is a HMO selected from the group consisting of 2'-fucosyllactose (2'-FL), 3-fucosyllactose (3-FL), 2'3-difucosyllactose (DFL), lacto-*N*-triose II, lacto-*N*-tetraose (LNT), lacto-*N*-neotetraose (LNnT), lacto-*N*-fucopentaose I (LNFP-I), lacto-*N*-neofucopentaose I (LN*n*FP-I), lacto-*N*-fucopentaose II (LNFP-II), lacto-*N*-fucopentaose III (LNFP-III), lacto-*N*-fucopentaose V (LNFP-V), lacto-*N*-*neo*fucopentaose V (LNnFP-V), lacto-*N*-difucohexaose I, lacto-*N*-difucosylhexaose II, para-Lacto-*N*-fucosylhexaose, fucosyl-lacto-*N*-sialylpentaose b, fucosyl-lacto-*N*-sialylpentaose c, fucosyl-lacto-*N*-sialylpentaose c, disialyl-lacto-*N*-fucopentaose, 3-fucosyl-3'-sialyllactose, 3-fucosyl-6'-sialyllactose, lacto-*N*-*neo*difucohexaose I, 3'-sialyllactose (3-SL), 6'-sialyllactose (6-SL), sialyllacto-*N*-tetraose a (LST-a), sialyllacto-*N*-tetraose b (LST-b), sialyllacto-*N*-tetraose c (LST-c), and disialyllacto-*N*-tetraose.

The method comprises providing a genetically-engineered microbial host cell that is is able to produce the desired oligosaccharide.

The term "genetically-engineered" as used herein refers to the modification of the cell's genetic make-up using molecular biological methods. The modification of the cell's genetic make-up may include the transfer of genes within and/or across species boundaries, inserting, deleting, substituting and/or modifying nucleotides, triplets, genes, open reading frames, promoters, enhancers, terminators and other nucleotide sequences mediating and/or controlling gene expression. The modification of the cell's genetic make-up aims to generate a genetically modified organism possessing particular, desired properties. Genetically-engineered microbial host cell can contain one or more genes that are not present in the native (not genetically engineered) form of the cell. Techniques for introducing exogenous nucleic acid molecules and/or inserting exogenous nucleic acid molecules (recombinant, heterologous) into a cell's hereditary information for inserting, deleting or altering the nucleotide sequence of a cell's genetic information are known to the skilled artisan. Genetically-engineered cells can contain one or more genes that are present in the native form of the cell, wherein said genes are modified and re-introduced into the cell by artificial means. The term "genetically-engineered" also encompass cells that contain a nucleic acid molecule being endogenous to the cell, and that has been modified without removing the nucleic acid molecule from the cell. Such modifications include those obtained by gene replacement, site-specific mutations, and related techniques including those commonly referred to as "gene editing".

The genetically-engineered microbial host cell may be a prokaryotic cell or a eukaryotic cell. Suitable microbial host cells include yeast cells, bacterial cells, archaebacterial cells and fungal cells.

In an additional and/or alternative embodiment, the prokaryotic cell is a bacterial cell, preferably a bacterial cell selected from bacteria of a genus selected from the group consisting of *Bacillus, Bifidobacterium, Clostridium, Corynebacterium, Enterococcus, Lactobacillus, Lactococcus, Micrococcus, Micromonospora, Pseudomonas, Rhodococcus* and *Sporolactobacillus.* Suitable bacterial species are *Bacillus subtilis, B. licheniformis, B. coagulans, B. thermophilus, B. laterosporus, B. mega-terium, B. mycoides, B. pumilus, B. lentus, B. cereus, B. circulans, Bifidobacterium longum, B. infantis, B. bifidum, Citrobacter freundii, Clostridium cellulolyticum, C. Ijungdahlii, C. autoethanogenum, C. acetobutylicum, Corynebacterium glutamicum, Enterococcus faecium, E. thermophiles, Escherichia coli, Erwinia herbicola (Pantoea agglomerans), Lactobacillus acidophilus, L. salivarius, L. plantarum, L. helveticus, L. delbrueckii, L. rhamnosus, L. bulgaricus, L. crispatus, L. gasseri, L. casei, L. reuteri, L. jensenii, L. lactis, Pantoea citrea, Pectobacterium carotovorum, Proprionibacterium freudenreichii, Pseudomonas fluorescens, P. aeruginosa, Streptococcus thermophiles* and *Xanthomonas campestris.*

In an additional and/or alternative embodiment, the eukaryotic cell is a yeast cell, preferably a yeast cell selected from the group consisting of *Saccharomyces sp.,* in particular *Saccharomyces cerevisiae, Saccharomycopsis sp., Pichia sp.,* in particular *Pichia pastoris, Hansenula sp., Kluyveromyces sp., Yarrowia sp., Rhodotorula sp.,* and *Schizosaccharomyces sp.*

The genetically-engineered microbial host cell is able to produce the desired oligosaccharide. The term "able to produce" as used herein refers to the capability of the genetically-engineered microbial host cell to produce the desired oligosaccharide provided that the microbial host cell is cultivated under conditions and in a medium that are permissive for the microbial host cell to produce the desired oligosaccharide. Thus, the medium has to comprise a pH value in a defined range, a composition of ions and nutrients as well as of compounds required for maintaining viability and metabolic activity of the microbial host cell. If essential for the production of the desired oligosaccharide, the medium also has to contain sufficient amounts of any precursor required for biosynthesis of the desired oligosaccharide by the microbial host cell. Likewise, the conditions (e.g. temperature, pH, oxygen supply, agitation, supply of nutrients, etc.) for culturing the microbial host cell for producing the desired oligosaccharide have to be maintained such that the microbial host cell can be or remain metabolically active to produce the desired oligosaccharide.

In an additional and/or alternative embodiment, the genetically-engineered microbial host cell being able to produce a desired oligosaccharide is a microbial host cell that has been genetically engineered to be able to produce the desired oligosaccharide.

In an additional and/or alternative embodiment, the genetically-engineered microbial host cell has been genetically engineered to express a heterologous glycosyltransferase. The heterologous glycosidase is expressed in the genetically-engineered microbial host cell during fermentation, i.e. during the production or biosynthesis of the desired oligosaccharide. In an additional and/or alternative embodiment, expression of the heterologous glycosidase is constitutive in the genetically engineered microbial host.

The term "heterologous" as used herein refers to a nucleotide sequence, nucleic acid molecule or polypeptide that is foreign to a cell or organism, i.e. to a nucleotide sequence, nucleic acid molecule or polypeptide that does not naturally occur in said cell or organism. A "heterologous sequence" or a "heterologous nucleic acid" or "heterologous polypeptide", as used herein, is one that originates from a source foreign to the particular host cell (e.g. from a different species), or, if from the same source, is modified from its original form. Thus, a heterologous nucleic acid operably linked to a promoter is from a source different from that from which the promoter was derived, or, if from the same source, is modified from its original form. The heterologous sequence may be stably introduced, e.g. by transfection, transformation, conjugation or transduction, into the genome of the host microbial host cell, thus representing a genetically modified host cell. Techniques may be applied which will depend on the host cell the sequence is to be introduced. Various techniques are known to a person skilled in the art and are, e.g., disclosed in Sambrook et al., Molecular Cloning: A Laboratory Manual, 2nd Ed., Cold Spring Harbor Laboratory Press, Cold Spring Harbor, N.Y. (1989). Accordingly, a "heterologous polypeptide" is a polypeptide that does not naturally occur in the wild-type cell the genetically engineered cell is derived from, and a "heterologous glycosyltransferase" is a glycosyltransferase that does not naturally occur in the wild-type cell the genetically engineered cell is derived from.

In an additional and/or alternative embodiment, the heterologous glycosyltransferase is selected from the group consisting of fucosyltransferases, preferably α-1,2-fucosyltransferases and α-1,3-fucosyltransferases, glucosyltransferases, galactosyltransferases, preferably β-1,3-galactosyltransferases and β-1,4-galactosyltransferases, sialyltransferases, preferably α-2,3-sialyltransferases and α-2,6-sialyltransferases, and *N*-acetylglucosaminyltransferases.

Fucosyltransferases catalyze the transfer of fucose residues from the donor guanosine-diphosphate activated L-fucose (GDP-fucose) to several acceptor molecules. Fucosyltransferases are expressed in animals, plants, fungi and bacteria, and they are categorized according to the fucose linkage at the acceptor substrate. Therefore, α-1,2-, α-1,3/4- and α-1,6-fucosyltransferases are distinguished from each other. Suitable fucosyltransferases for heterologous expression in a genetically-engineered microbial host cell are disclosed -for example - in European patent application No. 17 180 176.

Sialyltransferases catalyze the transfer of *N*-acetylneuraminic acid (Neu5Ac) residues from the donor CMP-Neu5Ac to acceptor molecules. Sialyltransferases were found to be expressed in animals, plants, fungi and bacteria. Sialyltransferases are categorized according to the linkage that is formed between Neu5Ac and the acceptor molecule. Hence, α-2,3-, α-2,6- and α-2,8-sialyltransferases are distinguished from each other. Suitable sialyltransferases for heterologous expression in a genetically-engineered microbial host cell are disclosed - for example - in European patent application No. 17 183 391.

Galactosyltransferases catalyze the transfer of a galactose residue from the donor UDP-galactose to acceptor substrates. Galactosyltransferases are distinguished based on the linkage between the galactose and the acceptor molecule that is formed. Hence, β-1,3- and β-1,4-galactosyltransferases are distinguished from each other. A suitable β-1,3-galactosyltransferse for heterologous expression in a genetically-engineered microbial host cell is encoded by the *Salmonella enterica wbd*O gene. A suitable β-1,4-galctosyltransferse for heterologous expression in a genetically-engineered microbial host cell is encoded by the *lex1* gene of *Aggregatibacter aphrophilus.*

The genetically-engineered microbial host cell has been genetically engineered to express a heterologous glycosidase which is able to intracellularly degrade metabolic saccharide by-products that are generated during the intracellular biosynthesis of the desired oligosaccharide. Suitable glycosidases are glycosidases which are specific with respect to the glycosidic linkage that is hydrolyzed by the enzymatic activity and/or with respect to the substrate that is hydrolyzed by the glycosidase. Due to said specificity, the glycosidase hydrolyzes the undesired by-products, but not the desired oligosaccharide to be produced. In an additional and/or alternative embodiment, the glycosidase does not hydrolyze one or more of the precursors that are internalized or synthesized by the microbial host cell for producing the desired oligosaccharide. Preferably, the glycosidase is an exoglycosidase.

Exoglycosidases are glycoside hydrolase enzymes which break the glycosidic bonds at the terminal residue of an oligosaccharide structure.

In an additional and/or alternative embodiment, the heterologous glycosidase is selected from the group consisting of fucosidases including α-1,2-fucosidases and α-1,3-fucosidases, sialidases such as α-2,3-sialidases, α-2,6-sialidases, α-2,8-sialidases, galactosidases such as β-1,3-galactosidases, β-1,4-galactosidases and β-1,6-galactosidases, β-*N*-acetylhexosaminidases and glucosidases such as β-1,3-glucosidases.

A suitable fucosidase is an α-1,2-fucosidase. The α-1,2-fucosidase is a highly specific exoglycosidase that catalyzes the hydrolysis of linear αlpha-1,2-linked L-fucopyranosyl residues from oligosaccharides. A preferred α-1,2-fucosidase is AfcA of *Bifidobacterium bifidum* (SEQ ID NO: 2).

In an additional and/or alternative embodiment, a genetically-engineered microbial host cell is provided that is able to produce 3-FL, wherein said genetically-engineered microbial host cell expresses an α-1,2-fucosidase. To be able to produce 3-FL, the genetically-engineered microbial host cell expresses an alpha-1,3-fucosyltransferase. Said alpha-1,3-fucosyltransferase is able to transfer a fucose residue from GDP-fucose to the glucose moiety of lactose as an acceptor substrate, thereby synthesizing 3-FL as desired oligosaccharide. 2'-FL and 2'3-DFL are undesired saccharide by-products in the production of 3-FL.

By expressing a heterologous α-1,2-fucosidase in the genetically-engineered microbial host cell that is able to produced 3-FL, production of the by-products 2'-FL and 2'3-DFL can be abolished or at least diminished in that these by-products are hydrolyzed within the genetically-engineered microbial host cell by the heterologous α-1,2-fucosidase. The resulting degradation products are fucose and lactose. Both, fucose and lactose, can be utilized by the genetically-engineered microbial host cell for the production of the desired 3-FL.

In an additional and/or alternative embodiment, the genetically-engineered microbial host cell has been genetically engineered to express the α-1,2-fucosidase. In an additional and/or alternative embodiment the genetically-engineered microbial host cell has been genetically engineered to contain a nucleic acid molecule comprising a nucleotide sequence encoding the α-1,2-fucosidase for its expression. Preferably, the nucleotide sequence encoding the α-1,2-fucosidase is a nucleotide sequence selected from the group consisting of
- a nucleotide sequence as represented by SEQ ID NO: 1;
- nucleotide sequences that are complementary to a nucleotide sequence which hybridizes to the nucleotide sequence as represented by SEQ ID NO: 1 under stringent conditions;
- nucleotide sequences having a sequence identity of at least 70%, 75%, 80%, 85%, 90%, 95%, 96%, 97%, 98% or 99 % to the nucleotide sequence as represented by SEQ ID NO: 1;
- nucleotide sequences encoding a polypeptide having an amino acid sequence as represented by SEQ ID NO: 2; and
- nucleotide sequences encoding a functional variant of the polypeptide sequences as represented by SEQ ID NO: 2, wherein the amino acid sequence of the functional variant has a sequence identity of at least 70%, 75%, 80%, 85%, 90%, 95%, 96%, 97%, 98% or 99% to the amino acid sequence as represented by SEQ ID NO: 2.

The term "hybridize" or "hybridizing" as used herein means hybridizing under conventional conditions, as described in Sambrook et al. (1989) "Molecular Cloning, A Laboratory Manual" (Cold Spring Harbor Laboratory Press, New York), preferably under stringent conditions. Stringent hybridization conditions are for example: hybridizing in 4 × SSC at 65 °C and subsequent multiple washing in 0.1 x SSC at 65 °C for a total of about 1 hour. Less stringent hybridization conditions are for example: hybridizing in 4 × SSC at 37 °C. and subsequent multiple washing in 1 x SSC at room temperature (about 21 °C). "Stringent hybridization conditions" can also mean: hybridizing at 68 °C in 0.25 M sodium phosphate, pH 7.2, 7% SDS, 1 mM EDTA and 1% BSA for 16 hours and followed by two washes with 2 x SSC and 0 1% SDS at 68 °C.

For expression of the nucleotide sequence encoding the α-1,2-fucosidase or functional variant thereof, said nucleotide sequence is operably linked to expression control sequences which mediate expression of the nucleotide sequence encoding the α-1,2-fucosidase or functional variant thereof in the genetically-engineered microbial host cell.

"Expression control sequences" are regulatory nucleotide sequences which are not part of the protein-encoding nucleotide sequence, but mediate the expression of protein-encoding nucleotide sequence. Regulatory element nucleotide sequences include promoters, cis regulatory elements, enhancers, introns and terminators. Depending on the type of regulatory element it is present on the nucleic acid molecule before the protein-coding nucleotide sequence (i.e. 3' of) or behind the protein-encoding nucleotide sequence (i.e. 5' of). The regulatory elements are functional in the microbial host cell.

The term "operably linked" means that a regulatory element is connected in such a way with the protein-encoding nucleotide sequence, i.e. is positioned in such a way relative to the protein-coding nucleotide sequence on, for example, a nucleic acid molecule that an expression of the protein-encoding nucleotide sequence under the control of the regulatory element can take place in a living cell.

For the purposes of the present invention, a "promoter" is an expression of a gene regulating nucleotide sequence, which is usually at the 5 'end of a gene and via interaction with specific DNA-binding proteins mediates the initiation of transcription by RNA polymerase.

Furthermore, suitable promoters include synthetic promoters. These are promotors that have been created by molecular biology techniques that are not found in nature in this configuration. A synthetic promoter is a minimalistic promoter containing only one or more selected, defined cis-elements in addition to a minimal promoter. These cis-elements are binding sites for DNA-binding proteins such as transcription factors and are isolated from natural promoters, derived from previously isolated cis-elements, or produced technically by random recombination techniques and selected by appropriate methods; as compared with a natural promoter, due to its less complex construction a synthetic promoter is activated only by a few exogenous and endogenous factors and is therefore more specifically regulated.

The "minimal promoter" or "core"-promoter is a nucleotide sequence which contains the binding sites for the basal transcription factor complex and allows the accurate initiation of transcription by RNA polymerase II. Characteristic sequence motifs of the minimal promoter are the TATA box, the initiator element (Inr), the "TFBII recognition element" (BRE) and the "downstream core promoter element" (OPE). In the minimal promoter these elements can occur individually or in combination. The minimal promoter is or its sequence motifs are available, for example, from a bacterial, fungal or viral gene.

"Cis-elements" are nucleotide sequences that are located on the same nucleic acid molecule as the protein-encoding nucleotide sequence to be expressed. Cis-elements do not have to encode RNA or protein and in the direction of transcription can be located before or after the protein-encoding nucleotide sequence to be expressed. Cis-elements upstream before a protein-encoding nucleotide sequence to be expressed often provide necessary binding motifs in particular for transcription factors which engage as *trans-acting elements* (of Lat. *trans,* 'beyond'), on the molecular level, from the other side in the regulation of the transcription of this gene. If, in addition, cis elements lead to an inhibition of the transcription, they are called silencers. Cis-elements that lead to an enhancement of the transcription are called enhancers. The totality of the cis/trans activities in the promoter determines the intensity with which the RNA polymerase carries out transcription.

Furthermore, a promoter may be a chimeric promoter and/or a promoter that has been modified by cis elements. The modification of a promoter can also mean the additional incorporation of a cis-element in the promoter which for example already has a cis-element naturally. Further, the modification also includes a multimerization of a cis element, in particular a multimerization of a naturally existing cis-element.

Compared with the native version such modified promoter may have altered properties with respect to specificity, expression level or background activity, for example.

Terminators are nucleotide sequences on the DNA, which usually mark the end of a gene and lead to the termination of transcription.

Another suitable fucosidase is a α-1,3-fucosidase. The α-1,3-fucosidase is a highly specific glycosidase that catalyzes the hydrolysis of α-1,3-linked L-fucopyranosyl residues from oligosaccharides. A preferred α-1,3-fucosidase is AfcB from *Bifidobacterium bifidum* (SEQ ID NO: 4).

In an additional and/or alternative embodiment, a genetically-engineered microbial host cell is provided that is able to produce 2'-FL, wherein said genetically-engineered microbial host organism expresses an α-1,3-fucosidase. To be able to produce 2'-FL, the genetically-engineered microbial host cell expresses an α-1,2-fucosyltranferase. Said alpha-1,2-fucosyltransferase is able to transfer a fucose residue from GDP-fucose to the galactose moiety of lactose as an acceptor substrate, thereby synthesizing 2'-FL as desired oligosaccharide. 3-FL and 2'3-DFL are undesired saccharide by-products in the production of 2'-FL.

By expressing a heterologous α-1,3-fucosidase in the genetically-engineered microbial host cell that is able to produce 2'-FL, production of the by-products 3-FL and 2'3-DFL can be abolished or at least diminished in that these by-products are hydrolyzed within the genetically-engineered microbial host cell by the heterologous α-1,3-fucosidase. The resulting degradation products are fucose and lactose. Both, fucose and lactose, can be utilized by the genetically-engineered microbial host organism for the production of the desired 2'-FL.

In an additional and/or alternative embodiment, the genetically-engineered microbial host cell has been genetically engineered to express the α-1,3-fucosidase. In an additional and/or alternative embodiment, the genetically-engineered microbial host cell has been genetically engineered to contain a nucleic acid molecule comprising a nucleotide sequence encoding the α-1,3-fucosidase for its expression. Preferably, the nucleotide sequence encoding the α-1,3-fucosidase is a nucleotide sequence selected from the group consisting of
- a nucleotide sequence as represented by SEQ ID NO: 3;
- nucleotide sequences that are complementary to a nucleotide sequence which hybridizes to the nucleotide sequence as represented by SEQ ID NO: 3 under stringent conditions;
- nucleotide sequences having a sequence identity of at least 70%, 75%, 80%, 85%, 90%, 95%, 96%, 97%, 98% or 99 % to the nucleotide sequence as represented by SEQ ID NO: 3;
- nucleotide sequences encoding a polypeptide having an amino acid sequence as represented by SEQ ID NO: 4; and
- nucleotide sequences encoding a functional variant of the polypeptide sequences as represented by SEQ ID NO: 4, wherein the amino acid sequence of the functional variant has a sequence identity of at least 70%, 75%, 80%, 85%, 90%, 95%, 96%, 97%, 98% or 99% to the amino acid sequence as represented by SEQ ID NO: 4.

For expression of the nucleotide sequence encoding the α-1,3-fucosidase or functional variant thereof, said nucleotide sequence is operably linked to expression control sequences which mediate expression of the nucleotide sequence encoding the α-1,3-fucosidase or functional variant thereof in the genetically-engineered microbial host cell.

In an additional and/or alternative embodiment, a genetically engineered-microbial host cell is provided that is able to produce LNFP-I, wherein said genetically-engineered microbial host cell expresses an α-1,3-fucosidase. To be able to produce LNFP-I, the genetically-engineered microbial host cell expresses a β-1,3-*N-*acetylglucosaminylransferase, a β-1,3-galactosyltransferase and an α-1,2-fucosyltransferase. Said β-1,3-*N*-acetylglucosaminylransferase is able to transfer a GlcNAc residue from UDP-GlcNAc to the galactose moiety of lactose, thereby synthesizing lacto-N-triose-II (LNT-II). Said β-1,3-galactosyltransferase is able to transfer a galactose residue from UDP-galactose to the GlcNAc moiety of LNT-II, thereby synthesizing lacto-*N*-tetraose (LNT). Said α-1,2-fucosyltransferase is able to transfer a fucose residue from GDP-fucose to the terminal galactose moiety of LNT, thereby synthesizing LNFP-I. 3-FL and 2'3-DFL would be undesired by-products in the production of LNFP-I. By expressing an α-1,3-fucosidase in the genetically-engineered microbial host cell being able to produce LNFP-I, production of the by-products 3-FL and 2'3-DFL can be abolished or at least diminished in that these by-products are hydrolyzed by the α-1,3-fucosidase within the genetically-engineered microbial host cell. The resulting degradation products are fucose, lactose, and 2'-FL. Fucose and lactose can be utilized by the genetically-engineered microbial host organism for the production of the desired LNFP-I.

In an additional and/or alternative embodiment, the genetically-engineered microbial host cell has been genetically engineered to express the α-1,3-fucosidase. In an additional and/or alternative embodiment, the genetically-engineered microbial host cell has been genetically engineered to contain a nucleic acid molecule comprising a nucleotide sequence encoding the α-1,3-fucosidase for its expression. Preferably, the nucleotide sequence encoding the α-1,3-fucosidase is a nucleotide sequence selected from the group consisting of
- a nucleotide sequence as represented by SEQ ID NO: 3;
- nucleotide sequences that are complementary to a nucleotide sequence which hybridizes to the nucleotide sequence as represented by SEQ ID NO: 3 under stringent conditions;
- nucleotide sequences having a sequence identity of at least 70%, 75%, 80%, 85%, 90%, 95%, 96%, 97%, 98% or 99 % to the nucleotide sequence as represented by SEQ ID NO: 3;
- nucleotide sequences encoding a polypeptide having an amino acid sequence as represented by SEQ ID NO: 4; and
- nucleotide sequences encoding a functional variant of the polypeptide sequences as represented by SEQ ID NO: 4, wherein the amino acid sequence of the functional variant has a sequence identity of at least 70%, 75%, 80%, 85%, 90%, 95%, 96%, 97%, 98% or 99% to the amino acid sequence as represented by SEQ ID NO: 4.

For expression of the nucleotide sequence encoding the α-1,3-fucosidase or functional variant thereof, said nucleotide sequence is operably linked to expression control sequences which mediate expression of the nucleotide sequence encoding the α-1,3-fucosidase or functional variant thereof in the genetically-engineered microbial host cell.

A suitable sialidase is an α-2,3-sialidase. The α-2,3-sialidase is a highly specific exoglycosidase that catalyzes the hydrolysis of linear α-2,3-linked L-sialyl residues from oligosaccharides. A preferred α-2,3-sialidase is NanB of *Streptococcus pneumoniae* (SEQ ID NO: 6).

In an additional and/or alternative embodiment, a genetically-engineered microbial host cell that is able to produce 6'-SL is provided, wherein said genetically-engineered microbial host cell expresses an α-2,3 sialidase. To be able to produce 6'-SL, the genetically engineered microbial host cell expresses an α-2,6-sialyltransferase. Said α-2,6-sialyltransferase is able to transfer a Neu5Ac residue from CMP-Neu5Ac to the galactose moiety of lactose as substrate, thereby synthesizing 6'-SL. 3'-SL is an undesired by-product in the production of 6'-SL.

By expressing an α-2,3-sialidase in the genetically-engineered microbial host cell that is able to produce 6'-SL, production of the by-product 3'-SL can be abolished or at least diminished in that this by-product is hydrolyzed by the α-2,3-sialidase within the genetically-modified microbial host cell. The resulting degradation products are *N*-acetylneuraminic acid and lactose. Both, *N*-acetylneuraminic acid and lactose, can be utilized by the genetically-engineered microbial host organism for producing the desired 6'-SL.

In an additional and/or alternative embodiment, the genetically-engineered microbial host cell has been genetically engineered to express the α-2,3-sialidase. In an additional and/or alternative embodiment, the genetically-engineered microbial host cell has been genetically engineered to contain a nucleic acid molecule comprising a nucleotide sequence encoding the α-2,3-sialidase for its expression. Preferably, the nucleotide sequence encoding the α-2,3-sialidase is a nucleotide sequence selected from the group consisting of
- a nucleotide sequence as represented by SEQ ID NO: 5;
- nucleotide sequences that are complementary to a nucleotide sequence which hybridizes to the nucleotide sequence as represented by SEQ ID NO: 5 under stringent conditions;
- nucleotide sequences having a sequence identity of at least 70%, 75%, 80%, 85%, 90%, 95%, 96%, 97%, 98% or 99 % to the nucleotide sequence as represented by SEQ ID NO: 5;
- nucleotide sequences encoding a polypeptide having an amino acid sequence as represented by SEQ ID NO: 6; and
- nucleotide sequences encoding a functional variant of the polypeptide sequences as represented by SEQ ID NO: 6, wherein the amino acid sequence of the functional variant has a sequence identity of at least 70%, 75%, 80%, 85%, 90%, 95%, 96%, 97%, 98% or 99% to the amino acid sequence as represented by SEQ ID NO: 6.

For expression of the nucleotide sequence encoding the α-2,3-sialidase or functional variant thereof, said nucleotide sequence is operably linked to expression control sequences which mediate expression of the nucleotide sequence encoding the α-2,3-sialidase or functional variant thereof in the genetically-engineered microbial host cell.

A suitable galactosidase is a ß-1,3-galactosidase. The ß-1,3-galactosidase is an enzyme that catalyzes the hydrolysis of a ß-1,3-linked galactose residue from oligosaccharides. A preferred ß-1,3-galactosidase is Bga42A of *Bifidobacterium longum* (SEQ ID NO: 8).

In an additional and/or alternative embodiment, a genetically-engineered microbial host cell is provided that is able to produce LNnT, wherein said genetically-engineered microbial host cell expresses a ß-1,3-galactosidase. To be able to produce LNnT, the genetically-engineered microbial host cell expresses a β-1,3-*N*-acetyl-glucosaminylransferase and a β-1,4-galactosyltransferase. Said β-1,3-*N*-acetyl-glucosaminylransferase is able to transfer a GlcNAc residue from UDP-GlcNAc to the galactose moiety of lactose, thereby synthesizing LNT-II. Said β-1,4-galacto-syltransferase is able to transfer a galactose residue from UDP-galactose to the GlcNAc moiety of LNT-II, thereby synthesizing LN*n*T as desired oligosaccharide.

LNT is an undesired by-product in the production of LN*n*T. By expressing a ß-1,3-galactosidase in the genetically-engineered microbial host cell being able to produce LN*n*T, production of the by-product LNT can be abolished or at least diminished in that this by-product is hydrolyzed within the genetically engineered microbial host cell by the heterologous ß-1,3-galactosidase. The resulting degradation products are galactose and LNT-II. Galactose as well as LNT-II can be utilized by the genetically-engineered microbial host organism for the production of the desired LN*n*T.

In an additional and/or alternative embodiment, the genetically-engineered microbial host cell has been genetically engineered to express the ß-1,3-galactosidase. In an additional and/or alternative embodiment the genetically-engineered microbial host cell has been genetically engineered to contain a nucleic acid molecule comprising a nucleotide sequence encoding the ß-1,3-galactosidase for its expression.

In an additional and/or alternative embodiment, the genetically-engineered microbial host cell has been genetically engineered to express the ß-1,3-galactosidase. In an additional and/or alternative embodiment the genetically-engineered microbial host cell has been genetically engineered to contain a nucleic acid molecule comprising a nucleotide sequence encoding the ß-1,3-galactosidase for its expression. Preferably, the nucleotide sequence encoding the ß-1,3-galactosidase is a nucleotide sequence selected from the group consisting of
- a nucleotide sequence as represented by SEQ ID NO: 7;
- nucleotide sequences that are complementary to a nucleotide sequence which hybridizes to the nucleotide sequence as represented by SEQ ID NO: 7 under stringent conditions;
- nucleotide sequences having a sequence identity of at least 70%, 75%, 80%, 85%, 90%, 95%, 96%, 97%, 98% or 99 % to the nucleotide sequence as represented by SEQ ID NO: 7;
- nucleotide sequences encoding a polypeptide having an amino acid sequence as represented by SEQ ID NO: 8; and
- nucleotide sequences encoding a functional variant of the polypeptide sequences as represented by SEQ ID NO: 8, wherein the amino acid sequence of the functional variant has a sequence identity of at least 70%, 75%, 80%, 85%, 90%, 95%, 96%, 97%, 98% or 99% to the amino acid sequence as represented by SEQ ID NO: 8.

For expression of the nucleotide sequence encoding the ß1,3-galactosidase or functional variant thereof, said nucleotide sequence is operably linked to expression control sequences which mediate expression of the nucleotide sequence encoding the ß1,3-glucosidase or functional variant thereof in the genetically-engineered microbial host cell.

Another suitable galactosidase is a galactan ß-1,3-galactosidase. The galactan ß-1,3-galactosidase is an enzyme that catalyzes the hydrolysis of a ß-1,3-linked galactose residue from galactose bearing oligosaccharide chains. A preferred galactan ß-1,3-galactosidase is Ct1,3Gal43A of *Clostridium thermocellum* (SEQ ID NO: 10).

In an additional and/or alternative embodiment, the genetically-engineered microbial host cell has been genetically engineered to express the galactan ß-1,3-galactosidase. In an additional and/or alternative embodiment the genetically-engineered microbial host cell has been genetically engineered to contain a nucleic acid molecule comprising a nucleotide sequence encoding the galactan ß-1,3-galactosidase for its expression. Preferably, the nucleotide sequence encoding the galactan ß-1,3-galactosidase is a nucleotide sequence selected from the group consisting of
- a nucleotide sequence as represented by SEQ ID NO: 9;
- nucleotide sequences that are complementary to a nucleotide sequence which hybridizes to the nucleotide sequence as represented by SEQ ID NO: 9 under stringent conditions;
- nucleotide sequences having a sequence identity of at least 70%, 75%, 80%, 85%, 90%, 95%, 96%, 97%, 98% or 99 % to the nucleotide sequence as represented by SEQ ID NO: 9;
- nucleotide sequences encoding a polypeptide having an amino acid sequence as represented by SEQ ID NO: 10; and
- nucleotide sequences encoding a functional variant of the polypeptide sequences as represented by SEQ ID NO: 10, wherein the amino acid sequence of the functional variant has a sequence identity of at least 70%, 75%, 80%, 85%, 90%, 95%, 96%, 97%, 98% or 99% to the amino acid sequence as represented by SEQ ID NO: 10.

For expression of the nucleotide sequence encoding the galactan ß-1,3-galactosidase or functional variant thereof, said nucleotide sequence is operably linked to expression control sequences which mediate expression of the nucleotide sequence encoding the galactan ß-1,3-glucosidase or functional variant thereof in the genetically-engineered microbial host cell.

A suitable glucosidase is a ß-1,3-glucosidase. The ß-1,3-glucosidase is a highly specific exoglycosidase that catalyzes the hydrolysis of a ß-1,3-linked glucose residue from oligosaccharides. A preferred ß-1,3-glucosidase is PglA of *Paenibacillus* sp. (SEQ ID NO: 12).

In an additional and/or alternative embodiment, a genetically-engineered microbial host organism is provided that is able to produce LNT or LNnT, wherein said genetically-engineered microbial host cell expresses a ß1,3-glucosidase and/or a ß-1,3-galactosidase. To be able to produce LNT, the genetically-engineered microbial host cell expresses a β-1,3-*N*-acetylglucosaminyltransferase and a β-1,3-galactosyltransferase. Said β-1,3-*N*-acetylglucosaminyltransferase is able to transfer a GlcNAc residue from UDP-GlcNAc to the galactose moiety of lactose, thereby synthesizing lacto-*N*-triose-II (LNT-II). Said β-1,3-galactosyltransferase is able to transfer a galactose residue from UDP-galactose to the GlcNAc moiety of LNT-II, thereby synthesizing lacto-*N*-tetraose (LNT). To be able to produce LNnT, the genetically-engineered microbial host cell expresses a β-1,3-*N*-acetylglucosaminyl-transferase and a β-1,4-galactosyltransferase. Said β-1,3-*N*-acetylglucosaminyl-transferase is able to synthesize LNT-II. Said a β-1,4-galactosyltransferase is able to transfer a galactose residue from UDP-galactose to the GlcNAc moiety of LNT-II, thereby synthesizing LNnT as desired oligosaccharide.

It is known to the skilled artisan that β-1,3-*N*-acetylglucosaminyltransferases like LgtA of *Neisseria meningitidis* accept a broad spectrum of donor substrates. While primarily transferring GlcNAc from UDP-GlcNAc to an appropriate acceptor saccharide, LgtA is also capable to use UDP-galactose or UDP-glucose as donor substrates. Using a genetically-engineered microbial host organism capable to produce LNT or LNnT as described, said β-1,3-*N*-acetylglucosaminyltransferase is also able to transfer a galactose residue from UDP-galactose as well as a glucose residue from UDP-glucose to the galactose moiety of lactose, thereby synthesizing the undesired by-products Gal(β1,3)Gal(β1,4)Glc and Glc(β1,3)Gal(β1,4)Glc, respectively.

By expressing a galactan ß-1,3-gactosidase and/or a ß-1,3-glucosidase in the genetically-engineered microbial host cell being able to produce LNT or LNnT, production of the by-products Gal(β1,3)Gal(β1,4)Glc and Glc(β1,3)Gal(β1,4)Glc can be abolished or at least diminished in that these by-products are hydrolyzed within the genetically-engineered microbial host cell by the galactan ß-1,3-galactosidase and/or the ß1,3-glucosidase. The resulting degradation products are galactose and/or glucose and lactose. Both monosaccharides as well as lactose can be utilized by the genetically-engineered microbial host cell to produce the desired LNT or LNnT.

In an additional and/or alternative embodiment, the genetically-engineered microbial host cell has been genetically engineered to express the ß-1,3-glucosidase. In an additional and/or alternative embodiment, the genetically-engineered microbial host cell has been genetically engineered to contain a nucleic acid molecule comprising a nucleotide sequence encoding the ß-1,3-glucosidase for its expression. Preferably, the nucleotide sequence encoding the ß-1,3-glucosidase is a nucleotide sequence selected from the group consisting of
- a nucleotide sequence as represented by SEQ ID NO: 11;
- nucleotide sequences that are complementary to a nucleotide sequence which hybridizes to the nucleotide sequence as represented by SEQ ID NO: 11 under stringent conditions;
- nucleotide sequences having a sequence identity of at least 70%, 75%, 80%, 85%, 90%, 95%, 96%, 97%, 98% or 99 % to the nucleotide sequence as represented by SEQ ID NO: 11;
- nucleotide sequences encoding a polypeptide having an amino acid sequence as represented by SEQ ID NO: 12; and
- nucleotide sequences encoding a functional variant of the polypeptide sequences as represented by SEQ ID NO: 12, wherein the amino acid sequence of the functional variant has a sequence identity of at least 70%, 75%, 80%, 85%, 90%, 95%, 96%, 97%, 98% or 99% to the amino acid sequence as represented by SEQ ID NO: 10.

For expression of the nucleotide sequence encoding the ß-1,3-glucosidase or functional variant thereof, said nucleotide sequence is operably linked to expression control sequences which mediate expression of the nucleotide sequence encoding the ß-1,3-glucosidase or functional variant thereof in the genetically-engineered microbial host cell.

The genetically engineered microbial host cell is able to recycle at least one of the degradation products resulting from the enzymatic activity of the heterologous glycosidase in the genetically-engineered microbial host cell. Thus, the genetically-engineered microbial host cell can use at least one of the degradation products resulting from the enzymatic activity of the heterologous glycosidase for the production of the desired oligosaccharide. For example, a monosaccharide residue released from the undesired saccharide by-product by the heterologous glycosidase can be reactivated, i.e. bound to a nucleotide, to be transferred from the resulting nucleotide-activated monosaccharide to an acceptor substrate by a respective glycosyltransferase to give the desired oligosaccharide or a precursor of the desired oligosaccharide.

The method comprises the step of cultivating the genetically engineered microbial host cell in a medium that is permissive for the production of the desired oligosaccharide by said genetically engineered microbial host organism, and under conditions that are permissive for the production of the desired oligosaccharide by said genetically engineered microbial host organism.

The medium that is permissive for the production of the desired oligosaccharide by the genetically-engineered microbial host cell contains nutrients, at least one energy source, essential metals and minerals and a buffering agent. The medium optionally contains a precursor of the desired oligosaccharide, said precursor may be internalized by the genetically-engineered microbial host cell and utilized for the production of the desired oligosaccharide provided that the genetically-engineered microbial host cell is not able to synthesize said precursor on its own. Then, the genetically-engineered microbial host cell internalizes the precursor and subjects the precursor to the biosynthesis of the desired oligosaccharide. For example, lactose can be considered a precursor of 2'-fucosyllactose.

During the cultivation of the genetically-engineered microbial host cells for producing the desired oligosaccharide, permissive conditions are maintained. Conditions are "permissive" if the genetically-engineered microbial host cells that are cultured under these conditions stay alive and produce the desired oligosaccharide. Preferably the permissive culture conditions enable the genetically-engineered microbial host cells to multiply. Conditions that need to be kept at a certain value or within a certain range include pH, temperature, oxygen and concentrations of nutrients, energy sources and essential metals and minerals.

In an additional and/or alternative embodiment, the method comprises the step of recovering the desired oligosaccharide. The desired oligosaccharide may be recovered from the fermentation broth and/or from the genetically engineered microbial host organism.

The method as describe herein before is advantageous in that less or no undesired by-products are produced during the production of the desired oligosaccharide. Therby, it is less cumbersome and costly to recover and purify the desired oligosaccharide from the fermentation broth or cell lysate.

In addition, much more substrate is specifically used for the production of the desired oligosaccharide instead of becoming inaccessible for the production of the desired oligosaccharide as it is incoporated in undesired by-products which can not be metabolized by the microbial host cell.

According to the second aspect, provided are genetically-engineered microbial host cells for the production of a desired oligosaccharide, wherein the microbial host cell is able to produce the desired oligosaccharide, and wherein the microbial host cell has been genetically engineered to express a heterologous glycosidase which is able to intracellularly degrade metabolic by-products that are generated during the intracellular biosynthesis of the desired oligosaccharide.

According to the third aspect, the genetically-enigneered microbial host cells as described herein before are used for the production of a desired oligosaccharide. Using these genetically-engineered mcirobial host cells for the production of a desired oligosaccharide by fermentation is advantageous, because the production of undesired saccharide by-products is prevented or even abolished. Therefore, it saves resources and is less cumbersome to recover the desired oligosaccharide from the fermentation broth, as separation of the desired oligosaccharide from undesired oligosaccharide by-products can be avoided. Moreover, much more educt and energy-sources provided to the genetically-engineered microbial host cells according to the present invention are converted to the desired product as compared to a native microbial host cell that has not been genetically engineered to express a heterologous glycosidase.

According to the fourth aspect, the desired oligosaccharides that are produced by the method and/or the use of the genetically-engineered microbial host cells described herein before are preferably selected from the group of HMOs.

The desired oligosaccharides that are produced by the method and/or the use of the genetically-engineered microbial host cells described herein can be used for the production of a nutritional composition.

The nutritional composition is a medicinal composition, a dietary composition, an infant formula or the like.

The present invention will be described with respect to particular embodiments and with reference to drawings, but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description and drawings provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

### Example 1: Metabolic engineering of an E. coli BL21 (DE3) strain for the production of 2'-fucosyllactose

*E. coli* BL21 (DE3) (Novagen) was used as parental strain for the construction of a host strain for the production of 2'-FL. Genetic engineering of the parental strain included gene disruption and deletion events and integration of heterologous genes.

Since 2'-fucosyllactose is synthesized from lactose, that is applied to the bacterial culture, and from GDP-L-fucose that is produced from the living cells, first the wild-type copy of the *lacZ* gene encoding the endogenous β-galactosidase was inactivated by mutagenesis using mismatch oligonucleotides (Ellis et al., "High efficiency mutagenesis, repair, and engineering of chromosomal DNA using single-stranded oligonucleotides", Proc. Natl. Acad. Sci. USA 98: 6742-6746 (2001)). Using the same method, the gene for the arabinose-isomerase *araA* was disrupted.

A *lacZΩ* gene fragment was introduced under the control of the temperature sensitive transcriptional repressor cl857. The *lacZα* fragment gene is expressed under the control of the *E*. *coli* BL21 (DE3) *PgbA* promoter in the strain, revealing a LacZ⁺ strain.

Genomic deletions were performed by λ Red mediated recombination according to the method of Datsenko and Warner ("One-step inactivation of chromosomal genes in Escherichia coli K-12 using PCR products", Proc. Natl. Acad. Sci. USA 97:6640-6645 (2000)). The genes *fucI* and *fucK,* coding for the L-fucose isomerase and the L-fuculose kinase, respectively, have been deleted to prevent degradation of L-fucose. Also genes *wzxC-wcaJ* were deleted. *Wca*J probably encodes a UDP-glucose: undecaprenyl phosphate glucose-1-phosphate transferase catalysing the first step in colanic acid synthesis (Stevenson et al., "Organization of the Escherichia coli K-12 gene cluster responsible for production of the extracellular polysaccharide colonic acid", J. Bacteriol. 178:4885-4893; (1996)); production of colanic acid would compete for GDP-fucose with the fucosyltransferase reaction.

Genomic integration of heterologous genes was performed by transposition. Large gene clusters were integrated into the genome mediated by the hyperactive C9-mutant of the mariner transposase Himar1 (Lampe et al., "Hyperactive transposase mutants of the Himar1 mariner transposon", Proc. Natl. Acad. Sci. USA 96:11428-11433 (1999)), that was inserted into the plasmid pEcomar under transcriptional control of the Pₐᵣₐ promotor. To enhance *de novo* synthesis of GDP-fucose, genes encoding phosphomannomutase (*man*B), mannose-1-phosphate guanosyltransferase (*manC*)*,* GDP-mannose-4,6-dehydratase (*gmd*), and GDP-L-fucose synthase (*wca*G) from *E. coli* K12 DH5α were overexpressed in the *E*. *coli* BL21(DE3) strain; the operon manCB was set under control of the constitutive promoter Pₜₑₜ, the operon *gmd, wca*G is transcribed from the constitutive P_{T5} promoter. The transposon cassette <Pₜₑₜ₋*man*CB-P_{T5}-*gmd*, *wca*G-FRT-*dhfr*-FRT> (SEQ ID NO: 13), including the gene for the dihydrofolate reductase for trimethoprim resistance, flanked by the inverted terminal repeats specifically recognized by the mariner-like element Himar1 transposase was inserted into the *E*. *coli* genome from pEcomar C*9-manCB-gmd, wca*G*-dhfr.*

For chromosomal integration of single genes, the EZ-Tn5™ transposase (Epicentre, USA) was used. To produce EZ-Tn5 transposomes the gene of interest together with a FRT-site flanked antibiotic resistance cassette was amplified with primers that carried on both sites the 19-bp Mosaic End recognition sites (5'-CTGTCTCTTAT-ACACATCT) for the EZ-Tn5 transposase. Using the EZ-Tn5TM transposase, the gene for the lactose importer LacY from *E. coli* K12 TG1 (acc. no. ABN72583), the 2-fucosyltransferase gene *wbg*L from *E. coli*:O126 (acc. no. ADN43847), and the gene *yberc0001_9420* encoding a sugar efflux transporter of the major facilitator superfamily from *Yersinia bercovieri* ATCC 43970 (acc.no. EEQ08298) were integrated using the respective integration cassettes: <Pₜₑₜ-*lacY*-FRT-*aadA*-FRT> (SEQ ID NO: 14), <Pₜₑₜ-*wbgLco*-FRT-*neo*-FRT> (SEQ ID NO: 15), and <Pₜₑₜ-*yberc0001_9420co-*FRT*-cat-*FRT*>* (SEQ ID NO: 16). The genes *wbg*L and *yberc0001_9420* were synthetically synthesized and codon optimized (co) by GenScript Cooperation (USA). After successful integration of the *lac*Y gene the resistance gene was eliminated from streptomycine resistant clones by the FLP recombinase encoded on plasmid pCP20 (Datsenko and Warner, "One-step inactivation of chromosomal genes in Escherichia coli K-12 using PCR products", Proc. Natl. Acad. Sci. USA 97:6640-6645 (2000)).

Since *E*. *coli* BL21 (DE3) lacks a functional *gal*-operon, a natively regulated copy of the ga/ETKM operon from *E. coli* K was integrated into the B strain by EZ-transposition using integration cassette <P_{gal}-*gal*E-*gal*T-*gal*K-*gal*M> (SEQ ID NO: 17). Integrants were selected from MacConkey-agar containing 1% galactose as red colonies. The resulting strain is able to metabolize the monosaccharides glucose and galactose originating from lactose hydrolysis.

Further improvement concerning the synthesis of 2'-fucosyllactose by the *E*. *coli* strain was achieved by deletion of the *pfkA* gene, encoding the phosphofructokinase A. When cultivating *E*. *coli* on a gluconeogenic substrate like glycerol the phosphorylation of fructose-6-phosphate by PfkA is a highly ATP consuming treadmill reaction and, in addition, it competes with ManA for the substrate. The *pfkA* gene was deleted by homologous recombination according to Datsenko and Wanner (2000) using a gentamycin resistance cassette (*aac*C1) that was flanked by *lox*71/66 sites (Lambert, JM et al. (2007) Cre-lox-based system for multiple gene deletions and selectable -marker removal in Lactobacillus plantarum. Appl. Environ. Microbiol 73: 1126-1135). After successful deletion of the *pfkA* gene the antibiotic resistance gene was removed from *E*. *coli* genome using the Cre recombinase (Abremski, K et al. (1983) Studies on the properties of P1 site-specific recombination: evidence for topologically unlinked products following recombination. Cell 32: 1301-1311) that was cloned under the control of the Pₐᵣₐ promoter in the pKD46 (Datsenko and Wanner, 2000) chassis.

For different fucosyltransferases besides the transferase activity a GDP-L-fucose hydrolase activity was demonstrated. Also for *wbg*L, the α-1,2-fucosyltransferase used here for 2'-fucosyllactose synthesis this hydrolytic activity was shown (see EP 3 050 973 A1). To rescue free L-fucose for the 2'-fucosyllactose production and to eliminate the contaminating L-fucose from the culture broth, the *fkp* gene, encoding the bifunctional L-fucokinase/L-fucose 1-phosphat guanylyltranferase of *Bacteroides fragilis*, under transcriptional control of the Pₜₑₜ promoter, together with the lox71/66 flanked aacC1 gene was chromosomally integrated by transposition using the EZ-Tn5™ transposase, <Pₜₑₜ-*fkp*-lox-*aac*C1-lox> (SEQ ID NO: 18). After successful integration the gentamycin resistance gene was removed from the genome as described above.

To enhance the flux of the metabolized carbon source glycerol through the gluconeogenic pathway from triose-phosphates to fructose-6-phophate to feed the GDP-L-fucose biosynthesis the genes encoding the fructose-1,6-bisphosphate aldolase (*fba*B) and a heterologous fructose-1,6-bisphosphate phosphatase (*fbpase*) from *Pisum sativum* were overexpressed. The *fba*B gene from *E. coli* BL21 (DE3) was fused with the Pₜₑₜ promoter. The activity of the chloroplasic *P. sativum* FBPase is allosterically regulated by a disulfide-dithiol exchange due to reduction by thio-redoxins. Exchange of the cysteine residue 153 to serine results in a constitutively active enzyme. The gene encoding the chloroplastic FBPase from *P. sativum* (acc. No. AAD10213) was purchased codon optimized for expression in *E. coli,* N-terminally tagged with a hexahistidine-tag and modified to encode the C153S variant of the enzyme from Genescript. The *fbpase* gene is transcribed from a T7 promoter. The cassette <Pₜₑₜ-*fbaB*-P_{T7}-His₆-*fbpase*-lox-*aacC1*-lox> (SEQ ID NO: 19) was used for EZ-Tn5™ transposase mediated integration in the host strain. After removal of the gentamycin resistance gene from the *E*. *coli* genome the strain was used for 2'-fucosyllactose production. Subsequently, this strain is named "strain A".

### Example 2: Engineering of an E. coli BL21 (DE3) strain for the production of 2'-fucosyllactose at high purity

Fed-batch cultivations using strain A for 2'-fucosyllactose production revealed the presence of by-products (3-fucosyllactose and 2'3-difucosyllactose) in the culture broth. In order to minimize the production of these by-products as well as for improving the carbon yield, an α-1,3-fucosidase was sub-cloned behind a constitutive promotor and integrated into the genome of strain A. Therefore, the afcB gene of *Bifidobacterium bifidum* (acc. No. AB474964) was fused with the constitutive P_{and} promoter and the gentamycin resistance gene. The resulting transposon cassette <P_{and}-*afcB*-lox-*aacC1*-lox> (SEQ ID NO: 20), flanked by the inverted terminal repeats specifically recognized by the mariner-like element Himar1 transposase, was inserted into the *E. coli* genome from pEcomar *afcB-aacC1*, generating "strain B".

### Example 3: HPLC analysis for the detection of 2'-fucosyllactose in culture supernatant

Analyses by HPLC were performed using a refractive index detector (RID-10A) (Shimadzu, Germany) and a Waters XBridge Amide Column 3.5µm (250 x 4.6mm) (Eschborn, Germany) connected to an HPLC system (Shimadzu, Germany). Elution was performed isocratically with 30% A: 50% (v/v) ACN in ddH₂O, 0.1% (v/v) NH₄OH and 70% B: 80% (v/v) ACN in ddH₂O, 0.1% (v/v) NH₄OH (v/v) as eluent at 35°C and at a flow rate of 1.4 ml·min⁻¹. HPLC samples were sterile filtered (0.22 µm pore size) and cleared by solid phase extraction on an ion exchange matrix (Strata ABW, Phenomenex). 10 µl of the samples were applied to the column, and the 2'-fucosyllactose concentration was calculated according to a standard curve. Other sugars like L-fucose and/or other monosaccharides, lactose and/or other disaccharides, 3-fucosyllactose and/or other trisaccharides, 2'3-difucosyllactose and/or other tetrasaccharides as well as glycerol are also detectable using these analysis conditions. The relative amounts of detected sugars can be determined by comparing the AUC (area under the curve) of the all peaks in the chromatogram. Peaks also present in the water control are excluded from this calculation.

### Example 4: Production of 2'-fucosyllactose in a fermentative process

Fermentations were conducted in 3 L-fermenters at 33°C (New Brunswick, Edison, USA) starting with 1000 mL mineral salts medium containing 3 g/L KH₂PO₄, 12 g/L K₂HPO₄, 5 g/L (NH₄)₂SO₄, 0.3 g/L citric acid, 2 g/L MgSO₄ × 7·H₂O, 0.1 g/L NaCl and 0.015 g/L CaCl₂ × 6·H₂O supplemented with 1 g/L trace element solution (54.4 g·L⁻¹ ammonium ferric citrate, 9.8 g/L MnCl₂ × 4·H₂O, 1.6 g/L CoCl₂ × 6·H₂O, 1 g/L CuCl₂ × 2·H₂O, 1.9 g/L H₃BO₃, 9 g/L ZnSO₄ × 7·H₂O, 1.1 g/L Na₂MoO₄ × 2·H₂O, 1.5 g/L Na₂SeO₃, 1.5 g/L NiSO₄ × 6·H₂O) and containing 2 % (v/v) glycerol as carbon source, 60 mM lactose and the antibiotic kanamycin (25 µg/mL). Aeration was maintained at 3 L/min. Dissolved oxygen was maintained at 20 - 30 % saturation by controlling the rate of agitation. The pH was maintained at 7.0 by adding 25 % ammonia solution. Cultivation was started with a 2.5 % (v/v) inoculum from a preculture grown in the same glycerol containing medium but lacking lactose. After leaving the batch phase, indicated by a rise in the dissolved oxygen level, glycerol feeding (60 % (v/v), supplemented with 2 g/L MgSO₄ × 7·H₂O, 0.015 g/L CaCl₂ × 6·H₂O and 1 mL/L trace element solution) was carried out at flow rates of 7.0 - 8.0 mL/h, referring to the starting volume. Lactose feeding (0.66 M) was conducted throughout the cultivation and was adjusted intuitively in order to realize a constant lactose supply in the culture broth. Lactose feeding was stopped towards the end of the fermentation and cultivation was continued until lactose was completely converted to 2'-fucosyllactose. At around 94 hours after seeding the fermenter a 2'-fucosyllactose titer in the cell medium of about 150 g/L was reached when using the strain described in example 1 (strain A). The 2'-fucosyllactose production strain, genetically modified as described in example 2 (strain B), was cultivated comparably and yielded as well a 2'-fucosyllactose titer of about 150 g/L. However, the amount of by-products was significantly lower than after cultivating strain A (Table 2). Whereas the saccharide content in the culture supernatant of strain A consisted only to 94.22 % of 2'-fucosyllactose, it was increased by 5.50 % in the culture supernatant of strain B, exposing a purity of 99.72 %.

**Table 2: Qualitative HPLC analysis of the relative amount of saccharides detectable in the culture supernatants of strain A and strain B after 94 hours of cultivation (n.d.: not detectable).**

| | Purity [area percentage] | | |
|---|---|---|---|
| | 2'-fucosyllactose | 3-fucosyllactose | 2'3-difucosyllactose |
| Strain A | 94.22 % | 1.01 % | 4.77 % |
| Strain B | 99.72 % | n. d. | 0.28 % |

SEQUENCE LISTING

<110>  Jennewein Biotechnologie GmbH

<120>  Use of Glycosidases in the Production of Oligosaccharides

<130>  P 1804 EP

<160>  20

<170>  PatentIn version 3.5

<210>  1
<211>  5880
<212>  DNA
<213>  Bifidobacterium bifidum

<400>  1
atgaaacata gagcgatgtc atcgcgtctg atgccactgg tggcgtcctg cgcgacggtc      60

ggcatgctgc tggccggact acctgtgtcg gccgtcgcgg tcggcacgac gagagcggca     120

gcgtccgacg cctcgtcctc caccacagca accatcaccc cctccgccga taccacgttg     180

cagacatgga cgagcgagaa gaattcctca atggcgtcca agccgtacat cggcacactg     240

caagggccct cgcaaggcgt gttcggcgag aagttcgagt ccacggatgc cgcggacacc     300

accgatctga agaccggcct gctgacgttc gacctgagcg cctacgacca tgcccccgat     360

tccgcaacgt tcgagatgac gtacctcggc taccgcggca acccgacggc caccgacacc     420

gacaccatca aggtgacccc cgtcgacacc accgtgtgca ccaataacgc cacagactgc     480

ggcgcgaatg tcgcgaccgg cgcgaccaag ccgaagttca gcatcaacga ctcctcattc     540

gtcgccgagt ccaagccgtt cgagtacggt acgacggttt acacgggcga cgccatcacc     600

gtggttcccg ccaataccaa gaaggtcacc gtagatgtga ccgaaatcgt gcgccagcag     660

ttcgccgaag gcaagaaggt catcaccctg gccgtgggcg agaccaagaa gaccgaggtt     720

cgtttcgcca gttccgaagg cacgacgtcc ctgaacggcg cgaccgcaga catggctccg     780

aagctgaccg tttccgtgtc caccaaggac gatctcaagc cctccgccga caccacgttg     840

caggcatggg ccagcgagaa gaacgagaag aagaacactg cggcctatgt cggcgcgctg     900

cagccggaag gcgattacgg cgacttcggt gagaagttca agtccaccga cgtccacgat     960

gtcacagacg ccaagatggg tctgatgacg ttcgacctgt ccgattacac cgcggcgccc    1020

gagcactcca tcctcacctt gacgtatctg ggctacgccg gtgcagacaa gaccgccacg    1080

gccaccgata aggtcaaggt ggtcgctgtt gacacgtcgc ggtgcaccgg caccgctccc    1140

tgcgacacca acaatgccac gtgggcgaac cgcccggact tcgaggtgac cgataccacg    1200

aagaccgcga cgtcccatgc gttcgcttat ggatctaaga agtattccga tggcatgacc    1260

gtcgaatcgg gcaacgccaa gaaggtcctg ctcgacgtgt ccgatgtcat caaggcagag    1320

ttcgccaagt tcagcgccgg cgccaccgag aagaagatca cgctggccct gggcgagctc    1380

aacaagtccg acatgcgttt cggcagcaag gaagtcacct cgctgaccgg cgccaccgaa 1440

gccatgcagc cgaccttgtc cgtcaccaag aagccgaagg catacacgct gagcatcgaa 1500

ggcccgacca aggtcaagta ccagaagggc gaggcgttcg acaaggccgg actcgtggtc 1560

aaggccacca gcacggctga cggcacggtc aagacgctga ccgaaggcaa cggtgaggat 1620

aactacacca tcgacaccag cgctttcgat agtgccagca tcggcgtata ccctgttacc 1680

gtgaagtaca acaaggaccc cgaaatcgcc gcttcgttca acgcctatgt catcgccagt 1740

gtcgaggacg gcggagacgg cgacaccagc aaagacgact ggctgtggta caagcagccc 1800

gcgtcgcaga ccgacgccac cgccaccgcc ggcggcaatt acggcaaccc cgacaacaac 1860

cgttggcagc agaccacctt gccgttcggc aacggcaaga tcggcggcac cgtctggggc 1920

gaggtcagcc gtgaacgcgt caccttcaac gaggagacgc tgtggaccgg cggccccgga 1980

tcctcgacca gctacaacgg cggcaacaac gagaccaagg gtcagaacgg cgccacgctg 2040

cgcgcgctca acaagcagct cgcgaacggc gccgagacgg tcaatcccgg caacctgacc 2100

ggcggcgaga acgcggccga gcagggcaac tacctgaact ggggcgacat ctacctcgac 2160

tacgggttca acgatacgac cgtcaccgaa taccgccgcg acctgaacct gagcaagggc 2220

aaggccgacg tcacgttcaa gcatgacggc gtcacctaca cgcgcgaata cttcgcgtcg 2280

aaccccgaca atgtcatggt cgcccgcctc acggccagca aagccggcaa gctgaacttc 2340

aacgtcagca tgccgaccaa cacgaactac tccaagaccg gcgaaaccac gacggtcaag 2400

ggtgacacgc tcaccgtcaa gggcgctctc ggcaacaacg gcctgctgta caactcgcag 2460

atcaaggtcg tcctcgacaa cggtgagggc acgctctccg aaggctccga cggcgcttcg 2520

ctgaaggtct ccgacgcgaa ggcggtcacg ctgtacatcg ccgccgcgac ggactacaag 2580

cagaagtatc cgtcctaccg caccggcgaa accgccgccg aggtgaacac ccgcgtcgcc 2640

aaggtcgtgc aggacgccgc caacaagggc tacaccgccg tcaagaaagc gcacatcgac 2700

gatcattccg ccatctacga ccgcgtgaag atcgatttgg gccagtccgg ccacagctcc 2760

gacggcgccg tcgccaccga cgcgctgctc aaggcgtacc agagaggctc cgcaaccacc 2820

gcgcagaagc gcgagctgga gacgctggtg tacaagtacg gccgctactt gaccatcggc 2880

tcctcccgtg agaacagcca gctgcccagc aacctgcagg gcatctggtc ggtcaccgcg 2940

ggcgacaacg cccacggcaa cacgccttgg ggctccgact tccacatgaa cgtgaacctc 3000

cagatgaact actggccgac ctattcggcc aacatgggag agctcgccga gccgctcatc 3060

gagtatgtgg agggtctggt caagcccggc cgtgtgaccg ccaaggtcta cgcgggcgcg 3120

gagacgacga accccgagac cacgccgatc ggcgagggcg agggctacat ggcccacacc 3180

gagaacaccg cctacggctg gaccgcaccc ggtcaatcgt tctcgtgggg ttggagcccg 3240

gccgccgtgc cgtggatcct gcagaacgtg tacgaggcgt acgagtactc cggcgaccct 3300

gccctgcttg atcgcgtgta cgcgctgctc aaggaggaat cgcacttcta cgtcaactac 3360

atgctgcaca aggccggctc cagctccggt gaccgcctga ctaccggcgt cgcgtactcg 3420

cccgaacagg gcccgctggg caccgacggc aacacgtacg agagctcgct cgtgtggcag 3480

atgctcaacg acgccatcga ggcggccaag gccaagggag atccggacgg tctggtcggc 3540

aataccaccg actgctcggc cgacaactgg gccaagaatg acagcggcaa cttcaccgat 3600

gcgaacgcca accgttcctg gagctgcgcc aagagcctgc tcaagccgat cgaggtcggc 3660

gactccggcc agatcaagga atggtacttc gaaggtgcgc tcggcaagaa gaaggatgga 3720

tccaccatca gcggctacca ggcggacaac cagcaccgtc acatgtccca cctgctcgga 3780

ctgttccccg gtgatttgat caccatcgac aactccgagt acatggatgc ggccaagacc 3840

tcgctgaggt accgctgctt caagggcaac gtgctgcagt ccaacaccgg ctgggccatt 3900

ggccagcgca tcaattcgtg ggctcgcacc ggcgacggca acaccacgta ccagctggtc 3960

gagctgcagc tcaagaacgc gatgtatgca aacctgttcg attaccatgc gccgttccag 4020

atcgacggca acttcggcaa cacctccggt gtcgacgaaa tgctgctgca gtccaactcc 4080

accttcaccg acaccgccgg caagaagtac gtgaactaca cgaacatcct gcccgccctg 4140

cccgatgcct gggcgggcgg ctcggtgagc ggcctcgtgg cccgcggcaa cttcaccgtc 4200

ggcacgacat ggaagaacgg caaggccacc gaagtcaggc tgacctccaa caagggcaag 4260

caggcggccg tcaagatcac cgccggcggc gcccagaact acgaggtcaa gaacggtgac 4320

accgccgtga acgccaaggt cgtgaccaac gcggacggcg cctcgctgct cgtgttcgat 4380

accaccgcag gcaccacgta cacgatcacg aagaaggcga gcgccaacgt gcccgtcacc 4440

ggcgtgaccg tgaccggcgc caacaccgcc accgcaggcg acaccgtcac tcttacggct 4500

accgtcgccc cggccaatgc gaccgacaag tccgtcacct ggtcgacctc cgacgccgcc 4560

gtagctacgg tcaacgccaa cggcgtggtg accacgaaga aggccggcaa ggtgaccatc 4620

accgccacgt cgaacggcga caagacgaag ttcggttcca tcgagatcac cgtctccgcc 4680

gcgaccgtgc ccgtcaccag cgtcaccgtt gccggcgacg ccgcgatgac cgtcgatgga 4740

gagcagaccc tgacggcgac cgtcgccccg gccactgcga ccgacaagac ggtcacgtgg 4800

aagtcctccg acgccactgt ggcgacggtt gacgccaacg gcaaggtcgt cgcgaagaag 4860

gccggcgaag tgacgatcac cgccacggcc ggtggcgtgt ccggcacgct gaagatcacg 4920

gtgagcgaca aggccccgac cgtcatcccg gtccagtccg tgaccgtgac aggcaagcag 4980

gagctcgtcg aaggcgcctc cacgaccctg acggcgaccg tcgccccggc tgacgcgacc 5040

gacaagacgg ttacgtggaa gtcgagcgac gagtccgtcg ccacggtcga caaggacggc 5100

gtcgtgaccg ccaagaaggc cggcacggtg accatcaccg ccacggccgg tggcgtgtcc 5160

ggcacgctcc acatcaccgt gacggccaag cccgtcgaga ccgtccccgt caccagcgtg 5220

gaggtcaccg tcgaggccgg caccaccgtc tccgtcggca agacactcca ggccaccgcg 5280

accgtcaagc ccggcaacgc caccaacaag aaggtgacgt ggaagtcgag cgacgaatcc 5340

atcgcgacgg tcgacgccaa cggcgtcatc accgcgaaga aggccggcaa ggtcgtcatc 5400

acggccacct cgaccgacgg cacggacaag tccggcagcg tcgagatcac cgtcgtggat 5460

gagaccaagc cgacgcccga ccacaagtcc gtcaaggccg ataccggcga cgtgaccgcc 5520

ggcaagaccg gtacggtcac cgagccgaag gacgtggcgg gctggaagag ccgctccatc 5580

atcaagcaag gcaagctcgg caaggccgaa atcgccgacg gcacgctcgt gtatgcggcc 5640

ggcgacaaga ccggtgacga cagcttcgtc gtgcagtaca cgatggccga cggcacggtc 5700

atcgacgtga cctacagcgt cacggtcaag gccgccgaaa ccggcaagaa cgacggcgac 5760

ggcaagggcg acggtgtcgc gaagaccggc gccgccgtcg gcgcgctcgc cggcctcggc 5820

ttgatgctgc tcgccgtcgg agtgagcgtg gtgatgattc gccgcaagca ctccgcctga 5880

<210> 2
<211> 1959
<212> PRT
<213> Bifidobacterium bifidum

<400> 2

Met Lys His Arg Ala Met Ser Ser Arg Leu Met Pro Leu Val Ala Ser
1               5                   10                  15

Cys Ala Thr Val Gly Met Leu Leu Ala Gly Leu Pro Val Ser Ala Val
            20                  25                  30

Ala Val Gly Thr Thr Arg Ala Ala Ala Ser Asp Ala Ser Ser Ser Thr
        35                  40                  45

Thr Ala Thr Ile Thr Pro Ser Ala Asp Thr Thr Leu Gln Thr Trp Thr
    50                  55                  60

Ser Glu Lys Asn Ser Ser Met Ala Ser Lys Pro Tyr Ile Gly Thr Leu
65                  70                  75                  80

Gln Gly Pro Ser Gln Gly Val Phe Gly Glu Lys Phe Glu Ser Thr Asp
                85                  90                  95

Ala Ala Asp Thr Thr Asp Leu Lys Thr Gly Leu Leu Thr Phe Asp Leu
            100                 105                 110

Ser Ala Tyr Asp His Ala Pro Asp Ser Ala Thr Phe Glu Met Thr Tyr
        115                 120                 125

Leu Gly Tyr Arg Gly Asn Pro Thr Ala Thr Asp Thr Asp Thr Ile Lys
    130                 135                 140

Val Thr Pro Val Asp Thr Thr Val Cys Thr Asn Asn Ala Thr Asp Cys
145                 150                 155                 160

Gly Ala Asn Val Ala Thr Gly Ala Thr Lys Pro Lys Phe Ser Ile Asn
                165                 170                 175

Asp Ser Ser Phe Val Ala Glu Ser Lys Pro Phe Glu Tyr Gly Thr Thr
            180                 185                 190

Val Tyr Thr Gly Asp Ala Ile Thr Val Val Pro Ala Asn Thr Lys Lys
        195                 200                 205

Val Thr Val Asp Val Thr Glu Ile Val Arg Gln Gln Phe Ala Glu Gly
    210                 215                 220

Lys Lys Val Ile Thr Leu Ala Val Gly Glu Thr Lys Lys Thr Glu Val
225                 230                 235                 240

Arg Phe Ala Ser Ser Glu Gly Thr Thr Ser Leu Asn Gly Ala Thr Ala
                245                 250                 255

Asp Met Ala Pro Lys Leu Thr Val Ser Val Ser Thr Lys Asp Asp Leu
            260                 265                 270

Lys Pro Ser Ala Asp Thr Thr Leu Gln Ala Trp Ala Ser Glu Lys Asn
        275                 280                 285

Glu Lys Lys Asn Thr Ala Ala Tyr Val Gly Ala Leu Gln Pro Glu Gly
    290                 295                 300

Asp Tyr Gly Asp Phe Gly Glu Lys Phe Lys Ser Thr Asp Val His Asp
305                 310                 315                 320

Val Thr Asp Ala Lys Met Gly Leu Met Thr Phe Asp Leu Ser Asp Tyr
                325                 330                 335

Thr Ala Ala Pro Glu His Ser Ile Leu Thr Leu Thr Tyr Leu Gly Tyr
            340                 345                 350

Ala Gly Ala Asp Lys Thr Ala Thr Ala Thr Asp Lys Val Lys Val Val
        355                 360                 365

Ala Val Asp Thr Ser Arg Cys Thr Gly Thr Ala Pro Cys Asp Thr Asn

370                 375                 380

Asn Ala Thr Trp Ala Asn Arg Pro Asp Phe Glu Val Thr Asp Thr Thr
385                 390                 395                 400

Lys Thr Ala Thr Ser His Ala Phe Ala Tyr Gly Ser Lys Lys Tyr Ser
                405                 410                 415

Asp Gly Met Thr Val Glu Ser Gly Asn Ala Lys Lys Val Leu Leu Asp
            420                 425                 430

Val Ser Asp Val Ile Lys Ala Glu Phe Ala Lys Phe Ser Ala Gly Ala
        435                 440                 445

Thr Glu Lys Lys Ile Thr Leu Ala Leu Gly Glu Leu Asn Lys Ser Asp
    450                 455                 460

Met Arg Phe Gly Ser Lys Glu Val Thr Ser Leu Thr Gly Ala Thr Glu
465                 470                 475                 480

Ala Met Gln Pro Thr Leu Ser Val Thr Lys Lys Pro Lys Ala Tyr Thr
                485                 490                 495

Leu Ser Ile Glu Gly Pro Thr Lys Val Lys Tyr Gln Lys Gly Glu Ala
            500                 505                 510

Phe Asp Lys Ala Gly Leu Val Val Lys Ala Thr Ser Thr Ala Asp Gly
        515                 520                 525

Thr Val Lys Thr Leu Thr Glu Gly Asn Gly Glu Asp Asn Tyr Thr Ile
    530                 535                 540

Asp Thr Ser Ala Phe Asp Ser Ala Ser Ile Gly Val Tyr Pro Val Thr
545                 550                 555                 560

Val Lys Tyr Asn Lys Asp Pro Glu Ile Ala Ala Ser Phe Asn Ala Tyr
                565                 570                 575

Val Ile Ala Ser Val Glu Asp Gly Gly Asp Gly Asp Thr Ser Lys Asp
            580                 585                 590

Asp Trp Leu Trp Tyr Lys Gln Pro Ala Ser Gln Thr Asp Ala Thr Ala
        595                 600                 605

Thr Ala Gly Gly Asn Tyr Gly Asn Pro Asp Asn Asn Arg Trp Gln Gln
    610                 615                 620

Thr Thr Leu Pro Phe Gly Asn Gly Lys Ile Gly Gly Thr Val Trp Gly
625                 630                 635                 640

Glu Val Ser Arg Glu Arg Val Thr Phe Asn Glu Glu Thr Leu Trp Thr
                645                 650                 655

Gly Gly Pro Gly Ser Ser Thr Ser Tyr Asn Gly Gly Asn Asn Glu Thr
            660                 665                 670

Lys Gly Gln Asn Gly Ala Thr Leu Arg Ala Leu Asn Lys Gln Leu Ala
        675                 680                 685

Asn Gly Ala Glu Thr Val Asn Pro Gly Asn Leu Thr Gly Gly Glu Asn
    690                 695                 700

Ala Ala Glu Gln Gly Asn Tyr Leu Asn Trp Gly Asp Ile Tyr Leu Asp
705                 710                 715                 720

Tyr Gly Phe Asn Asp Thr Thr Val Thr Glu Tyr Arg Arg Asp Leu Asn
                725                 730                 735

Leu Ser Lys Gly Lys Ala Asp Val Thr Phe Lys His Asp Gly Val Thr
            740                 745                 750

Tyr Thr Arg Glu Tyr Phe Ala Ser Asn Pro Asp Asn Val Met Val Ala
        755                 760                 765

Arg Leu Thr Ala Ser Lys Ala Gly Lys Leu Asn Phe Asn Val Ser Met
    770                 775                 780

Pro Thr Asn Thr Asn Tyr Ser Lys Thr Gly Glu Thr Thr Thr Val Lys
785                 790                 795                 800

Gly Asp Thr Leu Thr Val Lys Gly Ala Leu Gly Asn Asn Gly Leu Leu
                805                 810                 815

Tyr Asn Ser Gln Ile Lys Val Val Leu Asp Asn Gly Glu Gly Thr Leu
            820                 825                 830

Ser Glu Gly Ser Asp Gly Ala Ser Leu Lys Val Ser Asp Ala Lys Ala
        835                 840                 845

Val Thr Leu Tyr Ile Ala Ala Ala Thr Asp Tyr Lys Gln Lys Tyr Pro
    850                 855                 860

Ser Tyr Arg Thr Gly Glu Thr Ala Ala Glu Val Asn Thr Arg Val Ala
865                 870                 875                 880

Lys Val Val Gln Asp Ala Ala Asn Lys Gly Tyr Thr Ala Val Lys Lys
                885                 890                 895

Ala His Ile Asp Asp His Ser Ala Ile Tyr Asp Arg Val Lys Ile Asp
            900                 905                 910

Leu Gly Gln Ser Gly His Ser Ser Asp Gly Ala Val Ala Thr Asp Ala
        915                 920                 925

Leu Leu Lys Ala Tyr Gln Arg Gly Ser Ala Thr Thr Ala Gln Lys Arg
    930                 935                 940

Glu Leu Glu Thr Leu Val Tyr Lys Tyr Gly Arg Tyr Leu Thr Ile Gly
945                 950                 955                 960

Ser Ser Arg Glu Asn Ser Gln Leu Pro Ser Asn Leu Gln Gly Ile Trp
                965                 970                 975

Ser Val Thr Ala Gly Asp Asn Ala His Gly Asn Thr Pro Trp Gly Ser
            980                 985                 990

Asp Phe His Met Asn Val Asn Leu Gln Met Asn Tyr Trp Pro Thr Tyr
        995                 1000                1005

Ser Ala Asn Met Gly Glu Leu Ala Glu Pro Leu Ile Glu Tyr Val
    1010                1015                1020

Glu Gly Leu Val Lys Pro Gly Arg Val Thr Ala Lys Val Tyr Ala
    1025                1030                1035

Gly Ala Glu Thr Thr Asn Pro Glu Thr Thr Pro Ile Gly Glu Gly
    1040                1045                1050

Glu Gly Tyr Met Ala His Thr Glu Asn Thr Ala Tyr Gly Trp Thr
    1055                1060                1065

Ala Pro Gly Gln Ser Phe Ser Trp Gly Trp Ser Pro Ala Ala Val
    1070                1075                1080

Pro Trp Ile Leu Gln Asn Val Tyr Glu Ala Tyr Glu Tyr Ser Gly
    1085                1090                1095

Asp Pro Ala Leu Leu Asp Arg Val Tyr Ala Leu Leu Lys Glu Glu
    1100                1105                1110

Ser His Phe Tyr Val Asn Tyr Met Leu His Lys Ala Gly Ser Ser
    1115                1120                1125

Ser Gly Asp Arg Leu Thr Thr Gly Val Ala Tyr Ser Pro Glu Gln
    1130                1135                1140

Gly Pro Leu Gly Thr Asp Gly Asn Thr Tyr Glu Ser Ser Leu Val
    1145                1150                1155

Trp Gln Met Leu Asn Asp Ala Ile Glu Ala Ala Lys Ala Lys Gly
    1160                1165                1170

Asp Pro Asp Gly Leu Val Gly Asn Thr Thr Asp Cys Ser Ala Asp
    1175                1180                1185

Asn Trp Ala Lys Asn Asp Ser Gly Asn Phe Thr Asp Ala Asn Ala
    1190                1195                1200

Asn Arg Ser Trp Ser Cys Ala Lys Ser Leu Leu Lys Pro Ile Glu
    1205                1210                1215

Val Gly Asp Ser Gly Gln Ile Lys Glu Trp Tyr Phe Glu Gly Ala
    1220                1225                1230

Leu Gly Lys Lys Lys Asp Gly Ser Thr Ile Ser Gly Tyr Gln Ala
    1235                1240                1245

Asp Asn Gln His Arg His Met Ser His Leu Leu Gly Leu Phe Pro
    1250                1255                1260

Gly Asp Leu Ile Thr Ile Asp Asn Ser Glu Tyr Met Asp Ala Ala
    1265                1270                1275

Lys Thr Ser Leu Arg Tyr Arg Cys Phe Lys Gly Asn Val Leu Gln
    1280                1285                1290

Ser Asn Thr Gly Trp Ala Ile Gly Gln Arg Ile Asn Ser Trp Ala
    1295                1300                1305

Arg Thr Gly Asp Gly Asn Thr Thr Tyr Gln Leu Val Glu Leu Gln
    1310                1315                1320

Leu Lys Asn Ala Met Tyr Ala Asn Leu Phe Asp Tyr His Ala Pro
    1325                1330                1335

Phe Gln Ile Asp Gly Asn Phe Gly Asn Thr Ser Gly Val Asp Glu
    1340                1345                1350

Met Leu Leu Gln Ser Asn Ser Thr Phe Thr Asp Thr Ala Gly Lys

1355                1360                1365

Lys Tyr Val Asn Tyr Thr Asn Ile Leu Pro Ala Leu Pro Asp Ala
    1370                1375                1380

Trp Ala Gly Gly Ser Val Ser Gly Leu Val Ala Arg Gly Asn Phe
    1385                1390                1395

Thr Val Gly Thr Thr Trp Lys Asn Gly Lys Ala Thr Glu Val Arg
    1400                1405                1410

Leu Thr Ser Asn Lys Gly Lys Gln Ala Ala Val Lys Ile Thr Ala
    1415                1420                1425

Gly Gly Ala Gln Asn Tyr Glu Val Lys Asn Gly Asp Thr Ala Val
    1430                1435                1440

Asn Ala Lys Val Val Thr Asn Ala Asp Gly Ala Ser Leu Leu Val
    1445                1450                1455

Phe Asp Thr Thr Ala Gly Thr Thr Tyr Thr Ile Thr Lys Lys Ala
    1460                1465                1470

Ser Ala Asn Val Pro Val Thr Gly Val Thr Val Thr Gly Ala Asn
    1475                1480                1485

Thr Ala Thr Ala Gly Asp Thr Val Thr Leu Thr Ala Thr Val Ala
    1490                1495                1500

Pro Ala Asn Ala Thr Asp Lys Ser Val Thr Trp Ser Thr Ser Asp
    1505                1510                1515

Ala Ala Val Ala Thr Val Asn Ala Asn Gly Val Val Thr Thr Lys
    1520                1525                1530

Lys Ala Gly Lys Val Thr Ile Thr Ala Thr Ser Asn Gly Asp Lys
    1535                1540                1545

Thr Lys Phe Gly Ser Ile Glu Ile Thr Val Ser Ala Ala Thr Val
    1550                1555                1560

Pro Val Thr Ser Val Thr Val Ala Gly Asp Ala Ala Met Thr Val
    1565                1570                1575

Asp Gly Glu Gln Thr Leu Thr Ala Thr Val Ala Pro Ala Thr Ala
    1580                1585                1590

Thr Asp Lys Thr Val Thr Trp Lys Ser Ser Asp Ala Thr Val Ala
    1595                1600                1605

Thr Val Asp Ala Asn Gly Lys Val Val Ala Lys Lys Ala Gly Glu
    1610                1615                1620

Val Thr Ile Thr Ala Thr Ala Gly Gly Val Ser Gly Thr Leu Lys
    1625                1630                1635

Ile Thr Val Ser Asp Lys Ala Pro Thr Val Ile Pro Val Gln Ser
    1640                1645                1650

Val Thr Val Thr Gly Lys Gln Glu Leu Val Glu Gly Ala Ser Thr
    1655                1660                1665

Thr Leu Thr Ala Thr Val Ala Pro Ala Asp Ala Thr Asp Lys Thr
    1670                1675                1680

Val Thr Trp Lys Ser Ser Asp Glu Ser Val Ala Thr Val Asp Lys
    1685                1690                1695

Asp Gly Val Val Thr Ala Lys Lys Ala Gly Thr Val Thr Ile Thr
    1700                1705                1710

Ala Thr Ala Gly Gly Val Ser Gly Thr Leu His Ile Thr Val Thr
    1715                1720                1725

Ala Lys Pro Val Glu Thr Val Pro Val Thr Ser Val Glu Val Thr
    1730                1735                1740

Val Glu Ala Gly Thr Thr Val Ser Val Gly Lys Thr Leu Gln Ala
    1745                1750                1755

Thr Ala Thr Val Lys Pro Gly Asn Ala Thr Asn Lys Lys Val Thr
    1760                1765                1770

Trp Lys Ser Ser Asp Glu Ser Ile Ala Thr Val Asp Ala Asn Gly
    1775                1780                1785

Val Ile Thr Ala Lys Lys Ala Gly Lys Val Val Ile Thr Ala Thr
    1790                1795                1800

Ser Thr Asp Gly Thr Asp Lys Ser Gly Ser Val Glu Ile Thr Val
    1805                1810                1815

Val Asp Glu Thr Lys Pro Thr Pro Asp His Lys Ser Val Lys Ala
    1820                1825                1830

Asp Thr Gly Asp Val Thr Ala Gly Lys Thr Gly Thr Val Thr Glu
    1835                1840                1845

Pro Lys Asp Val Ala Gly Trp Lys Ser Arg Ser Ile Ile Lys Gln
    1850                1855                1860

Gly Lys Leu Gly Lys Ala Glu Ile Ala Asp Gly Thr Leu Val Tyr
    1865                1870                1875

Ala Ala Gly Asp Lys Thr Gly Asp Asp Ser Phe Val Val Gln Tyr
    1880                1885                1890

Thr Met Ala Asp Gly Thr Val Ile Asp Val Thr Tyr Ser Val Thr
    1895                1900                1905

Val Lys Ala Ala Glu Thr Gly Lys Asn Asp Gly Asp Gly Lys Gly
    1910                1915                1920

Asp Gly Val Ala Lys Thr Gly Ala Ala Val Gly Ala Leu Ala Gly
    1925                1930                1935

Leu Gly Leu Met Leu Leu Ala Val Gly Val Ser Val Val Met Ile
    1940                1945                1950

Arg Arg Lys His Ser Ala
    1955

<210> 3
<211> 4482
<212> DNA
<213> Bifidobacterium bifidum

<400> 3
atgctacaca cagcatcaag aggatgctcg cgttcgtggc tgcgcagact caccgcattg 60

atagcggtct cggcgctcgc gttcgtggca ttgccgaacg tcgcggtggc ggcggatccg 120

atggaatacc tcgatgtgtc gttcggcggc acgttcgctg cagacaccta caccacaggt 180

ggcgacgagg tggcgaaggg ccccgtgacc aagcacggca gcataccgac caagcttgac 240

ggcggcggca tcaccctcgc tggcggcacc aacggcgtga cattcacctc gaccgcgagc 300

ttcagcgaga gtgggaaggt gaacaaggga ttccgcgccg aaatggagta ccgtacgacg 360

cagacgccca gcaacctcgc cacattgttc tccgccatgg gcaacatctt cgtgcgggcg 420

aacggcagca acctcgaata cggcttctcc acgaaccctt ccggcagtac atggaacgac 480

tacacaaagt ccgtgacgct gccttccaac aatgtgaagc acatcatcca gctgacatat 540

ctgccgggag ccgacggcgc tgcctcgacg ttgcagttgt cggtggatgg cgtggccggc 600

gagaccgcca cctccgcggc cggcgagctc gcggccgtca gcgattccgt cgggaacaag 660
ttcgggatcg gctacgaggt gaaccccgct tccggcgcgg cgagccgcgg tcttgccggt 720
gacgtgttcc gcgcgcgtgt cgccgattcg gacgccccgt gggagattct tgacgcatcc 780
cagctgctgc atgtcaattt caacggcacg ttcagcggca cctcatatac cgcggcgagc 840
ggcgagcaga tgctgggctc gctggtgtcg cgctcggcca atccgtccat ctcgaactcc 900
gccgtcacgc tgggcggcgg cacggccgga ttcgatttca cgcccacgga cttcaccctc 960
ggtgacaacg aggccatcac ccgcccgctg gtcgcggagc tgcgcttcac cccgacgcag 1020
accggcgaca accagaccct gttcggcgcg ggcggcaacc tgttcctgcg ctacgagtcg 1080
aacaagctcg tgttcggcgc ctccaccaag tccggcgata attggaccga ccacaagatc 1140
gagtccgcgg ccgccacggg tgcggagcac gtcgtgtcgg tggcgtacgt gcccaataag 1200
gccggcaccg gcgcgaagct tgtcatgcgc gtggatggcg gcgacgccca gaccaaggac 1260
atcactggtc tggcttacct gaattcgagc atcaaggcca aggtcggctt cggcaacgac 1320
gtgcataccg acgcgctcag ccgcggcttc gtcggctcgc tgagcgagat ccgcctggcc 1380
gaaacctccg cgaacttcac caccaacgaa ttcaagctgg tctactctca ggtcagctgc 1440
gacacgtcgg gcatcaagga ggcgaatacc ttcgacgtgg agcccgccga gtgcgaggcc 1500
gcgcttaaga ccaagctgtc caagctgcgt ccgaccgaag ggcaggccga ctacatcgac 1560
tggggtcaga tcggattcct ccattacggc atcaacacgt actacaacca ggagtggggt 1620
cacggtaacg aggatccctc ccgcatcaac ccgaccggcc tcgacaccga ccagtgggcg 1680
aagtccttcg ccgacggtgg cttcaagatg atcatggtga cggtcaagca ccatgacggt 1740
ttcgagctgt acgactcgcg gtacaacacc gagcacgact gggcaaacac cgccgtcgcc 1800
aagcgcacgg gggagaagga cctgttccgc aagattgtcg cctcggcgaa gaaatacggc 1860
ctgaaggtcg gcatctacta ttcgccggcc gattcctaca tggagaggaa gggcgtctgg 1920
ggcaacaact ccgcacgcgt cgagcgcacg atccccacgc tggtggagaa cgacgaccgc 1980
gccggcaagg tggcttccgg caaactgccc acgttcaagt acaaggccac ggattacggc 2040
gcctacatgc tcaaccagct ctatgagctg ctgactgagt acggcgacat ctccgaggtc 2100
tggttcgacg gtgcccaagg caacaccgca ggcactgagc attacgacta tggcgtgttc 2160
tacgagatga tccgccggct tcagccccag gcaattcagg ccaacgccgc atacgatgcc 2220
cgatgggtgg gcaacgagga cggctgggcc cgtcagaccg agtggagccc gcaggcggca 2280
tacaacgacg gcgtggacaa ggtgtcgctc aagcctggcc agatggcccc cgacggtaag 2340
cttggcagca tgtcgagcgt gctgtccgag atccgcagcg gcgccgccaa ccagctgcac 2400
tggtatccgg ccgaagtcga cgccaagaac cggcccggat ggttctaccg tgccagccaa 2460
tcgccggcgt ccgtagccga agtcgtgaag tactacgagc agtccacggg acgcaactcg 2520

cagtatctgc tgaacgtccc accgtccgat accggcaagc tcgccgatgc ggatgccgcg 2580

ggacttaagg ggctgggcga ggagctcgcc cgacgctacg gcaccgatct tgccctgggc 2640

aagagcgcga ccgtcgccgc gtccgcgaac gacactgcgg tagcggcccc gaagctgacc 2700

gacggttcga agctctcctc cgacaaggcc gtgggcaata cgccgacgta caccatcgat 2760

ctgggcagca ctgtcgccgt ggatgcagtg aagatctccg aggacgtgcg caatgccggc 2820

cagcagatcg aaagcgccac tctgcaggga cgagtcaatg gaacatggac gaatctggcg 2880

actatgacga cggtcggcca gcagcgcgac cttcgcttca cgtcccagaa catcgatgcc 2940

atccgtctgg tggtcaactc ctcccgcggt ccggtgcgtc tgagccgtct tgaggtgttc 3000

cacaccgaat ccgagattca gaccggcgcc cgcgcctact acatcgatcc gacggcgcag 3060

accgcgggag atggattcac gaaggacaag cccatgacgt cgatcgagca gctgcacgat 3120

gtgaccgtcg cgccaggctc cgtgatcttc gtcaaggcgg gcaccgagct gaccggggac 3180

ttcgccgtct tcggctacgg caccaaggac gagcccatca ccgtgacgac atacggcgaa 3240

agcgacaaag ccaccaccgc gagcttcgac ggcatgaccg ccgggctgac gctgaagcag 3300

gcgctgaagg cgctcggcaa ggacgacgcc ggctgggtcg tggccgattc cgccactgca 3360

ccggcctccc gcgtgtatgt cccgcaggat gagatcagcg tgcacgccca gtcgtcgcag 3420

aactccggcg cagaggcggc gagggcgctc gacggcgact cgtcgacgag ctggcactcc 3480

cagtacagcc cgaccaccgc gtctgctccg cattgggtga ctctcgatct cggcaaatcg 3540

cgtgagaacg tcgcctactt cgactacctc gcccgtatcg acggcaacaa taacggtgcc 3600

gccaaggatt acgaggtgta tgtctccgac gatcccaacg attttggagc ccctgtggcc 3660

tcgggcacgt tgaagaacgt cgcctacacg cagcgcatca agctgacccc caagaacgga 3720

cggtacgtca agttcgtcat caagaccgat tattccggat cgaacttcgg ctccgcggcg 3780

gaaatgaatg tcgagttgct gcccacggcc gtagaggagg acaaggtcgc caccccgcag 3840

aagccgacag tggacgatga tgccgataca tacaccatcc ccgacatcga gggagtcgtg 3900

tacaaggtcg acggcaaggt gttggccgct ggttccgtag tgaacgtggg cgatgaggac 3960

gtgaccgtca cggtcaccgc cgagcccgcc gacggatacc gcttcccgga tggtgtgacg 4020

tccccagtca cgtatgagct gacgttcacc aagaagggtg gcgagaagcc tccgaccgaa 4080

gtcaacaagg acaagctgca cgccacgatc accaaggctc aggcgatcga ccgttccgcc 4140

tatacggacg agtcgctcaa ggtgcttgat gacaagctcg ccgcagcgct caaggtctat 4200

gacgatgaca aggtgagcca ggatgatgtc gatgccgccg aggcggctct gtctgcggcg 4260

atcgacgcgc tgaagaccaa gccgacgacc cccggcggtg aaggtgagaa gcctggtgaa 4320

ggtgaaaagc ccggtgacgg caacaagccc ggtgacggca agaagcccgg cgacgtgatc 4380

gcaaagaccg gcgcctccac aatgggcgtt gtcttcgctg cactcgcgat ggtagcgggt 4440

gcggtcgtga cgcttgaagc caagcgtaag tccaaccggt aa 4482

<210> 4
<211> 1493
<212> PRT
<213> Bifidobacterium bifidum

<400> 4

Met Leu His Thr Ala Ser Arg Gly Cys Ser Arg Ser Trp Leu Arg Arg
1               5                   10                  15

Leu Thr Ala Leu Ile Ala Val Ser Ala Leu Ala Phe Val Ala Leu Pro
            20                  25                  30

Asn Val Ala Val Ala Ala Asp Pro Met Glu Tyr Leu Asp Val Ser Phe
        35                  40                  45

Gly Gly Thr Phe Ala Ala Asp Thr Tyr Thr Thr Gly Gly Asp Glu Val
    50                  55                  60

Ala Lys Gly Pro Val Thr Lys His Gly Ser Ile Pro Thr Lys Leu Asp
65                  70                  75                  80

Gly Gly Gly Ile Thr Leu Ala Gly Gly Thr Asn Gly Val Thr Phe Thr
                85                  90                  95

Ser Thr Ala Ser Phe Ser Glu Ser Gly Lys Val Asn Lys Gly Phe Arg
            100                 105                 110

Ala Glu Met Glu Tyr Arg Thr Thr Gln Thr Pro Ser Asn Leu Ala Thr
        115                 120                 125

Leu Phe Ser Ala Met Gly Asn Ile Phe Val Arg Ala Asn Gly Ser Asn
    130                 135                 140

Leu Glu Tyr Gly Phe Ser Thr Asn Pro Ser Gly Ser Thr Trp Asn Asp
145                 150                 155                 160

Tyr Thr Lys Ser Val Thr Leu Pro Ser Asn Asn Val Lys His Ile Ile
                165                 170                 175

Gln Leu Thr Tyr Leu Pro Gly Ala Asp Gly Ala Ala Ser Thr Leu Gln
            180                 185                 190

Leu Ser Val Asp Gly Val Ala Gly Glu Thr Ala Thr Ser Ala Ala Gly
        195                 200                 205

Glu Leu Ala Ala Val Ser Asp Ser Val Gly Asn Lys Phe Gly Ile Gly
    210                 215                 220

Tyr Glu Val Asn Pro Ala Ser Gly Ala Ala Ser Arg Gly Leu Ala Gly
225                 230                 235                 240

Asp Val Phe Arg Ala Arg Val Ala Asp Ser Asp Ala Pro Trp Glu Ile
                245                 250                 255

Leu Asp Ala Ser Gln Leu Leu His Val Asn Phe Asn Gly Thr Phe Ser
            260                 265                 270

Gly Thr Ser Tyr Thr Ala Ala Ser Gly Glu Gln Met Leu Gly Ser Leu
        275                 280                 285

Val Ser Arg Ser Ala Asn Pro Ser Ile Ser Asn Ser Ala Val Thr Leu
    290                 295                 300

Gly Gly Gly Thr Ala Gly Phe Asp Phe Thr Pro Thr Asp Phe Thr Leu
305                 310                 315                 320

Gly Asp Asn Glu Ala Ile Thr Arg Pro Leu Val Ala Glu Leu Arg Phe
                325                 330                 335

Thr Pro Thr Gln Thr Gly Asp Asn Gln Thr Leu Phe Gly Ala Gly Gly
            340                 345                 350

Asn Leu Phe Leu Arg Tyr Glu Ser Asn Lys Leu Val Phe Gly Ala Ser
        355                 360                 365

Thr Lys Ser Gly Asp Asn Trp Thr Asp His Lys Ile Glu Ser Ala Ala
    370                 375                 380

Ala Thr Gly Ala Glu His Val Val Ser Val Ala Tyr Val Pro Asn Lys
385                 390                 395                 400

Ala Gly Thr Gly Ala Lys Leu Val Met Arg Val Asp Gly Gly Asp Ala
                405                 410                 415

Gln Thr Lys Asp Ile Thr Gly Leu Ala Tyr Leu Asn Ser Ser Ile Lys
            420                 425                 430

Gly Lys Val Gly Phe Gly Asn Asp Val His Thr Asp Ala Leu Ser Arg
        435                 440                 445

Gly Phe Val Gly Ser Leu Ser Glu Ile Arg Leu Ala Glu Thr Ser Ala
    450                 455                 460

Asn Phe Thr Thr Asn Glu Phe Lys Leu Val Tyr Ser Gln Val Ser Cys
465                 470                 475                 480

Asp Thr Ser Gly Ile Lys Glu Ala Asn Thr Phe Asp Val Glu Pro Ala
                485                 490                 495

Glu Cys Glu Ala Ala Leu Lys Thr Lys Leu Ser Lys Leu Arg Pro Thr
            500                 505                 510

Glu Gly Gln Ala Asp Tyr Ile Asp Trp Gly Gln Ile Gly Phe Leu His
        515                 520                 525

Tyr Gly Ile Asn Thr Tyr Tyr Asn Gln Glu Trp Gly His Gly Asn Glu
    530                 535                 540

Asp Pro Ser Arg Ile Asn Pro Thr Gly Leu Asp Thr Asp Gln Trp Ala
545                 550                 555                 560

Lys Ser Phe Ala Asp Gly Gly Phe Lys Met Ile Met Val Thr Val Lys
                565                 570                 575

His His Asp Gly Phe Glu Leu Tyr Asp Ser Arg Tyr Asn Thr Glu His
            580                 585                 590

Asp Trp Ala Asn Thr Ala Val Ala Lys Arg Thr Gly Glu Lys Asp Leu
        595                 600                 605

Phe Arg Lys Ile Val Ala Ser Ala Lys Lys Tyr Gly Leu Lys Val Gly
    610                 615                 620

Ile Tyr Tyr Ser Pro Ala Asp Ser Tyr Met Glu Arg Lys Gly Val Trp
625                 630                 635                 640

Gly Asn Asn Ser Ala Arg Val Glu Arg Thr Ile Pro Thr Leu Val Glu
                645                 650                 655

Asn Asp Asp Arg Ala Gly Lys Val Ala Ser Gly Lys Leu Pro Thr Phe
            660                 665                 670

Lys Tyr Lys Ala Thr Asp Tyr Gly Ala Tyr Met Leu Asn Gln Leu Tyr
        675                 680                 685

Glu Leu Leu Thr Glu Tyr Gly Asp Ile Ser Glu Val Trp Phe Asp Gly
    690                 695                 700

Ala Gln Gly Asn Thr Ala Gly Thr Glu His Tyr Asp Tyr Gly Val Phe

705                 710                 715                 720

Tyr Glu Met Ile Arg Arg Leu Gln Pro Gln Ala Ile Gln Ala Asn Ala
                725                 730                 735

Ala Tyr Asp Ala Arg Trp Val Gly Asn Glu Asp Gly Trp Ala Arg Gln
            740                 745                 750

Thr Glu Trp Ser Pro Gln Ala Ala Tyr Asn Asp Gly Val Asp Lys Val
        755                 760                 765

Ser Leu Lys Pro Gly Gln Met Ala Pro Asp Gly Lys Leu Gly Ser Met
    770                 775                 780

Ser Ser Val Leu Ser Glu Ile Arg Ser Gly Ala Ala Asn Gln Leu His
785                 790                 795                 800

Trp Tyr Pro Ala Glu Val Asp Ala Lys Asn Arg Pro Gly Trp Phe Tyr
                805                 810                 815

Arg Ala Ser Gln Ser Pro Ala Ser Val Ala Glu Val Val Lys Tyr Tyr
            820                 825                 830

Glu Gln Ser Thr Gly Arg Asn Ser Gln Tyr Leu Leu Asn Val Pro Pro
        835                 840                 845

Ser Asp Thr Gly Lys Leu Ala Asp Ala Asp Ala Ala Gly Leu Lys Gly
    850                 855                 860

Leu Gly Glu Glu Leu Ala Arg Arg Tyr Gly Thr Asp Leu Ala Leu Gly
865                 870                 875                 880

Lys Ser Ala Thr Val Ala Ala Ser Ala Asn Asp Thr Ala Val Ala Ala
                885                 890                 895

Pro Lys Leu Thr Asp Gly Ser Lys Leu Ser Ser Asp Lys Ala Val Gly
            900                 905                 910

Asn Thr Pro Thr Tyr Thr Ile Asp Leu Gly Ser Thr Val Ala Val Asp
        915                 920                 925

Ala Val Lys Ile Ser Glu Asp Val Arg Asn Ala Gly Gln Gln Ile Glu
    930                 935                 940

Ser Ala Thr Leu Gln Gly Arg Val Asn Gly Thr Trp Thr Asn Leu Ala
945                 950                 955                 960

Thr Met Thr Thr Val Gly Gln Gln Arg Asp Leu Arg Phe Thr Ser Gln
                965                 970                 975

Asn Ile Asp Ala Ile Arg Leu Val Val Asn Ser Ser Arg Gly Pro Val
            980                 985                 990

Arg Leu Ser Arg Leu Glu Val Phe His Thr Glu Ser Glu Ile Gln Thr
        995                 1000                1005

Gly Ala Arg Ala Tyr Tyr Ile Asp Pro Thr Ala Gln Thr Ala Gly
    1010                1015                1020

Asp Gly Phe Thr Lys Asp Lys Pro Met Thr Ser Ile Glu Gln Leu
    1025                1030                1035

His Asp Val Thr Val Ala Pro Gly Ser Val Ile Phe Val Lys Ala
    1040                1045                1050

Gly Thr Glu Leu Thr Gly Asp Phe Ala Val Phe Gly Tyr Gly Thr
    1055                1060                1065

Lys Asp Glu Pro Ile Thr Val Thr Thr Tyr Gly Glu Ser Asp Lys
    1070                1075                1080

Ala Thr Thr Ala Ser Phe Asp Gly Met Thr Ala Gly Leu Thr Leu
    1085                1090                1095

Lys Gln Ala Leu Lys Ala Leu Gly Lys Asp Asp Ala Gly Trp Val
    1100                1105                1110

Val Ala Asp Ser Ala Thr Ala Pro Ala Ser Arg Val Tyr Val Pro
    1115                1120                1125

Gln Asp Glu Ile Ser Val His Ala Gln Ser Ser Gln Asn Ser Gly
    1130                1135                1140

Ala Glu Ala Ala Arg Ala Leu Asp Gly Asp Ser Ser Thr Ser Trp
    1145                1150                1155

His Ser Gln Tyr Ser Pro Thr Thr Ala Ser Ala Pro His Trp Val
    1160                1165                1170

Thr Leu Asp Leu Gly Lys Ser Arg Glu Asn Val Ala Tyr Phe Asp
    1175                1180                1185

Tyr Leu Ala Arg Ile Asp Gly Asn Asn Asn Gly Ala Ala Lys Asp
    1190                1195                1200

Tyr Glu Val Tyr Val Ser Asp Asp Pro Asn Asp Phe Gly Ala Pro
    1205                1210                1215

Val Ala Ser Gly Thr Leu Lys Asn Val Ala Tyr Thr Gln Arg Ile
    1220                1225                1230

Lys Leu Thr Pro Lys Asn Gly Arg Tyr Val Lys Phe Val Ile Lys
    1235                1240                1245

Thr Asp Tyr Ser Gly Ser Asn Phe Gly Ser Ala Ala Glu Met Asn
    1250                1255                1260

Val Glu Leu Leu Pro Thr Ala Val Glu Glu Asp Lys Val Ala Thr
    1265                1270                1275

Pro Gln Lys Pro Thr Val Asp Asp Asp Ala Asp Thr Tyr Thr Ile
    1280                1285                1290

Pro Asp Ile Glu Gly Val Val Tyr Lys Val Asp Gly Lys Val Leu
    1295                1300                1305

Ala Ala Gly Ser Val Val Asn Val Gly Asp Glu Asp Val Thr Val
    1310                1315                1320

Thr Val Thr Ala Glu Pro Ala Asp Gly Tyr Arg Phe Pro Asp Gly
    1325                1330                1335

Val Thr Ser Pro Val Thr Tyr Glu Leu Thr Phe Thr Lys Lys Gly
    1340                1345                1350

Gly Glu Lys Pro Pro Thr Glu Val Asn Lys Asp Lys Leu His Ala
    1355                1360                1365

Thr Ile Thr Lys Ala Gln Ala Ile Asp Arg Ser Ala Tyr Thr Asp
    1370                1375                1380

Glu Ser Leu Lys Val Leu Asp Asp Lys Leu Ala Ala Ala Leu Lys
    1385                1390                1395

Val Tyr Asp Asp Asp Lys Val Ser Gln Asp Asp Val Asp Ala Ala
    1400                1405                1410

Glu Ala Ala Leu Ser Ala Ala Ile Asp Ala Leu Lys Thr Lys Pro
    1415                1420                1425

Thr Thr Pro Gly Gly Glu Gly Glu Lys Pro Gly Glu Gly Glu Lys
    1430                1435                1440

Pro Gly Asp Gly Asn Lys Pro Gly Asp Gly Lys Lys Pro Gly Asp
    1445                1450                1455

Val Ile Ala Lys Thr Gly Ala Ser Thr Met Gly Val Val Phe Ala
    1460                1465                1470

Ala Leu Ala Met Val Ala Gly Ala Val Val Thr Leu Glu Ala Lys
    1475                1480                1485

Arg Lys Ser Asn Arg
    1490

<210> 5
<211> 2094
<212> DNA
<213> Streptococcus pneumoniae

<400> 5
atgaataaaa gaggtcttta ttcaaaacta ggaatttctg ttgtaggcat tagtctttta      60

atgggagtcc ccactttgat tcatgcgaat gaattaaact atggtcaact gtccatatct     120

cctatttttc aaggaggttc atatcaactg aacaataaga gtatagatat cagctctttg     180

ttattagata aattgtctgg agagagtcag acagtagtaa tgaaatttaa agcagataaa     240

ccaaactctc ttcaagcttt gtttggccta tctaatagta aagcaggctt taaaaataat     300

tacttttcaa ttttcatgag agattctggt gagataggtg tagaaataag agacgcccaa     360

gagggaataa attatttatt ttctagacca gcttcattat ggggaaagca taaaggacag     420

gcagttgaaa atacactagt atttgtatct gattctaaag ataaaacata cacaatgtat     480

gttaatggaa tagaagtgtt ctctgaaaca gttgatacat ttttgccaat ttcaaatata     540

aatggtatag ataaggcaac actaggagct gttaatcgtg aaggtaagga acattacctc     600

gcaaaaggaa gtattggtga aatcagtcta tttaacaaag caattagtga tcaggaagtt     660

tcaaatattc ccttgtcaaa tccatttcag ttaattttcc aatcaggaga ttctactcaa     720

gctaactatt ttagaatacc gacactatat acattaagta gtggaagagt tctatcaagt     780

attgatgcac gttatggtgg gactcatgat tctaaaagta agattaatat tgccacttct     840

tatagtgatg ataatgggaa aacgtggagt gagccaattt ttgctatgaa gtttaatgac     900

tatgaggagc agttagttta ctggccacga gataataaat taaagaatag tcaaattagt     960

ggaagtgctt cattcataga ttcatccatt gttgaagata aaaaatctgg gaaaacgata    1020

ttactagctg atgttatgcc tgcgggtatt ggaaataata atgcaaataa agccgactca    1080

ggttttaaag aaataaatgg tcattattat ttaaaactaa agaagaatgg agataacgat    1140

ttccgttata cagttagaga aaatggtgtc gtttatgatg aaacaactaa taaacctaca    1200

aattatacta taaatgataa gtatgaagtt ttggagggag gaaagtcttt aacagtcgaa 1260

caatattcgg ttgattttga tagtggctct ttaagagaaa ggcataatgg aaaacaggtt 1320

cctatgaatg ttttctacaa agattcgtta tttaaagtga ctcctactaa ttatatagca 1380

atgacaacta gtcagaatag aggagagagt tgggaacaat ttaagttgtt gcctccgttc 1440

ttaggagaaa aacataatgg aacttacttg tgtcctggac aaggtttagc attaaaatca 1500

agtaacagat tgatttttgc aacatatact agtggagaac taacctatct catttcggat 1560

gatagtggtc aaacatggaa gaaatcctca gcttcaattc cgtttaaaaa tgcaacagca 1620

gaagcacaaa tggttgaact gagagatggt gtgattagaa cattctttag aaccactaca 1680

ggtaagatag cttatatgac tagtagagat tctggagaaa catggtcgaa agtttcgtat 1740

attgatggaa ttcaacaaac ttcatatggc acacaagtat ctgcaattaa atactctcaa 1800

ttaattgatg gaaaagaagc agtcattttg agtacaccaa attctagaag tggccgtaag 1860

ggaggccaat tagttgtcgg tttggtcaat aaagaagatg atagtattga ttggagatac 1920

cactatgata ttgatttgcc ttcgtatggt tatgcctatt ctgcgattac agaattgcca 1980

aatcatcaca taggtgtact gtttgaaaaa tatgattcgt ggtcgagaaa tgaattgcat 2040

ttaagcaatg tagttcagta tatagatttg gaaattaatg atttaacaaa ataa 2094

<210> 6
<211> 697
<212> PRT
<213> Streptococcus pneumoniae

<400> 6

Met Asn Lys Arg Gly Leu Tyr Ser Lys Leu Gly Ile Ser Val Val Gly
1               5                   10                  15

Ile Ser Leu Leu Met Gly Val Pro Thr Leu Ile His Ala Asn Glu Leu
            20                  25                  30

Asn Tyr Gly Gln Leu Ser Ile Ser Pro Ile Phe Gln Gly Gly Ser Tyr
        35                  40                  45

Gln Leu Asn Asn Lys Ser Ile Asp Ile Ser Ser Leu Leu Leu Asp Lys
    50                  55                  60

Leu Ser Gly Glu Ser Gln Thr Val Val Met Lys Phe Lys Ala Asp Lys
65                  70                  75                  80

Pro Asn Ser Leu Gln Ala Leu Phe Gly Leu Ser Asn Ser Lys Ala Gly
                85                  90                  95

Phe Lys Asn Asn Tyr Phe Ser Ile Phe Met Arg Asp Ser Gly Glu Ile
            100                 105                 110

Gly Val Glu Ile Arg Asp Ala Gln Lys Gly Ile Asn Tyr Leu Phe Ser
        115                 120                 125

Arg Pro Ala Ser Leu Trp Gly Lys His Lys Gly Gln Ala Val Glu Asn
    130                 135                 140

Thr Leu Val Phe Val Ser Asp Ser Lys Asp Lys Thr Tyr Thr Met Tyr
145                 150                 155                 160

Val Asn Gly Ile Glu Val Phe Ser Glu Thr Val Asp Thr Phe Leu Pro
                165                 170                 175

Ile Ser Asn Ile Asn Gly Ile Asp Lys Ala Thr Leu Gly Ala Val Asn
            180                 185                 190

Arg Glu Gly Lys Glu His Tyr Leu Ala Lys Gly Ser Ile Asp Glu Ile
        195                 200                 205

Ser Leu Phe Asn Lys Ala Ile Ser Asp Gln Glu Val Ser Thr Ile Pro
    210                 215                 220

Leu Ser Asn Pro Phe Gln Leu Ile Phe Gln Ser Gly Asp Ser Thr Gln
225                 230                 235                 240

Ala Asn Tyr Phe Arg Ile Pro Thr Leu Tyr Thr Leu Ser Ser Gly Arg
                245                 250                 255

Val Leu Ser Ser Ile Asp Ala Arg Tyr Gly Gly Thr His Asp Ser Lys
            260                 265                 270

Ser Lys Ile Asn Ile Ala Thr Ser Tyr Ser Asp Asp Asn Gly Lys Thr
        275                 280                 285

Trp Ser Glu Pro Ile Phe Ala Met Lys Phe Asn Asp Tyr Glu Glu Gln
    290                 295                 300

Leu Val Tyr Trp Pro Arg Asp Asn Lys Leu Lys Asn Ser Gln Ile Ser
305                 310                 315                 320

Gly Ser Ala Ser Phe Ile Asp Ser Ser Ile Val Glu Asp Lys Lys Ser
                325                 330                 335

Gly Lys Thr Ile Leu Leu Ala Asp Val Met Pro Ala Gly Ile Gly Asn
            340                 345                 350

Asn Asn Ala Asn Lys Ala Asp Ser Gly Phe Lys Glu Ile Asn Gly His
        355                 360                 365

Tyr Tyr Leu Lys Leu Lys Lys Asn Gly Asp Asn Asp Phe Arg Tyr Thr
    370                 375                 380

Val Arg Glu Asn Gly Val Val Tyr Asn Glu Thr Thr Asn Lys Pro Thr
385                 390                 395                 400

Asn Tyr Thr Ile Asn Asp Lys Tyr Glu Val Leu Glu Gly Gly Lys Ser
                405                 410                 415

Leu Thr Val Glu Gln Tyr Ser Val Asp Phe Asp Ser Gly Ser Leu Arg
            420                 425                 430

Glu Arg His Asn Gly Lys Gln Val Pro Met Asn Val Phe Tyr Lys Asp
        435                 440                 445

Ser Leu Phe Lys Val Thr Pro Thr Asn Tyr Ile Ala Met Thr Thr Ser
    450                 455                 460

Gln Asn Arg Gly Glu Ser Trp Glu Gln Phe Lys Leu Leu Pro Pro Phe
465                 470                 475                 480

Leu Gly Glu Lys His Asn Gly Thr Tyr Leu Cys Pro Gly Gln Gly Leu
                485                 490                 495

Ala Leu Lys Ser Ser Asn Arg Leu Ile Phe Ala Thr Tyr Thr Ser Gly
            500                 505                 510

Glu Leu Thr Tyr Leu Ile Ser Asp Asp Ser Gly Gln Thr Trp Lys Lys
        515                 520                 525

Ser Ser Ala Ser Ile Pro Phe Lys Asn Ala Thr Ala Glu Ala Gln Met
    530                 535                 540

Val Glu Leu Arg Asp Gly Val Ile Arg Thr Phe Phe Arg Thr Thr Thr
545                 550                 555                 560

Gly Lys Ile Ala Tyr Met Thr Ser Arg Asp Ser Gly Glu Thr Trp Ser
                565                 570                 575

Lys Val Ser Tyr Ile Asp Gly Ile Gln Gln Thr Ser Tyr Gly Thr Gln
            580                 585                 590

Val Ser Ala Ile Lys Tyr Ser Gln Leu Ile Asp Gly Lys Glu Ala Val
        595                 600                 605

Ile Leu Ser Thr Pro Asn Ser Arg Ser Gly Arg Lys Gly Gly Gln Leu
    610                 615                 620

Val Val Gly Leu Val Asn Lys Glu Asp Asp Ser Ile Asp Trp Lys Tyr
625                 630                 635                 640

His Tyr Asp Ile Asp Leu Pro Ser Tyr Gly Tyr Ala Tyr Ser Ala Ile
                645                 650                 655

Thr Glu Leu Pro Asn His His Ile Gly Val Leu Phe Glu Lys Tyr Asp
            660                 665                 670

Ser Trp Ser Arg Asn Glu Leu His Leu Ser Asn Val Val Gln Tyr Ile
        675                 680                 685

Asp Leu Glu Ile Asn Asp Leu Thr Lys
    690                 695

<210>  7
<211>  2076
<212>  DNA
<213>  Bifidobacterium longum

<400>  7
atggaacata gagcgttcaa gtggccgcag ccacttgcgg gcaacaagcc ccgcatctgg      60

tacggcggcg attacaaccc cgaccaatgg cctgaggaag tgtgggacga agatgtagcc     120

ctcatgcagc aggccggcgt caacctcgtc tccgtagcca tcttctcctg ggccaagctt     180

gagcccgaag aaggcgtgta cgacttcgat tggctcgacc gcgtcatcga caagctcggc     240

aaggccggca tcgccgtcga tctcgcctcc ggcaccgcat ccccgccgat gtggatgacc     300

caggcccacc cggagatcct ctgggtcgac taccgcggcg acgtctgcca gcccggtgcc     360

cgccagcact ggcgcgccac cagcccggtc ttccttgact acgcgctcaa cctgtgccgc     420

aagatggccg agcactacaa ggacaacccc tatgtggtct cttggcatgt gagcaacgag     480

tacggctgcc acaaccgctt cgactattcc gaagacgccg agcgcgcctt ccagaagtgg     540

tgcgagaaga agtacggcac catcgacgct gtcaacgacg cctggggcac cgccttctgg     600

gcgcagcgca tgaacaattt ctccgagatc atcccgccgc gattcatcgg cgacggcaac     660

ttcatgaacc cgggcaagct gcttgattgg aagcgtttca gctccgacgc gctgctggac     720

ttctacaagg ccgagcgcga cgccctgctc gagatcgccc ccaagccgca gaccaccaac     780

ttcatggtct ccgcgggctg caccgtcctc gactacgaca agtggggtca tgacgtggac     840

ttcgtgtcca acgaccatta cttctcgccc ggcgaggccc acttcgacga gatggcctac     900

gcggcctgcc tcaccgacgg catcgcccgc aagaacccgt ggttcctcat ggaacattcc     960

acgtccgccg tcaactggcg cccgaccaac taccggctcg agcccggcga gctggtgcgc 1020

gactccctgg cccatctggc catgggcgcc gacgccatct gctacttcca gtggcgtcag 1080

tccaaggccg gcgccgagaa gtggcattcc gccatggtgc cccacgcagg ccccgactcc 1140

cagatcttcc gcgatgtgtg cgagctgggt gccgacctca acaagcttgc tgacgagggc 1200

ctgctgagca ccaagctggt caagtccaag gtcgccatcg tcttcgacta cgagtcccag 1260

tgggccaccg agcacaccgc cacccccacg caggaggtgc gccactggac cgagccgctg 1320

gactggttcc gcgcgctggc ggacaatggc ctgaccgccg acgtggtgcc ggtccgcggt 1380

ccttgggatg agtacgaggc cgtcgtgttg ccgagcctgg ccatcctgtc cgagcagacc 1440

acgcgccgcg tgcgcgagta tgtggcgaac ggcggcaagc tgttcgtgac ctactacacc 1500

ggtctggtgg acgacaggga tcacgtctgg ctgggcggct accccggctc cattcgcgac 1560

gtggtgggcg tgcgcgtcga ggaattcgcc ccgatgggca ccgacgcccc cggcaccatg 1620

gaccaccttg acttggacaa cggaaccgtg gcgcacgatt tcgccgacgt gatcacctcc 1680

gtggccgata ccgctcacgt ggtcgcctcc ttcaaggcag ataagtggac cggtttcgac 1740

ggcgctcccg ccatcaccgt caacgacttc ggcgacggca aggccgcata cgtcggtgcc 1800

cgtctcgggc gtgagggctt ggccaagagc ctgcccgcgc tgctggagga actcggcatc 1860

gagacttcgg ctgaggacga tcgtggtgaa gtgctgcgcg tcgagcgtgc ggacgaaact 1920

ggcgagaacc acttcgtgtt cctgttcaac cgcacccacg atgttgcggt cgtggacgtg 1980

gaaggcgaac cgctggtcgc ctcgctggcc caggtcaacg agtccgagca cacggccgcc 2040

atccagccca acggcgtact cgtcgtcaag ctgtaa 2076

<210> 8
<211> 691
<212> PRT
<213> Bifidobacterium longum

<400> 8

Met Glu His Arg Ala Phe Lys Trp Pro Gln Pro Leu Ala Gly Asn Lys
1               5                   10                  15

Pro Arg Ile Trp Tyr Gly Gly Asp Tyr Asn Pro Asp Gln Trp Pro Glu
            20                  25                  30

Glu Val Trp Asp Glu Asp Val Ala Leu Met Gln Gln Ala Gly Val Asn
        35                  40                  45

Leu Val Ser Val Ala Ile Phe Ser Trp Ala Lys Leu Glu Pro Glu Glu
    50                  55                  60

Gly Val Tyr Asp Phe Asp Trp Leu Asp Arg Val Ile Asp Lys Leu Gly
65                  70                  75                  80

Lys Ala Gly Ile Ala Val Asp Leu Ala Ser Gly Thr Ala Ser Pro Pro
                85                  90                  95

Met Trp Met Thr Gln Ala His Pro Glu Ile Leu Trp Val Asp Tyr Arg
            100                 105                 110

Gly Asp Val Cys Gln Pro Gly Ala Arg Gln His Trp Arg Ala Thr Ser
        115                 120                 125

Pro Val Phe Leu Asp Tyr Ala Leu Asn Leu Cys Arg Lys Met Ala Glu
    130                 135                 140

His Tyr Lys Asp Asn Pro Tyr Val Val Ser Trp His Val Ser Asn Glu
145                 150                 155                 160

Tyr Gly Cys His Asn Arg Phe Asp Tyr Ser Glu Asp Ala Glu Arg Ala
                165                 170                 175

Phe Gln Lys Trp Cys Glu Lys Lys Tyr Gly Thr Ile Asp Ala Val Asn
            180                 185                 190

Asp Ala Trp Gly Thr Ala Phe Trp Ala Gln Arg Met Asn Asn Phe Ser
        195                 200                 205

Glu Ile Ile Pro Pro Arg Phe Ile Gly Asp Gly Asn Phe Met Asn Pro
    210                 215                 220

Gly Lys Leu Leu Asp Trp Lys Arg Phe Ser Ser Asp Ala Leu Leu Asp
225                 230                 235                 240

Phe Tyr Lys Ala Glu Arg Asp Ala Leu Leu Glu Ile Ala Pro Lys Pro
                245                 250                 255

Gln Thr Thr Asn Phe Met Val Ser Ala Gly Cys Thr Val Leu Asp Tyr
            260                 265                 270

Asp Lys Trp Gly His Asp Val Asp Phe Val Ser Asn Asp His Tyr Phe
        275                 280                 285

Ser Pro Gly Glu Ala His Phe Asp Glu Met Ala Tyr Ala Ala Cys Leu
    290                 295                 300

Thr Asp Gly Ile Ala Arg Lys Asn Pro Trp Phe Leu Met Glu His Ser
305                 310                 315                 320

Thr Ser Ala Val Asn Trp Arg Pro Thr Asn Tyr Arg Leu Glu Pro Gly
                325                 330                 335

Glu Leu Val Arg Asp Ser Leu Ala His Leu Ala Met Gly Ala Asp Ala
            340                 345                 350

Ile Cys Tyr Phe Gln Trp Arg Gln Ser Lys Ala Gly Ala Glu Lys Trp
        355                 360                 365

His Ser Ala Met Val Pro His Ala Gly Pro Asp Ser Gln Ile Phe Arg
    370                 375                 380

Asp Val Cys Glu Leu Gly Ala Asp Leu Asn Lys Leu Ala Asp Glu Gly
385                 390                 395                 400

Leu Leu Ser Thr Lys Leu Val Lys Ser Lys Val Ala Ile Val Phe Asp
                405                 410                 415

Tyr Glu Ser Gln Trp Ala Thr Glu His Thr Ala Thr Pro Thr Gln Glu
            420                 425                 430

Val Arg His Trp Thr Glu Pro Leu Asp Trp Phe Arg Ala Leu Ala Asp
        435                 440                 445

Asn Gly Leu Thr Ala Asp Val Val Pro Val Arg Gly Pro Trp Asp Glu
    450                 455                 460

Tyr Glu Ala Val Val Leu Pro Ser Leu Ala Ile Leu Ser Glu Gln Thr
465                 470                 475                 480

Thr Arg Arg Val Arg Glu Tyr Val Ala Asn Gly Gly Lys Leu Phe Val
                485                 490                 495

Thr Tyr Tyr Thr Gly Leu Val Asp Asp Arg Asp His Val Trp Leu Gly
            500                 505                 510

Gly Tyr Pro Gly Ser Ile Arg Asp Val Val Gly Val Arg Val Glu Glu
        515                 520                 525

Phe Ala Pro Met Gly Thr Asp Ala Pro Gly Thr Met Asp His Leu Asp
    530                 535                 540

Leu Asp Asn Gly Thr Val Ala His Asp Phe Ala Asp Val Ile Thr Ser
545                 550                 555                 560

Val Ala Asp Thr Ala His Val Val Ala Ser Phe Lys Ala Asp Lys Trp
                565                 570                 575

Thr Gly Phe Asp Gly Ala Pro Ala Ile Thr Val Asn Asp Phe Gly Asp
            580                 585                 590

Gly Lys Ala Ala Tyr Val Gly Ala Arg Leu Gly Arg Glu Gly Leu Ala
        595                 600                 605

Lys Ser Leu Pro Ala Leu Leu Glu Glu Leu Gly Ile Glu Thr Ser Ala
    610                 615                 620

Glu Asp Asp Arg Gly Glu Val Leu Arg Val Glu Arg Ala Asp Glu Thr
625                 630                 635                 640

Gly Glu Asn His Phe Val Phe Leu Phe Asn Arg Thr His Asp Val Ala
                645                 650                 655

Val Val Asp Val Glu Gly Glu Pro Leu Val Ala Ser Leu Ala Gln Val
            660                 665                 670

Asn Glu Ser Glu His Thr Ala Ala Ile Gln Pro Asn Gly Val Leu Val
        675                 680                 685

Val Lys Leu
    690

<210>  9
<211>  1626
<212>  DNA
<213>  Clostridium thermocellum

<400>  9
atggcagaag gggttatagt caacggaact cagtttaaag acacatcggg aaatgtgata      60

catgcccatg ggggaggcat gttaaagcat ggtgactatt attactggta cggtgaatac      120

cgggacgact ccaacttgtt tttgggtgta agttgctaca ggtcaaaaga tcttgtaaac      180

tgggaataca gaggagaagt gctgagccga aattccgctc ctgaactgaa tcactgcaat      240

attgaaagac cgaaagtcat gtacaacgca tcaaccggtg aatttgtcat gtggatgcac      300

tgggagaacg gcataaacta cggtcaggca agagcagctg ttgcgtattc caaaacgccc      360

gacggcaaat tcacatacat tcgaagcttt cgtcccatgc aggataccgg cgttatggat      420

catggccttc cgggatatat gtcaagggac tgcaatgtat ttgtggacac tgacggcaag      480

ggatatttta tatccgcagc caatgagaac atggacctgc acctttatga gctgacacct      540

gactataaaa atattgcatc ccttaaggca aagctgtttg tcggacagca gagggaagca      600

ccatgcctta taaagagaaa cggctactat taccttatta cttccggttg tacaggttgg      660

aacccgaatc aggctaaata cgcatattcc aaagatttgg ccagtggctg gtcccagctt      720

tacaatcttg gtaattcaac cacctacagg tcacagccga cttttatcat tcccgttcag 780

ggaagctcgg gaaccagtta tctttatatg ggtgaccgtt gggccggtgc ctggggagga 840

aaggttaatg actcccaata tgtatggctt cccttaaact tcatatccga tacaacactt 900

gaactgccct attatgactc tgtaaagatt gatgcttctt caggaataat ttccgagtac 960

ataccggaca ctacacgcta caagctggta aacaaaaaca gcggaaaagt cctggatgtt 1020

cttgacggtt ctgtcgataa tgcagcccag atagtccaat ggaccgataa cgggtctttg 1080

agtcaacagt ggtaccttgt ggacgtgggc ggtggttata aaaagattgt aaatgtaaag 1140

agcggaagag ccttggatgt aaaagacgaa tccaaggaag acggtggagt attaatacaa 1200

tataccagca acggcggata taatcagcac tggaaattca cagacatagg tgacgggtat 1260

tacaagattt ccagccgcca ctgcggaaaa cttatagatg tgcgaaaatg gtcaacggaa 1320

gacggcggaa taattcagca gtggtccgat gccggaggaa caaatcagca ttggaagctg 1380

gtgcttgtat caagtcccga gccttcacca tcaccttctc cccaagtggt taaaggagat 1440

gtaaacggcg acttgaaagt aaattcaacg gatttttcca tgttaagaag atatttactt 1500

aaaaccattg acaattttcc gacagaaaac ggaaaacagg ctgccgattt gaacggagac 1560

ggcagaataa actcttcgga tcttacaatg ctgaaaagat acttgcttat ggaagtggat 1620

ttgtaa 1626

<210> 10
<211> 541
<212> PRT
<213> Clostridium thermocellum

<400> 10

Met Ala Glu Gly Val Ile Val Asn Gly Thr Gln Phe Lys Asp Thr Ser
1               5                   10                  15

Gly Asn Val Ile His Ala His Gly Gly Gly Met Leu Lys His Gly Asp
            20                  25                  30

Tyr Tyr Tyr Trp Tyr Gly Glu Tyr Arg Asp Asp Ser Asn Leu Phe Leu
        35                  40                  45

Gly Val Ser Cys Tyr Arg Ser Lys Asp Leu Val Asn Trp Glu Tyr Arg
    50                  55                  60

Gly Glu Val Leu Ser Arg Asn Ser Ala Pro Glu Leu Asn His Cys Asn
65                  70                  75                  80

Ile Glu Arg Pro Lys Val Met Tyr Asn Ala Ser Thr Gly Glu Phe Val
                85                  90                  95

Met Trp Met His Trp Glu Asn Gly Ile Asn Tyr Gly Gln Ala Arg Ala
            100                 105                 110

Ala Val Ala Tyr Ser Lys Thr Pro Asp Gly Lys Phe Thr Tyr Ile Arg
        115                 120                 125

Ser Phe Arg Pro Met Gln Asp Thr Gly Val Met Asp His Gly Leu Pro
    130                 135                 140

Gly Tyr Met Ser Arg Asp Cys Asn Val Phe Val Asp Thr Asp Gly Lys
145                 150                 155                 160

Gly Tyr Phe Ile Ser Ala Ala Asn Glu Asn Met Asp Leu His Leu Tyr
                165                 170                 175

Glu Leu Thr Pro Asp Tyr Lys Asn Ile Ala Ser Leu Lys Ala Lys Leu
            180                 185                 190

Phe Val Gly Gln Gln Arg Glu Ala Pro Cys Leu Ile Lys Arg Asn Gly
        195                 200                 205

Tyr Tyr Tyr Leu Ile Thr Ser Gly Cys Thr Gly Trp Asn Pro Asn Gln
    210                 215                 220

Ala Lys Tyr Ala Tyr Ser Lys Asp Leu Ala Ser Gly Trp Ser Gln Leu
225                 230                 235                 240

Tyr Asn Leu Gly Asn Ser Thr Thr Tyr Arg Ser Gln Pro Thr Phe Ile
                245                 250                 255

Ile Pro Val Gln Gly Ser Ser Gly Thr Ser Tyr Leu Tyr Met Gly Asp
            260                 265                 270

Arg Trp Ala Gly Ala Trp Gly Gly Lys Val Asn Asp Ser Gln Tyr Val
        275                 280                 285

Trp Leu Pro Leu Asn Phe Ile Ser Asp Thr Thr Leu Glu Leu Pro Tyr
    290                 295                 300

Tyr Asp Ser Val Lys Ile Asp Ala Ser Ser Gly Ile Ile Ser Glu Tyr
305                 310                 315                 320

Ile Pro Asp Thr Thr Arg Tyr Lys Leu Val Asn Lys Asn Ser Gly Lys
                325                 330                 335

Val Leu Asp Val Leu Asp Gly Ser Val Asp Asn Ala Ala Gln Ile Val

340                 345                 350

Gln Trp Thr Asp Asn Gly Ser Leu Ser Gln Gln Trp Tyr Leu Val Asp
        355                 360                 365

Val Gly Gly Gly Tyr Lys Lys Ile Val Asn Val Lys Ser Gly Arg Ala
    370                 375                 380

Leu Asp Val Lys Asp Glu Ser Lys Glu Asp Gly Gly Val Leu Ile Gln
385                 390                 395                 400

Tyr Thr Ser Asn Gly Gly Tyr Asn Gln His Trp Lys Phe Thr Asp Ile
                405                 410                 415

Gly Asp Gly Tyr Tyr Lys Ile Ser Ser Arg His Cys Gly Lys Leu Ile
            420                 425                 430

Asp Val Arg Lys Trp Ser Thr Glu Asp Gly Gly Ile Ile Gln Gln Trp
        435                 440                 445

Ser Asp Ala Gly Gly Thr Asn Gln His Trp Lys Leu Val Leu Val Ser
    450                 455                 460

Ser Pro Glu Pro Ser Pro Ser Pro Ser Pro Gln Val Val Lys Gly Asp
465                 470                 475                 480

Val Asn Gly Asp Leu Lys Val Asn Ser Thr Asp Phe Ser Met Leu Arg
                485                 490                 495

Arg Tyr Leu Leu Lys Thr Ile Asp Asn Phe Pro Thr Glu Asn Gly Lys
            500                 505                 510

Gln Ala Ala Asp Leu Asn Gly Asp Gly Arg Ile Asn Ser Ser Asp Leu
        515                 520                 525

Thr Met Leu Lys Arg Tyr Leu Leu Met Glu Val Asp Leu
    530                 535                 540

<210>  11
<211>  2631
<212>  DNA
<213>  Paenibacillus sp.

<400>  11
atgaatcgac acgtcctgct tcatccgtat ctccaccgga aggcgttgcc tctgctcctg      60

gccttgacgc tgctgacggg catcgccctg ttcccggcct ccaccgcgca ggcggcgacg     120

accgtgacgt cgatgacgta cttctctgcc aatgacggtc ccgtcatctc caaatccggc     180

gtcgggcaag ccagctacgg tttcgtcatg ccgatcttca acggaggcgc tgcgacctgg 240

aacgatgtcg ccgatgacgt cggcgttcgc gtcaaggtcg gcggcagctg ggtcgacatt 300

gacagcgttg gcggctatgt gtacaaccag aactggggcc attggaacga cagcggcacc 360

tatggctact ggttcaccct ctccgccacg accgagctgc agctctactc caaggcgaac 420

agcagcgtca cactcaacta cacgctcgtc ttccagaatg tcaatgaaac gaccattacc 480

tcgatgacac cgacccaggg cccgcaattg accgcagggt ataccggcgg cgcaggcttc 540

acctatccgg tcttcaacaa cgatccctcc atcccgtatg cagccgtagc cggcgatctg 600

aaggtgtacg tcaagccagt cgccagcagt acctggatcg atatcgacaa caacgcggcg 660

agcggctgga tctacgacag caacttcggc cagttcaccg aaggcggcgg cggctactgg 720

ttcaccgtca ccgagtcgat caacgtcaag ctcgagtcca ggacgtcctc ggccaacgtc 780

gtctatacga tcaacttccc gcagccgacg cgcagcagct acacactctc cgcctatgac 840

ggcacgacct acagcgccga tgcgagcggc gcgatcggta tcccgctgcc gcggatcgac 900

ggcaccccgg cgatcggcag cgagctcggc aacttcgtct accagatcta ccggaacggc 960

cagtgggtcg agatgagcaa ctcggcgcag agcagcttcg tctactcggc caatggctac 1020

aacaacatgt ccgacgccaa tcaatggggc tactgggccg actacatcta cggcctctgg 1080

ttccggccga tccaggagga tatgcagatc cgcatcggct atccgctgaa tggccagtcc 1140

ggcggcagcg tcggcagcaa cttcgtcacc tatacgctga tcggcaaccc gaacgcgccg 1200

cgacccgatg tgagcgacca gggcgacgtc gagatcggca cgcccaccga tccggccatc 1260

gcaggatgga atctgtattg gcaggatgaa ttcgccggca gcgcgctcga tctgaacaag 1320

tggaactacg agaccggcta ctacatcggc aacgacccca atctgtgggg ctggggcaac 1380

gccgagatgc agcactatac gacgagcacg caaaatgtct tcgtcgctga cggcaaactc 1440

aacatccgag cgctccacga ttaccaatcg ttcccgcagg acccgaaccg ctacgcgacc 1500

tactcctccg gcaagatcaa caccaaggac aacatgtcgc tgcagtacgg ccgcgtcgat 1560

atccgcgcca agctgccgac tggcgatggc gtctggccgg cactgtggat gctgccggag 1620

gactccgtct acggcgcatg ggcggcatca ggagagatcg acatcatgga ggcgaagggc 1680

cgtctgcccg gcacgacgag cggcgcgatc cactacggcg gccaatggcc ggtcaaccgc 1740

tacctcgccg gagaatgcta cctcccgcaa ggtacgacat tcgccgacga ctttaatgtg 1800

tacacgatga tctgggaaga ggacaacatg aagtggtacg ttaacggtga gtttttcttc 1860

aaggtgacgc gcgagcagtg gtactccgtc gccgccccca acaatccgga cgcgccgttc 1920

gaccagccgt tctatctgat catgaacctg gcggtcggcg gccacttcga cggcgggcgt 1980

acgcccgacc cgtccgacat cccggcgacg atgcagatcg actacgtgcg ggtgtacaaa 2040

gagggcgcgg gcggcggtcc gggcaacccg ggcggcaacg tcgcggtgac cggcgttagc 2100

gtgaccccgg caacggcgca ggtgcaggtc ggtcagaccg tctcgctgag cgccaacgtc 2160

gcgccagcca atgcaacgaa caagcaagtg acctggtcag tcgccaatgg cagcatcgcc 2220

tcggtgagcg ccagcggcgt cgtcagtgga ctcgctgctg gcacgacgac cgtaaccgcc 2280

acgaccgcag acggcaaccg caccgcctcg gcgacgatca ccgtcgtgcc gccaccgacg 2340

acgaccgtca tcatcggcga tagcgtgcgc ggcatccgaa agaccggcga caacctgctc 2400

ttctacgtca acggcgcaac ctacgccgac ctgcactaca aggtgaacgg cggcggtcag 2460

cctaatgtcg cgatgacgca cacaggaggc ggcaactaca cctacccggt gcatggcctc 2520

caacaaggcg ataccgtcga atacttcttc acctacaacc ccggcaacgg cgcgctagac 2580

acgccttggc agacttatgt gcatggggta acacaaggtg ttgttgagta a 2631

<210> 12
<211> 876
<212> PRT
<213> Paenibacillus sp.

<400> 12

Met Asn Arg His Val Leu Leu His Pro Tyr Leu His Arg Lys Ala Leu
1               5                   10                  15

Pro Leu Leu Leu Ala Leu Thr Leu Leu Thr Gly Ile Ala Leu Phe Pro
            20                  25                  30

Ala Ser Thr Ala Gln Ala Ala Thr Thr Val Thr Ser Met Thr Tyr Phe
        35                  40                  45

Ser Ala Asn Asp Gly Pro Val Ile Ser Lys Ser Gly Val Gly Gln Ala
    50                  55                  60

Ser Tyr Gly Phe Val Met Pro Ile Phe Asn Gly Gly Ala Ala Thr Trp
65                  70                  75                  80

Asn Asp Val Ala Asp Asp Val Gly Val Arg Val Lys Val Gly Gly Ser
                85                  90                  95

Trp Val Asp Ile Asp Ser Val Gly Gly Tyr Val Tyr Asn Gln Asn Trp
            100                 105                 110

Gly His Trp Asn Asp Ser Gly Thr Tyr Gly Tyr Trp Phe Thr Leu Ser
        115                 120                 125

Ala Thr Thr Glu Leu Gln Leu Tyr Ser Lys Ala Asn Ser Ser Val Thr
    130                 135                 140

Leu Asn Tyr Thr Leu Val Phe Gln Asn Val Asn Glu Thr Thr Ile Thr
145                 150                 155                 160

Ser Met Thr Pro Thr Gln Gly Pro Gln Leu Thr Ala Gly Tyr Thr Gly
                165                 170                 175

Gly Ala Gly Phe Thr Tyr Pro Val Phe Asn Asn Asp Pro Ser Ile Pro
            180                 185                 190

Tyr Ala Ala Val Ala Gly Asp Leu Lys Val Tyr Val Lys Pro Val Ala
        195                 200                 205

Ser Ser Thr Trp Ile Asp Ile Asp Asn Asn Ala Ala Ser Gly Trp Ile
    210                 215                 220

Tyr Asp Ser Asn Phe Gly Gln Phe Thr Glu Gly Gly Gly Gly Tyr Trp
225                 230                 235                 240

Phe Thr Val Thr Glu Ser Ile Asn Val Lys Leu Glu Ser Arg Thr Ser
                245                 250                 255

Ser Ala Asn Val Val Tyr Thr Ile Asn Phe Pro Gln Pro Thr Arg Ser
            260                 265                 270

Ser Tyr Thr Leu Ser Ala Tyr Asp Gly Thr Thr Tyr Ser Ala Asp Ala
        275                 280                 285

Ser Gly Ala Ile Gly Ile Pro Leu Pro Arg Ile Asp Gly Thr Pro Ala
    290                 295                 300

Ile Gly Ser Glu Leu Gly Asn Phe Val Tyr Gln Ile Tyr Arg Asn Gly
305                 310                 315                 320

Gln Trp Val Glu Met Ser Asn Ser Ala Gln Ser Ser Phe Val Tyr Ser
                325                 330                 335

Ala Asn Gly Tyr Asn Asn Met Ser Asp Ala Asn Gln Trp Gly Tyr Trp
            340                 345                 350

Ala Asp Tyr Ile Tyr Gly Leu Trp Phe Arg Pro Ile Gln Glu Asp Met
        355                 360                 365

Gln Ile Arg Ile Gly Tyr Pro Leu Asn Gly Gln Ser Gly Gly Ser Val
    370                 375                 380

Gly Ser Asn Phe Val Thr Tyr Thr Leu Ile Gly Asn Pro Asn Ala Pro
385                 390                 395                 400

Arg Pro Asp Val Ser Asp Gln Gly Asp Val Glu Ile Gly Thr Pro Thr
                405                 410                 415

Asp Pro Ala Ile Ala Gly Trp Asn Leu Tyr Trp Gln Asp Glu Phe Ala
            420                 425                 430

Gly Ser Ala Leu Asp Leu Asn Lys Trp Asn Tyr Glu Thr Gly Tyr Tyr
        435                 440                 445

Ile Gly Asn Asp Pro Asn Leu Trp Gly Trp Gly Asn Ala Glu Met Gln
    450                 455                 460

His Tyr Thr Thr Ser Thr Gln Asn Val Phe Val Ala Asp Gly Lys Leu
465                 470                 475                 480

Asn Ile Arg Ala Leu His Asp Tyr Gln Ser Phe Pro Gln Asp Pro Asn
                485                 490                 495

Arg Tyr Ala Thr Tyr Ser Ser Gly Lys Ile Asn Thr Lys Asp Asn Met
            500                 505                 510

Ser Leu Gln Tyr Gly Arg Val Asp Ile Arg Ala Lys Leu Pro Thr Gly
        515                 520                 525

Asp Gly Val Trp Pro Ala Leu Trp Met Leu Pro Glu Asp Ser Val Tyr
    530                 535                 540

Gly Ala Trp Ala Ala Ser Gly Glu Ile Asp Ile Met Glu Ala Lys Gly
545                 550                 555                 560

Arg Leu Pro Gly Thr Thr Ser Gly Ala Ile His Tyr Gly Gly Gln Trp
                565                 570                 575

Pro Val Asn Arg Tyr Leu Ala Gly Glu Cys Tyr Leu Pro Gln Gly Thr
            580                 585                 590

Thr Phe Ala Asp Asp Phe Asn Val Tyr Thr Met Ile Trp Glu Glu Asp
        595                 600                 605

Asn Met Lys Trp Tyr Val Asn Gly Glu Phe Phe Phe Lys Val Thr Arg
    610                 615                 620

Glu Gln Trp Tyr Ser Val Ala Ala Pro Asn Asn Pro Asp Ala Pro Phe
625                 630                 635                 640

Asp Gln Pro Phe Tyr Leu Ile Met Asn Leu Ala Val Gly Gly His Phe
                645                 650                 655

Asp Gly Gly Arg Thr Pro Asp Pro Ser Asp Ile Pro Ala Thr Met Gln
            660                 665                 670

Ile Asp Tyr Val Arg Val Tyr Lys Glu Gly Ala Gly Gly Gly Pro Gly
        675                 680                 685

Asn Pro Gly Gly Asn Val Ala Val Thr Gly Val Ser Val Thr Pro Ala
    690                 695                 700

Thr Ala Gln Val Gln Val Gly Gln Thr Val Ser Leu Ser Ala Asn Val
705                 710                 715                 720

Ala Pro Ala Asn Ala Thr Asn Lys Gln Val Thr Trp Ser Val Ala Asn
                725                 730                 735

Gly Ser Ile Ala Ser Val Ser Ala Ser Gly Val Val Ser Gly Leu Ala
            740                 745                 750

Ala Gly Thr Thr Thr Val Thr Ala Thr Thr Ala Asp Gly Asn Arg Thr
        755                 760                 765

Ala Ser Ala Thr Ile Thr Val Val Pro Pro Pro Thr Thr Thr Val Ile
    770                 775                 780

Ile Gly Asp Ser Val Arg Gly Ile Arg Lys Thr Gly Asp Asn Leu Leu
785                 790                 795                 800

Phe Tyr Val Asn Gly Ala Thr Tyr Ala Asp Leu His Tyr Lys Val Asn
                805                 810                 815

Gly Gly Gly Gln Pro Asn Val Ala Met Thr His Thr Gly Gly Gly Asn
            820                 825                 830

Tyr Thr Tyr Pro Val His Gly Leu Gln Gln Gly Asp Thr Val Glu Tyr
        835                 840                 845

Phe Phe Thr Tyr Asn Pro Gly Asn Gly Ala Leu Asp Thr Pro Trp Gln
    850                 855                 860

Thr Tyr Val His Gly Val Thr Gln Gly Val Val Glu
865                 870                 875

<210>  13
<211>  6783
<212>  DNA
<213>  Artificial Sequence

<220>
<223>  Transpsosn cassette

<400>  13
gccagatgat taattcctaa tttttgttga cactctatca ttgatagagt tattttacca      60

ctccctatca gtgatagaga aaagtgaaat gaatagttcg acaaaaatct agaaataatt     120

ttgtttaact ttaagaagga gatatacaat ttcgtcgaca cacaggaaac atattaaaaa     180

ttaaaacctg caggagtttg aaggagatag aaccatggcg cagtcgaaac tctatccagt     240

tgtgatggca ggtggctccg gtagccgctt atggccgctt tcccgcgtac tttatcccaa     300

gcagttttta tgcctgaaag gcgatctcac catgctgcaa accaccatct gccgcctgaa     360

cggcgtggag tgcgaaagcc cggtggtgat ttgcaatgag cagcaccgct ttattgtcgc     420

ggaacagctg cgtcaactga acaaacttac cgagaacatt attctcgaac cggcagggcg     480

aaacacggca cctgccattg cgctggcggc gctggcggca aaacgtcata gcccggagag     540

cgacccgtta atgctggtat tggcggcgga tcatgtgatt gccgatgaag acgcgttccg     600

tgccgccgtg cgtaatgcca tgccatatgc cgaagcgggc aagctggtga ccttcggcat     660

tgtgccggat ctaccagaaa ccggttatgg ctatattcgt cgcggtgaag tgtctgcggg     720

tgagcaggat atggtggcct ttgaagtggc gcagtttgtc gaaaaaccga atctggaaac     780

cgctcaggcc tatgtggcaa gcggcgaata ttactggaac agcggtatgt tcctgttccg     840

cgccggacgc tatctcgaag aactgaaaaa atatcgcccg gatatcctcg atgcctgtga     900

aaaagcgatg agcgccgtcg atccggatct caattttatt cgcgtggatg aagaagcgtt     960

tctcgcctgc ccggaagagt cggtggatta cgcggtcatg gaacgtacgg cagatgctgt    1020

tgtggtgccg atggatgcgg gctggagcga tgttggctcc tggtcttcat tatgggagat    1080

cagcgcccac accgccgagg gcaacgtttg ccacggcgat gtgattaatc acaaaactga    1140

aaacagctat gtgtatgctg aatctggcct ggtcaccacc gtcggggtga aagatctggt    1200

agtggtgcag accaaagatg cggtgctgat tgccgaccgt aacgcggtac aggatgtgaa    1260

aaaagtggtc gagcagatca aagccgatgg tcgccatgag catcgggtgc atcgcgaagt    1320

gtatcgtccg tggggcaaat atgactctat cgacgcgggc gaccgctacc aggtgaaacg    1380

catcaccgtg aaaccgggcg agggcttgtc ggtacagatg caccatcacc gcgcggaaca    1440

ctgggtggtt gtcgcgggaa cggcaaaagt caccattgat ggtgatatca aactgcttgg    1500

tgaaaacgag tccatttata ttccgctggg ggcgacgcat tgcctggaaa acccggggaa    1560

aattccgctc gatttaattg aagtgcgctc cggctcttat ctcgaagagg atgatgtggt    1620

gcgtttcgcg gatcgctacg gacgggtgta aacgtcgcat caggcaatga atgcgaaacc    1680

gcggtgtaaa taacgacaaa aataaaattg gccgcttcgg tcagggccaa ctattgcctg    1740

aaaaagggta acgatatgaa aaaattaacc tgctttaaag cctatgatat tcgcgggaaa    1800

ttaggcgaag aactgaatga agatatcgcc tggcgcattg gtcgcgccta tggcgaattt 1860

ctcaaaccga aaaccattgt gttaggcggt gatgtccgcc tcaccagcga aaccttaaaa 1920

ctggcgctgg cgaaaggttt acaggatgcg ggcgttgacg tgctggatat tggtatgtcc 1980

ggcaccgaag agatctattt cgccacgttc catctcggcg tggatggcgg cattgaagtt 2040

accgccagcc ataatccgat ggattataac ggcatgaagc tggttcgcga gggggctcgc 2100

ccgatcagcg gagataccgg actgcgcgac gtccagcgtc tggctgaagc caacgacttt 2160

cctcccgtcg atgaaaccaa acgcggtcgc tatcagcaaa tcaacctgcg tgacgcttac 2220

gttgatcacc tgttcggtta tatcaatgtc aaaaacctca cgccgctcaa gctggtgatc 2280

aactccggga acggcgcagc gggtccggtg gtggacgcca ttgaagcccg ctttaaagcc 2340

ctcggcgcgc ccgtggaatt aatcaaagtg cacaacacgc cggacggcaa tttccccaac 2400

ggtattccta acccactact gccggaatgc cgcgacgaca cccgcaatgc ggtcatcaaa 2460

cacggcgcgg atatgggcat tgcttttgat ggcgattttg accgctgttt cctgtttgac 2520

gaaaaagggc agtttattga gggctactac attgtcggcc tgttggcaga agcattcctc 2580

gaaaaaaatc ccggcgcgaa gatcatccac gatccacgtc tctcctggaa caccgttgat 2640

gtggtgactg ccgcaggtgg cacgccggta atgtcgaaaa ccggacacgc ctttattaaa 2700

gaacgtatgc gcaaggaaga cgccatctat ggtggcgaaa tgagcgccca ccattacttc 2760

cgtgatttcg cttactgcga cagcggcatg atcccgtggc tgctggtcgc cgaactggtg 2820

tgcctgaaag ataaaacgct gggcgaactg gtacgcgacc ggatggcggc gtttccggca 2880

agcggtgaga tcaacagcaa actggcgcaa cccgttgagg cgattaaccg cgtggaacag 2940

cattttagcc gtgaggcgct ggcggtggat cgcaccgatg gcatcagcat gacctttgcc 3000

gactggcgct ttaacctgcg cacctccaat accgaaccgg tggtgcgcct gaatgtggaa 3060

tcgcgcggtg atgtgccgct gatggaagcg cgaacgcgaa ctctgctgac gttgctgaac 3120

gagtaaaaac gcggccgcga tatcgttgta aaacgacggc cagtgcaaga atcataaaaa 3180

atttatttgc tttcaggaaa atttttctgt ataatagatt cataaatttg agagaggagt 3240

ttttgtgagc ggataacaat tccccatctt agtatattag ttaagtataa atacaccgcg 3300

gaggacgaag gagatagaac catgtcaaaa gtcgctctca tcaccggtgt aaccggacaa 3360

gacggttctt acctggcaga gtttctgctg gaaaaaggtt acgaggtgca tggtattaag 3420

cgtcgcgcat cgtcattcaa caccgagcgc gtggatcaca tttatcagga tccgcacacc 3480

tgcaacccga aattccatct gcattatggc gacctgagtg atacctctaa cctgacgcgc 3540

attttgcgtg aagtacagcc ggatgaagtg tacaacctgg gcgcaatgag ccacgttgcg 3600

gtctcttttg agtcaccaga atataccgct gacgtcgacg cgatgggtac gctgcgcctg 3660

ctggaggcga tccgcttcct cggtctggaa aagaaaactc gtttctatca ggcttccacc 3720

tctgaactgt atggtctggt gcaggaaatt ccgcagaaag agaccacgcc gttctacccg 3780

cgatctccgt atgcggtcgc caaactgtac gcctactgga tcaccgttaa ctaccgtgaa 3840

tcctacggca tgtacgcctg taacggaatt ctcttcaacc atgaatcccc gcgccgcggc 3900

gaaaccttcg ttacccgcaa aatcacccgc gcaatcgcca acatcgccca ggggctggag 3960

tcgtgcctgt acctcggcaa tatggattcc ctgcgtgact ggggccacgc caaagactac 4020

gtaaaaatgc agtggatgat gctgcagcag gaacagccgg aagatttcgt tatcgcgacc 4080

ggcgttcagt actccgtgcg tcagttcgtg gaaatggcgg cagcacagct gggcatcaaa 4140

ctgcgctttg aaggcacggg cgttgaagag aagggcattg tggtttccgt caccgggcat 4200

gacgcgccgg gcgttaaacc gggtgatgtg attatcgctg ttgacccgcg ttacttccgt 4260

ccggctgaag ttgaaacgct gctcggcgac ccgaccaaag cgcacgaaaa actgggctgg 4320

aaaccggaaa tcaccctcag agagatggtg tctgaaatgg tggctaatga cctcgaagcg 4380

gcgaaaaaac actctctgct gaaatctcac ggctacgacg tggcgatcgc gctggagtca 4440

taagcatgag taaacaacga gtttttattg ctggtcatcg cgggatggtc ggttccgcca 4500

tcaggcggca gctcgaacag cgcggtgatg tggaactggt attacgcacc cgcgacgagc 4560

tgaacctgct ggacagccgc gccgtgcatg atttctttgc cagcgaacgt attgaccagg 4620

tctatctggc ggcggcgaaa gtgggcggca ttgttgccaa caacacctat ccggcggatt 4680

tcatctacca gaacatgatg attgagagca acatcattca cgccgcgcat cagaacgacg 4740

tgaacaaact gctgtttctc ggatcgtcct gcatctaccc gaaactggca aaacagccga 4800

tggcagaaag cgagttgttg cagggcacgc tggagccgac taacgagcct tatgctattg 4860

ccaaaatcgc cgggatcaaa ctgtgcgaat catacaaccg ccagtacgga cgcgattacc 4920

gctcagtcat gccgaccaac ctgtacgggc cacacgacaa cttccacccg agtaattcgc 4980

atgtgatccc agcattgctg cgtcgcttcc acgaggcgac ggcacagaat gcgccggacg 5040

tggtggtatg gggcagcggt acaccgatgc gcgaatttct gcacgtcgat gatatggcgg 5100

cggcgagcat tcatgtcatg gagctggcgc atgaagtctg gctggagaac acccagccga 5160

tgttgtcgca cattaacgtc ggcacgggcg ttgactgcac tatccgcgag ctggcgcaaa 5220

ccatcgccaa agtggtgggt tacaaaggcc gggtggtttt tgatgccagc aaaccggatg 5280

gcacgccgcg caaactgctg gatgtgacgc gcctgcatca gcttggctgg tatcacgaaa 5340

tctcactgga agcggggctt gccagcactt accagtggtt ccttgagaat caagaccgct 5400

ttcggggggg gagctaacgc gccatttaaa tcaacctcag cggtcatagc tgtttcctgt 5460

gactgagcaa taactagcat aaccccttgg ggcctctaaa cgggtcttga ggggtttttt 5520

gctgaaacca atttgcctgg cggcagtagc gcggtggtcc cacctgaccc catgccgaac 5580

tcagaagtga aacgccgtag cgccgatggt agtgtggggt ctccccatgc gagagtaggg 5640

aactgccagg catcaaataa aacgaaaggc tcagtcgaaa gactgggcct ttcgggatcc 5700

aggccggcct gttaacgaat taatcttccg cggcggtatc gataagcttg atatcgaatt 5760

ccgaagttcc tattctctag aaagtatagg aacttcaggt ctgaagagga gtttacgtcc 5820

agccaagcta gcttggctgc aggtcgtcga aattctaccg ggtaggggag gcgcttttcc 5880

caaggcagtc tggagcatgc gctttagcag ccccgctggg cacttggcgc tacacaagtg 5940

gcctctggcc tcgcacacat tccacatcca ccggtaggcg ccaaccggct ccgttctttg 6000

gtggcccctt cgcgccacct tctactcctc ccctagtcag gaagttcccc cccgccccgc 6060

agctcgcgtc gtgcaggacg tgacaaatgg aagtagcacg tctcactagt ctcgtgcaga 6120

tggacagcac cgctgagcaa tggaagcggg taggcctttg gggcagcggc caatagcagc 6180

tttgctcctt cgctttctgg gctcagaggc tgggaagggg tgggtccggg ggcgggctca 6240

ggggcgggct caggggcggg gcgggcgccc gaaggtcctc cggaggcccg gcattctgca 6300

cgcttcaaaa gcgcacgtct gccgcgctgt tctcctcttc ctcatctccg ggcctttcga 6360

cctgcagcct gttgacaatt aatcatcggc atagtatatc ggcatagtat aatacgacaa 6420

ggtgaggaac taaaccatgg gtcaaagtag cgatgaagcc aacgctcccg ttgcagggca 6480

gtttgcgctt cccctgagtg ccacctttgg cttaggggat cgcgtacgca agaaatctgg 6540

tgccgcttgg cagggtcaag tcgtcggttg gtattgcaca aaactcactc ctgaaggcta 6600

tgcggtcgag tccgaatccc acccaggctc agtgcaaatt tatcctgtgg ctgcacttga 6660

acgtgtggcc taatgagggg atcaattctc tagagctcgc tgatcagaag ttcctattct 6720

ctagaaagta taggaacttc gatggcgcct catccctgaa gccaataggg ataacagggt 6780

aat 6783

<210> 14
<211> 2851
<212> DNA
<213> Artificial Sequence

<220>
<223> Integration cassette

<400> 14
tggccagatg attaattcct aatttttgtt gacactctat cattgataga gttattttac 60

cactccctat cagtgataga gaaaagtgaa atgaatagtt cgacaaaaat ctagaaataa 120

ttttgtttaa ctttaagaag gagatataca aatgtactat ttaaaaaaca caaacttttg 180

gatgttcggt ttattctttt tcttttactt ttttatcatg ggagcctact tcccgttttt 240

cccgatttgg ctacatgaca tcaaccatat cagcaaaagt gatacgggta ttatttttgc 300

cgctatttct ctgttctcgc tattattcca accgctgttt ggtctgcttt ctgacaaact 360

cgggctgcgc aaatacctgc tgtggattat taccggcatg ttagtgatgt ttgcgccgtt 420

ctttattttt atcttcgggc cactgttaca atacaacatt ttagtaggat cgattgttgg 480

tggtatttat ctaggctttt gttttaacgc cggtgcgcca gcagtagagg catttattga 540

gaaagtcagc cgtcgcagta atttcgaatt tggtcgcgcg cggatgtttg gctgtgttgg 600

ctgggcgctg tgtgcctcga ttgtcggcat catgttcacc atcaataatc agtttgtttt 660

ctggctgggc tctggctgtg cactcatcct cgccgtttta ctctttttcg ccaaaacgga 720

tgcgccctct tctgccacgg ttgccaatgc ggtaggtgcc aaccattcgg catttagcct 780

taagctggca ctggaactgt tcagacagcc aaaactgtgg tttttgtcac tgtatgttat 840

tggcgtttcc tgcacctacg atgtttttga ccaacagttt gctaatttct ttacttcgtt 900

ctttgctacc ggtgaacagg gtacgcgggt atttggctac gtaacgacaa tgggcgaatt 960

acttaacgcc tcgattatgt tctttgcgcc actgatcatt aatcgcatcg gtgggaaaaa 1020

cgccctgctg ctggctggca ctattatgtc tgtacgtatt attggctcat cgttcgccac 1080

ctcagcgctg gaagtggtta ttctgaaaac gctgcatatg tttgaagtac cgttcctgct 1140

ggtgggctgc tttaaatata ttaccagcca gtttgaagtg cgtttttcag cgacgattta 1200

tctggtctgt ttctgcttct ttaagcaact ggcgatgatt tttatgtctg tactggcggg 1260

caatatgtat gaaagcatcg gtttccaggg cgcttatctg gtgctgggtc tggtggcgct 1320

gggcttcacc ttaatttccg tgttcacgct tagcggcccc ggcccgcttt ccctgctgcg 1380

tcgtcaggtg aatgaagtcg ctgggagcta agcggccgcg tcgacacgca aaaaggccat 1440

ccgtcaggat ggccttctgc ttaatttgat gcctggcagt ttatggcggg cgtcctgccc 1500

gccaccctcc gggccgttgc ttcgcaacgt tcaaatccgc tcccggcgga tttgtcctac 1560

tcaggagagc gttcaccgac aaacaacaga taaaacgaaa ggcccagtct ttcgactgag 1620

cctttcgttt tatttgatgc ctggcagttc cctactctcg catggggaga ccccacacta 1680

ccatcatgta tgaatatcct ccttagttcc tattccgaag ttcctattct ctagaaagta 1740

taggaacttc ggcgcgtcct acctgtgaca cgcgtgccgc agtctcacgc ccggagcgta 1800

gcgaccgagt gagctagcta tttgtttatt tttctaaata cattcaaata tgtatccgct 1860

catgagacaa taaccctgat aaatgcttca ataatattga aaaaggaaga gtatgaggga 1920

agcggtgatc gccgaagtat cgactcaact atcagaggta gttggcgtca tcgagcgcca 1980

tctcgaaccg acgttgctgg ccgtacattt gtacggctcc gcagtggatg gcggcctgaa 2040

gccacacagt gatattgatt tgctggttac ggtgaccgta aggcttgatg aaacaacgcg 2100

gcgagctttg atcaacgacc ttttggaaac ttcggcttcc cctggagaga gcgagattct 2160

ccgcgctgta gaagtcacca ttgttgtgca cgacgacatc attccgtggc gttatccagc 2220

taagcgcgaa ctgcaatttg gagaatggca gcgcaatgac attcttgcag gtatcttcga 2280

gccagccacg atcgacattg atctggctat cttgctgaca aaagcaagag aacatagcgt 2340

tgccttggta ggtccagcgg cggaggaact ctttgatccg gttcctgaac aggatctatt 2400

tgaggcgcta aatgaaacct taacgctatg gaactcgccg cccgactggg ctggcgatga 2460

gcgaaatgta gtgcttacgt tgtcccgcat ttggtacagc gcagtaaccg gcaaaatcgc 2520

gccgaaggat gtcgctgccg actgggcaat ggagcgcctg ccggcccagt atcagcccgt 2580

catacttgaa gctagacagg cttatcttgg acaagaagaa gatcgcttgg cctcgcgcgc 2640

agatcagttg gaagaatttg tccactacgt gaaaggcgag atcaccaagg tagtcggcaa 2700

ataatgtcta acaattcgtt caagccgagg ggccgcaaga tccggccacg atgacccggt 2760

cgtcgggtac cggcagggcg gggcgtaagg cgcgccattt aaatgaagtt cctattccga 2820

agttcctatt ctctagaaag tataggaact t 2851

<210> 15
<211> 2858
<212> DNA
<213> Artificial Sequence

<220>
<223> Integration cassette

<400> 15
ggccagatga ttaattccta atttttgttg acactctatc attgatagag ttattttacc 60

actccctatc agtgatagag aaaagtgaaa tgaatagttc gacaaaaatc tagaaataat 120

tttgtttaac tttaagaagg agatatacaa atgggcagca ttattcgtct gcagggtggt 180

ctgggtaatc agctgtttca gtttagcttt ggttatgccc tgagcaaaat taatggtaca 240

ccgctgtatt tcgacattag ccattatgcc gaaaacgatg atcatggtgg ttatcgtctg 300

aataatctgc agattccgga agaatatctg cagtattata ccccgaaaat taataatatt 360

tataaactgc tggtgcgtgg cagccgtctg tatccggata tttttctgtt tctgggcttt 420

tgcaacgaat ttcatgccta tggctacgat tttgaatata ttgcccagaa atggaaaagc 480

aaaaaataca ttggctactg gcagagcgaa cacttttttc ataaacatat tctggacctg 540

aaagaatttt ttattccgaa aaatgtgagc gaacaggcaa atctgctggc agcaaaaatt 600

ctggaaagcc agagcagcct gagcattcat attcgtcgtg gcgattatat taaaaacaaa 660

accgcaaccc tgacacatgg tgtttgtagc ctggaatatt ataaaaaagc cctgaacaaa 720

atccgcgatc tggcaatgat tcgtgatgtg tttatcttta gcgacgatat cttctggtgc 780

aaagaaaata ttgaaaccct gctgagcaaa aaatataata tttattatag cgaagatctg 840

agccaagaag aggatctgtg gctgatgagc ctggcaaatc atcatattat tgccaatagc 900

agctttagtt ggtggggtgc atatctgggt agcagcgcaa gccagattgt tatttatccg 960

accccgtggt atgatattac cccgaaaaac acctatatcc cgattgtgaa ccattggatc 1020

aacgttgata aacatagcag ctgctaagcg gccgcgtcga cacgcaaaaa ggccatccgt 1080

caggatggcc ttctgcttaa tttgatgcct ggcagtttat ggcgggcgtc ctgcccgcca 1140

ccctccgggc cgttgcttcg caacgttcaa atccgctccc ggcggatttg tcctactcag 1200

gagagcgttc accgacaaac aacagataaa acgaaaggcc cagtctttcg actgagcctt 1260

tcgttttatt tgatgcctgg cagttcccta ctctcgcatg gggagacccc acactaccat 1320

catgtatgaa tatcctcctt agttcctatt ccgaagttcc tattctctag aaagtatagg 1380

aacttcggcg cgtcctacct gtgacacgcg tcaagatccc ctcacgctgc cgcaagcact 1440

cagggcgcaa gggctgctaa aggaagcgga acacgtagaa agccagtccg cagaaacggt 1500

gctgaccccg gatgaatgtc agctactggg ctatctggac aagggaaaac gcaagcgcaa 1560

agagaaagca ggtagcttgc agtgggctta catggcgata gctagactgg gcggttttat 1620

ggacagcaag cgaaccggaa ttgccagctg gggcgccctc tggtaaggtt gggaagccct 1680

gcaaagtaaa ctggatggct ttcttgccgc caaggatctg atggcgcagg ggatcaagat 1740

ctgatcaaga gacaggatga ggatcgtttc gcatgattga acaagatgga ttgcacgcag 1800

gttctccggc cgcttgggtg gagaggctat tcggctatga ctgggcacaa cagacaatcg 1860

gctgctctga tgccgccgtg ttccggctgt cagcgcaggg gcgcccggtt ctttttgtca 1920

agaccgacct gtccggtgcc ctgaatgaac tgcaggacga ggcagcgcgg ctatcgtggc 1980

tggccacgac gggcgttcct tgcgcagctg tgctcgacgt tgtcactgaa gcgggaaggg 2040

actggctgct attgggcgaa gtgccggggc aggatctcct gtcatctcac cttgctcctg 2100

ccgagaaagt atccatcatg gctgatgcaa tgcggcggct gcatacgctt gatccggcta 2160

cctgcccatt cgaccaccaa gcgaaacatc gcatcgagcg agcacgtact cggatggaag 2220

ccggtcttgt cgatcaggat gatctggacg aagagcatca ggggctcgcg ccagccgaac 2280

tgttcgccag gctcaaggcg cgcatgcccg acggcgagga tctcgtcgtg acccatggcg 2340

atgcctgctt gccgaatatc atggtggaaa atggccgctt ttctggattc atcgactgtg 2400

gccggctggg tgtggcggac cgctatcagg acatagcgtt ggctacccgt gatattgctg 2460

aagagcttgg cggcgaatgg gctgaccgct tcctcgtgct ttacggtatc gccgctcccg 2520

attcgcagcg catcgccttc tatcgccttc ttgacgagtt cttctgagcg ggactctggg 2580

gttcgaaatg accgaccaag cgacgcccaa cctgccatca cgagatttcg attccaccgc 2640

cgccttctat gaaaggttgg gcttcggaat cgttttccgg gacgccggct ggatgatcct 2700

ccagcgcggg gatctcatgc tggagttctt cgcccacccc agcttcaaaa gcgctctcgg 2760

taccggcagg gcggggcgta aggcgcgcca tttaaatgaa gttcctattc cgaagttcct 2820

attctctaga aagtatagga acttcgaagc agctccag 2858

<210> 16
<211> 2631
<212> DNA
<213> Artificial Sequence

<220>
<223> Integration cassette

<400> 16
ggccagatga ttaattccta atttttgttg acactctatc attgatagag ttattttacc 60

actccctatc agtgatagag aaaagtgaaa tgaatagttc gacaaaaatc tagaaataat 120

tttgtttaac tttaagaagg agatatacaa atgaagtcgg cactgacctt ttcccgtcgc 180

atcaatccgg tgtttctggc gttctttgtc gttgcttttc tgagcggtat cgcaggcgca 240

ctgcaggctc cgaccctgag tctgtttctg tccacggaag tgaaagttcg tccgctgtgg 300

gttggtctgt tctataccgt caacgcaatc gctggcatta cggttagctt tatcctggcg 360

aaacgttcag attcgcgcgg tgaccgtcgc aagctgatta tggtgtgcta tctgatggcg 420

gttggcaact gtctgctgtt tgccttcaat cgtgattacc tgaccctgat cacggcaggt 480

gtgctgctgg cgagcgttgc caacaccgca atgccgcaga ttttcgcgct ggcccgtgaa 540

tatgccgaca gctctgcacg cgaagtggtt atgtttagtt ccatcatgcg cgctcaactg 600

agtctggcat gggtgattgg tccgccgctg tcctttatgc tggcgctgaa ttacggtttt 660

accctgatgt tctcaatcgc ggccggcatt ttcgttctgt cggccctggt cgtgtggttt 720

atcctgccga gtgtcccgcg tgcagaaccg gttgtcgatg caccggtggt tgtccagggt 780

tcactgttcg cagacaaaaa cgttctgctg ctgtttatcg cgtcgatgct gatgtggacc 840

tgcaatacga tgtatattat cgatatgccg ctgtacatta ccgcaagcct gggtctgccg 900

gaacgtctgg ctggtctgct gatgggtacc gcagctggcc tggaaattcc gatcatgctg 960

ctggcgggtt attctgtgcg ttactttggc aaacgcaaga ttatgctgtt cgctgttctg 1020

gcgggtgtcc tgttttatac cggcctggtt ctgtttaaat tcaagacggc cctgatgctg 1080

ctgcagatct ttaacgcaat tttcatcggt attgtggctg gcattggtat gctgtacttc 1140

caagatctga tgccgggtcg tgcaggtgca gcaaccacgc tgtttaccaa tagcatctct 1200

acgggtgtca ttctggcagg cgtgctgcaa ggcggtctga ccgaaacgtg gggccatgac 1260

agcgtctatg tgatggcgat ggtcctgtct attctggccc tgattatctg tgcacgtgtg 1320

cgcgaagctt aaatcgatac tagcataacc ccttggggcc tctaaacgcg tcgacacgca 1380

aaaaggccat ccgtcaggat ggccttctgc ttaatttgat gcctggcagt ttatggcggg 1440

cgtcctgccc gccaccctcc gggccgttgc ttcgcaacgt tcaaatccgc tcccggcgga 1500

tttgtcctac tcaggagagc gttcaccgac aaacaacaga taaaacgaaa ggcccagtct 1560

ttcgactgag cctttcgttt tatttgatgc ctggcagttc cctactctcg catggggaga 1620

ccccacacta ccatcatgta tgaatatcct ccttagttcc tattccgaag ttcctattct 1680

ctagaaagta taggaacttc ggcgcgtcct acctgtgacg gaagatcact tcgcagaata 1740

aataaatcct ggtgtccctg ttgataccgg gaagccctgg gccaactttt ggcgaaaatg 1800

agacgttgat cggcacgtaa gaggttccaa ctttcaccat aatgaaataa gatcactacc 1860

gggcgtattt tttgagttgt cgagattttc aggagctaag gaagctaaaa tggagaaaaa 1920

aatcactgga tataccaccg ttgatatatc ccaatggcat cgtaaagaac attttgaggc 1980

atttcagtca gttgctcaat gtacctataa ccagaccgtt cagctggata ttacggcctt 2040

tttaaagacc gtaaagaaaa ataagcacaa gttttatccg gcctttattc acattcttgc 2100

ccgcctgatg aatgctcatc cggaattacg tatggcaatg aaagacggtg agctggtgat 2160

atgggatagt gttcaccctt gttacaccgt tttccatgag caaactgaaa cgttttcatc 2220

gctctggagt gaataccacg acgatttccg gcagtttcta cacatatatt cgcaagatgt 2280

ggcgtgttac ggtgaaaacc tggcctattt ccctaaaggg tttattgaga atatgttttt 2340

cgtctcagcc aatccctggg tgagtttcac cagttttgat ttaaacgtgg ccaatatgga 2400

caacttcttc gcccccgttt tcaccatggg caaatattat acgcaaggcg acaaggtgct 2460

gatgccgctg gcgattcagg ttcatcatgc cgtttgtgat ggcttccatg tcggcagatg 2520

cttaatgaat acaacagtac tgcgatgagt ggcagggcgg ggcgtaaggc gcgccattta 2580

aatgaagttc ctattccgaa gttcctattc tctagaaagt ataggaactt c 2631

<210> 17
<211> 4259
<212> DNA
<213> Artificial Sequence

<220>
<223> Integration cassette

<400> 17
ttactcagca ataaactgat attccgtcag gctggaatac tcttcgccag gacgcaggaa 60

gcagtccggt tgcggccatt cagggtggtt cgggctgtcc ggtagaaact cgctttccag 120

agccagccct tgccagtcgg cgtaaggttc ggttccccgc gacggtgtgc cgccgaggaa 180

gttgccggag tagaattgca gagccggagc ggtggtgtag accttcagct gcaatttttc 240

atctgctgac cagacatgcg ccgccacttt cttgccatcg cctttggcct gtaacaagaa 300

tgcgtgatcg taacctttca ctttgcgctg atcgtcgtcg gcaagaaact cactggcgat 360

gattttggcg ctgcggaaat caaaagacgt tccggcgaca gatttcaggc cgtcgtgcgg 420

aatgccgcct tcatcaaccg gcagatattc gtccgccaga atctgcaact tgtgattgcg 480

cacgtcagac tgctcgccgt caagattgaa atagacgtga ttagtcatat tcaccgggca 540

aggtttatca actgtggcgc gataagtaat ggagatacgg ttatcgtcgg tcagacgata 600

ttgcaccgtc gcgccgagat tacccgggaa gccctgatca ccatcatctg aactcagggc 660

aaacagcacc tgacgatcgt tctggttcac aatctgccag cgacgtttgt cgaacccttc 720

cggcccgccg tgcagctggt taacgccctg acttggcgaa agcgtcacgg tttcaccgtc 780

aaaggtataa cggctattgg cgatacggtt ggcataacga ccaatagagg cccccagaaa 840

cgcggcctga tcctgatagc attccgggct ggcacagccg agcagcgcct cgcggacgct 900

gccatcggaa agcggaatac gggcggaaag taaagtcgca ccccagtcca tcagcgtgac 960

taccatccct gcgttgttac gcaaagttaa cagtcggtac ggctgaccat cgggtgccag 1020

tgcgggagtt tcgttcagca ctgtcctgct ccttgtgatg gtttacaaac gtaaaaagtc 1080

tctttaatac ctgtttttgc ttcatattgt tcagcgacag cttgctgtac ggcaggcacc 1140

agctcttccg ggatcagcgc gacgatacag ccgccaaatc cgccgccggt catgcgtacg 1200

ccacctttgt cgccaatcac agctttgacg atttctacca gagtgtcaat ttgcggcacg 1260

gtgatttcga aatcatcgcg catagaggca tgagactccg ccatcaactc gcccatacgt 1320

ttcaggtcgc cttgctccag cgcgctggca gcttcaacgg tgcgggcgtt ttcagtcagt 1380

atatgacgca cgcgttttgc cacgatcggg tccagttcat gcgcaacagc gttgaactct 1440

tcaatggtga catcacgcag ggctggctgc tggaagaaac gcgcaccggt ttcgcactgt 1500

tcacgacggg tgttgtattc gctgccaacc agggtacgtt tgaagttact gttgatgatg 1560

acgacagcca cacctttggg catggaaact gctttggtcc ccagtgagcg gcaatcgatc 1620

agcaaggcat gatctttctt gccgagcgcg gaaattagct gatccatgat cccgcagtta 1680

cagcctacaa actggttttc tgcttcctga ccgttaagcg cgatttgtgc gccgtccagc 1740

ggcagatgat aaagctgctg caatacggtt ccgaccgcga cttccagtga agcggaagaa 1800

cttaacccgg caccctgcgg cacattgccg ctgatcacca tgtccacgcc gccgaagctg 1860

ttgttacgca gttgcagatg tttcaccacg ccacgaacgt agttagccca ttgatagttt 1920

tcatgtgcga caatgggcgc atcgagggaa aactcgtcga gctgattttc ataatcggct 1980

gccatcacgc gaactttacg gtcatcgcgt ggtgcacaac tgatcacggt ttgataatca 2040

atcgcgcagg gcagaacgaa accgtcgttg tagtcggtgt gttcaccaat caaattcacg 2100

cggccaggcg cctgaatggt gtgagtggca gggtagccaa atgcgttggc aaacagagat 2160

tgtgtttttt ctttcagact catttcttac actccggatt cgcgaaaatg gatatcgctg 2220

actgcgcgca aacgctctgc tgcctgttct gcggtcaggt ctcgctgggt ctctgccagc 2280

atttcataac caaccataaa tttacgtacg gtggcggagc gcagcagagg cggataaaag 2340

tgcgcgtgca gctgccagtg ttgattctct tcgccattaa atggcgcgcc gtgccagccc 2400

atagagtagg ggaaggagca ctggaagagg ttgtcataac gactggtcag ctttttcaac 2460

gccagcgcca gatcgctgcg ctgggcgtcg gtcaaatcgg tgatccgtaa aacgtgggct 2520

ttgggcagca gtagcgtttc gaacggccag gcagcccagt aaggcacgac ggctaaccag 2580

tgttcggttt cgacaacggt acggctaccg tctgccagct cgcgctgaac ataatccacc 2640

agcattggtg atttctgttc ggcaaaatat tctttttgca ggcggtcttc gcgctcagct 2700

tcgttaggca ggaagctatt tgcccaaatc tgaccgtgcg gatgcgggtt agagcagccc 2760

atcgccgcgc ctttgttttc aaaaacctgc acccatgggt acgttttccc cagttctgcg 2820

gtttgctcct gccaggtttt gacgatttcc gtcaatgctg caacgctgag ctctggcagc 2880

gttttactgt gatccggtga aaagcagatc acccggctgg tgccgcgcgc gctctggcaa 2940

cgcatcagcg gatcgtgact ttctggcgca tctggcgtgt cagacatcaa agccgcaaag 3000

tcattagtga aaacgtaagt cccggtgtaa tcggggtttt tatcgcctgt cacccgcaca 3060

ttacctgcgc agaggaagca atctggatcg tgcgcaggta acacctgttt ggctggcgtt 3120

tcctgcgccc cctgccaggg gcgcttagcg cggtgcggtg aaaccagaat ccattgcccg 3180

gtgagcgggt tgtagcggcg atgtggatga tcaacgggat taaattgcgt catggtcgtt 3240

ccttaatcgg gatatccctg tggatggcgt gactgccagt gccaggtgtc ctgcgccatt 3300

tcatcgagtg tgcgcgttac gcgccagttc agttcacggt cggctttgct ggcgtccgcc 3360

cagtaggccg gaaggtcgcc ctcgcgacgc ggtgcaaaat gataattaac cggtttgccg 3420

caggctttgc tgaaggcatt aaccacgtcc agcacgctgt tgcctacgcc agcgccgagg 3480

ttgtagatgt gtacgcctgg cttgttcgcc agtttttcca tcgccacgac gtgaccgtcc 3540

gccagatcca ttacgtggat gtaatcgcgt acgccagtac catcttcggt cggataatcg 3600

ttaccaaaaa tcgccagcga gtcgcgacgg cctacagcaa cctgggcgat gtatggcatc 3660

aggttattcg gaatgccttg cggatcttcg cccatatcgc ccgacggatg cgcgccaacc 3720

gggttgaagt agcgcagcag ggcaatgctc cagtccggct gggctttttg cagatcggtg 3780

aggatctgtt ccaccatcag cttgcttttg ccgtaagggc tttgcggtgt gccggtcggg 3840

aagctttcaa cgtatggaat tttgggctga tcgccataaa cggtggcgga ggagctaaaa 3900

ataaagtttt tgacgttagc ggcgcgcatg gcgctaatca ggcgcagagt gccgttgaca 3960

ttgttgtcgt aatattccag cggtttttgt accgattcgc ccacggcttt cagcccggcg 4020

aagtggatca cggtgtcgat agcgtgatcg tgcaggatct cggtcatcaa cgcttcgtta 4080

cgaatatcgc cttcaacaaa cgttggatgt ttgccgccta aacgctcgat aacaggcagt 4140

acgctgcgct tactgttaca gaggttatca agaatgatga catcatgacc gttttgcagt 4200

aattgcacac aggtatgact tccaatgtaa ccgctaccac cggtaaccag aactctcat 4259

<210> 18
<211> 4223
<212> DNA
<213> Artificial Sequence

<220>
<223> Integration cassette

<400> 18
tggccagatg attaattcct aatttttgtt gacactctat cattgataga gttattttac 60

cactccctat cagtgataga gaaaagtgaa atgaatagtt cgacaaaaat ctagaaataa 120

ttttgtttaa ctttaagaag gagatataca aatgcaaaaa ctactatctt taccgtccaa 180

tctggttcag tcttttcatg aactggagag ggtgaatcgt accgattggt tttgtacttc 240

cgacccggta ggtaagaaac ttggttccgg tggtggaaca tcctggctgc ttgaagaatg 300

ttataatgaa tattcagatg gtgctacttt tggagagtgg cttgaaaaag aaaaaagaat 360

tcttcttcat gcgggtgggc aaagccgtcg tttacccggc tatgcacctt ctggaaagat 420

tctcactccg gttcctgtgt tccggtggga gagagggcaa catctgggac aaaatctgct 480

ttctctgcaa cttcccctat atgaaaaaat catgtctttg gctccggata aactccatac 540

actgattgcg agtggtgatg tctatattcg ttcggagaaa cctttgcaga gtattcccga 600

agcggatgtg gtttgttatg gactgtgggt agatccgtct ctggctaccc atcatggcgt 660

gtttgcttcc gatcgcaaac atcccgaaca actcgacttt atgcttcaga agccttcgtt 720

ggcagaattg gaatctttat cgaagaccca tttgttcctg atggacatcg gtatatggct 780

tttgagtgac cgtgccgtag aaatcttgat gaaacgttct cataaagaaa gctctgaaga 840

actaaagtat tatgatcttt attccgattt tggattagct ttgggaactc atccccgtat 900

tgaagacgaa gaggtcaata cgctatccgt tgctattctg cctttgccgg gaggagagtt 960

ctatcattac gggaccagta aagaactgat ttcttcaact ctttccgtac agaataaggt 1020

ttacgatcag cgtcgtatca tgcaccgtaa agtaaagccc aatccggcta tgtttgtcca 1080

aaatgctgtc gtgcggatac ctctttgtgc cgagaatgct gatttatgga tcgagaacag 1140

tcatatcgga ccaaagtgga agattgcttc acgacatatt attaccgggg ttccggaaaa 1200

tgactggtca ttggctgtgc ctgccggagt gtgtgtagat gtggttccga tgggtgataa 1260

gggctttgtt gcccgtccat acggtctgga cgatgttttc aaaggagatt tgagagattc 1320

caaaacaacc ctgacgggta ttccttttgg tgaatggatg tccaaacgcg gtttgtcata 1380

tacagatttg aaaggacgta cggacgattt acaggcagtt tccgtattcc ctatggttaa 1440

ttctgtagaa gagttgggat tggtgttgag gtggatgttg tccgaacccg aactggagga 1500

aggaaagaat atctggttac gttccgaaca tttttctgcg gacgaaattt cggcaggtgc 1560

caatctgaag cgtttgtatg cacaacgtga agagttcaga aaaggaaact ggaaagcatt 1620

ggccgttaat catgaaaaaa gtgtttttta tcaacttgat ttggccgatg cagctgaaga 1680

ttttgtacgt cttggtttgg atatgcctga attattgcct gaggatgctc tgcagatgtc 1740

acgcatccat aaccggatgt tgcgtgcgcg tattttgaaa ttagacggga aagattatcg 1800

tccggaagaa caggctgctt ttgatttgct tcgtgacggc ttgctggacg ggatcagtaa 1860

tcgtaagagt accccaaaat tggatgtata ttccgatcag attgtttggg gacgtagccc 1920

cgtgcgcatc gatatggcag gtggatggac cgatactcct ccttattcac tttattcggg 1980

aggaaatgtg gtgaatctag ccattgagtt gaacggacaa cctcccttac aggtctatgt 2040

gaagccgtgt aaagacttcc atatcgtcct gcgttctatc gatatgggtg ctatggaaat 2100

agtatctacg tttgatgaat tgcaagatta taagaagatc ggttcacctt tctctattcc 2160

gaaagccgct ctgtcattgg caggctttgc acctgcgttt tctgctgtat cttatgcttc 2220

attagaggaa cagcttaaag atttcggtgc aggtattgaa gtgactttat tggctgctat 2280

tcctgccggt tccggtttgg gcaccagttc cattctggct tctaccgtac ttggtgccat 2340

taacgatttc tgtggtttag cctgggataa aaatgagatt tgtcaacgta ctcttgttct 2400

tgaacaattg ctgactaccg gaggtggatg gcaggatcag tatggaggtg tgttgcaggg 2460

tgtgaagctt cttcagaccg aggccggctt tgctcaaagt ccattggtgc gttggctacc 2520

cgatcattta tttacgcatc ctgaatacaa agactgtcac ttgctttatt ataccggtat 2580

aactcgtacg gcaaaaggga tcttggcaga aatagtcagt tccatgttcc tcaattcatc 2640

gttgcatctc aatttacttt cggaaatgaa ggcgcatgca ttggatatga atgaagctat 2700

acagcgtgga agttttgttg agtttggccg tttggtagga aaaacctggg aacaaaacaa 2760

agcattggat agcggaacaa atcctccggc tgtggaggca attatcgatc tgataaaaga 2820

ttataccttg ggatataaat tgccgggagc cggtggtggc gggtacttat atatggtagc 2880

gaaagatccg caagctgctg ttcgtattcg taagatactg acagaaaacg ctccgaatcc 2940

gcgggcacgt tttgtcgaaa tgacgttatc tgataaggga ttccaagtat cacgatcata 3000

actgaaacca atttgcctgg cggcagtagc gcggtggtcc cacctgaccc catgccgaac 3060

tcagaagtga aacgccgtag cgccgatggt agtgtggggt ctccccatgc gagagtaggg 3120

aactgccagg catcaaataa aacgaaaggc tcagtcgaaa gactgggcct ttcgggatcc 3180

aggccggcct gttaagacgg ccagtgaatt cgagctcggt acctaccgtt cgtataatgt 3240

atgctatacg aagttatcga gctctagaga atgatcccct cattaggcca cacgttcaag 3300

tgcagcgcac accgtggaaa cggatgaagg cacgaaccca gttgacataa gcctgttcgg 3360

ttcgtaaact gtaatgcaag tagcgtatgc gctcacgcaa ctggtccaga accttgaccg 3420

aacgcagcgg tggtaacggc gcagtggcgg ttttcatggc ttgttatgac tgtttttttg 3480

tacagtctat gcctcgggca tccaagcagc aagcgcgtta cgccgtgggt cgatgtttga 3540

tgttatggag cagcaacgat gttacgcagc agcaacgatg ttacgcagca gggcagtcgc 3600

cctaaaacaa agttaggtgg ctcaagtatg ggcatcattc gcacatgtag gctcggccct 3660

gaccaagtca aatccatgcg ggctgctctt gatcttttcg gtcgtgagtt cggagacgta 3720

gccacctact cccaacatca gccggactcc gattacctcg ggaacttgct ccgtagtaag 3780

acattcatcg cgcttgctgc cttcgaccaa gaagcggttg ttggcgctct cgcggcttac 3840

gttctgccca ggtttgagca gccgcgtagt gagatctata tctatgatct cgcagtctcc 3900

ggcgagcacc ggaggcaggg cattgccacc gcgctcatca atctcctcaa gcatgaggcc 3960

aacgcgcttg gtgcttatgt gatctacgtg caagcagatt acggtgacga tcccgcagtg 4020

gctctctata caaagttggg catacgggaa gaagtgatgc actttgatat cgacccaagt 4080

accgccacct aacaattcgt tcaagccgag atcgtagaat ttcgacgacc tgcagccaag 4140

cataacttcg tataatgtat gctatacgaa cggtaggatc ctctagagtc gacctgcagg 4200

catgagatgt gtataagaga cag 4223

<210> 19
<211> 3792
<212> DNA
<213> Artificial Sequence

<220>
<223> Integration cassette

<400> 19
gggaattgat tctggtacca aatgagtcga ccggccagat gattaattcc taatttttgt 60

tgacactcta tcattgatag agttatttta ccactcccta tcagtgatag agaaaagtga 120

aatgaatagt tcgacaaaaa tctagaaata attttgttta actttaagaa ggagatatac 180

aaatgattac ccgcaaaagg cgggccagga caatccatag ccgatatcca atcggaattt 240

acgggagcat agtaatgaca gatattgcac agttgcttgg caaagacgcc gacaaccttt 300

tacagcaccg ttgtatgact attccttctg accagcttta tctccccgga catgactacg 360

tagaccgcgt gatgattgac aataatcgcc cgccagcggt gttacgtaat atgcagacgt 420

tgtacaacac tgggcgtctg gctggcacag gatatctttc tattctgccg gttgaccagg 480

gcgttgagca ctctgccgga gcttcatttg ctgctaaccc gctctacttt gacccgaaaa 540

acattgttga actggcgatc gaagcgggct gtaactgtgt ggcatcaact tacggcgtgt 600

tggcgtcggt atcgcggcgc tatgcgcatc gcattccatt cctcgtcaaa cttaatcaca 660

acgagacgct aagttacccg aacacctacg atcaaacgct gtatgccagc gtggagcagg 720

ccttcaacat gggcgcggtg gcggttggtg cgactatcta ttttggttcg gaagagtcac 780

gtcgccagat tgaagaaatt tctgcggctt ttgaacgtgc gcacgagctg ggcatggtga 840

cagtgctgtg ggcctatttg cgtaactccg cctttaagaa agatggcgtt gattaccatg 900

tttccgccga cctgaccggt caggcaaacc atctggcggc gaccataggt gcagatatcg 960

tcaaacaaaa aatggcggaa aataacggcg gctataaagc aattaattac ggttataccg 1020

acgatcgcgt gtacagcaag ttaaccagcg aaaacccgat tgatctggtg cgttatcagt 1080

tagctaactg ctatatgggc cgggccgggt tgataaactc cggcggtgct gcaggcggtg 1140

aaactgacct cagcgatgca gtgcgtactg cggttatcaa caaacgcgct ggcggaatgg 1200

ggctgattct tggacgtaag gcgttcaaga aatcgatggc tgacggcgtg aaactgatta 1260

acgccgtgca ggatgtttat ctcgatagca aaattactat cgcctaagag gatcgagatc 1320

tcgatcccgc gaaattaata cgactcacta taggggaatt gtgagcggat aacaattccc 1380

ctctagaaat aattttgttt aactttaaga aggagatata ccatgggcca tcatcatcat 1440

catcatcatc atcatcacag cagcggccat atcgaaggtc gtcatatggc ggtgaaagaa 1500

gcgaccagcg agaccaagaa gcgtagcggt tacgagatca ttaccctgac cagctggctg 1560

ctgcaacaag aacagaaggg tatcattgac gcggaactga ccatcgttct gagcagcatt 1620

agcatggcgt gcaaacagat cgcgagcctg gtgcaacgtg cgaacattag caacctgacc 1680

ggtacccaag gcgcggttaa catccagggt gaagaccaaa agaaactgga tgttattagc 1740

aacgaggtgt tcagcaactg cctgcgtagc agcggtcgta ccggcatcat tgcgagcgag 1800

gaagaggacg tggcggttgc ggtggaagag agctacagcg gtaactatat cgtggttttt 1860

gacccgctgg atggcagcag caacctggat gcggctgtga gcaccggtag catcttcggc 1920

atttacagcc cgaacgacga gagcctgccg gattttggtg acgatagcga cgataacacc 1980

ctgggcaccg aagagcaacg ttgcatcgtt aacgtgtgcc aaccgggtag caacctgctg 2040

gcggcgggct actgcatgta tagcagcagc gttgcgttcg tgctgaccat tggcaagggc 2100

gttttcgtgt ttaccctgga cccgctgtac ggtgaattcg tgctgaccca ggagaacctg 2160

caaatcccga agagcggtga aatttacagc tttaacgagg gcaactataa actgtgggat 2220

gaaaacctga agaaatatat cgacgatctg aaggaaccgg gtccgagcgg taaaccgtac 2280

agcgcgcgtt atatcggtag cctggttggc gacttccacc gtaccctgct gtacggtggc 2340

atttacggtt atccgcgtga taagaaaagc aagaacggca aactgcgtct gctgtatgaa 2400

tgcgcgccga tgagctttat tgttgagcag gcgggtggca aaggtagcga cggccaccag 2460

cgtgtgctgg atatccaacc gaccgaaatt caccagcgtg ttccgctgta cattggtagc 2520

accgaagagg ttgaaaaagt tgaaaagtat ctggcgtaat cgagtctggt aaagaaaccg 2580

ctgctgcgaa atttgaacgc cagcacatgg actcgtctac tagcgcagct taattaacct 2640

aggctgctgc caccgctgag caataactag cataacccct tggggcctct aaacgggtct 2700

tgaggggttt tttgctgaaa ggaggaacta tatccggatt ggcgaatggg acgcgccctg 2760

tagcggcgca ttaagcgcgg cgggtggacg gccagtgaat tcgagctcgg tacctaccgt 2820

tcgtataatg tatgctatac gaagttatcg agctctagag aatgatcccc tcattaggcc 2880

acacgttcaa gtgcagcgca caccgtggaa acggatgaag gcacgaaccc agttgacata 2940

agcctgttcg gttcgtaaac tgtaatgcaa gtagcgtatg cgctcacgca actggtccag 3000

aaccttgacc gaacgcagcg gtggtaacgg cgcagtggcg gttttcatgg cttgttatga 3060

ctgttttttt gtacagtcta tgcctcgggc atccaagcag caagcgcgtt acgccgtggg 3120

tcgatgtttg atgttatgga gcagcaacga tgttacgcag cagcaacgat gttacgcagc 3180

agggcagtcg ccctaaaaca aagttaggtg gctcaagtat gggcatcatt cgcacatgta 3240

ggctcggccc tgaccaagtc aaatccatgc gggctgctct tgatcttttc ggtcgtgagt 3300

tcggagacgt agccacctac tcccaacatc agccggactc cgattacctc gggaacttgc 3360

tccgtagtaa gacattcatc gcgcttgctg ccttcgacca agaagcggtt gttggcgctc 3420

tcgcggctta cgttctgccc aggtttgagc agccgcgtag tgagatctat atctatgatc 3480

tcgcagtctc cggcgagcac cggaggcagg gcattgccac cgcgctcatc aatctcctca 3540

agcatgaggc caacgcgctt ggtgcttatg tgatctacgt gcaagcagat tacggtgacg 3600

atcccgcagt ggctctctat acaaagttgg gcatacggga agaagtgatg cactttgata 3660

tcgacccaag taccgccacc taacaattcg ttcaagccga gatcgtagaa tttcgacgac 3720

ctgcagccaa gcataacttc gtataatgta tgctatacga acggtaggat cctctagagt 3780

cgacctgcag gc 3792

<210> 20
<211> 5917
<212> DNA
<213> Artificial Sequence

<220>
<223> Transposon cassette

<400> 20
acaggttggc tgataagtcc ccggtctagc ttgcatgcag attgcagcat tacacgtctt 60

gatttgacgg ctagctcagt cctaggtaca gtgctagcac tgctttgtgg aaggagatag 120

acttatggcg gatccgatgg aatacctcga tgtgtcgttc ggcggcacgt tcgctgcaga 180

cacctacacc acaggtggcg acgaggtggc gaagggcccc gtgaccaagc acggcagcat 240

accgaccaag cttgacggcg gcggcatcac cctcgctggc ggcaccaacg gcgtgacatt 300

cacctcgacc gcgagcttca gcgagagtgg gaaggtgaac aagggattcc gcgccgaaat 360

ggagtaccgt acgacgcaga cgcccagcaa cctcgccaca ttgttctccg ccatgggcaa 420

catcttcgtg cgggcgaacg gcagcaacct cgaatacggc ttctccacga acccttccgg 480

cagtacatgg aacgactaca caaagtccgt gacgctgcct tccaacaatg tgaagcacat 540

catccagctg acatatctgc cgggagccga cggcgctgcc tcgacgttgc agttgtcggt 600

ggatggcgtg gccggcgaga ccgccacctc cgcggccggc gagctcgcgg ccgtcagcga 660

ttccgtcggg aacaagttcg ggatcggcta cgaggtgaac cccgcttccg gcgcggcgag 720

ccgcggtctt gccggtgacg tgttccgcgc gcgtgtcgcc gattcggacg ccccgtggga 780

gattcttgac gcatcccagc tgctgcatgt caatttcaac ggcacgttca gcggcacctc 840

atataccgcg gcgagcggcg agcagatgct gggctcgctg gtgtcgcgct cggccaatcc 900

gtccatctcg aactccgccg tcacgctggg cggcggcacg gccggattcg atttcacgcc 960

cacggacttc accctcggtg acaacgaggc catcacccgc ccgctggtcg cggagctgcg 1020

cttcaccccg acgcagaccg gcgacaacca gaccctgttc ggcgcgggcg gcaacctgtt 1080

cctgcgctac gagtcgaaca agctcgtgtt cggcgcctcc accaagtccg gcgataattg 1140

gaccgaccac aagatcgagt ccgcggccgc cacgggtgcg gagcacgtcg tgtcggtggc 1200

gtacgtgccc aataaggccg gcaccggcgc gaagcttgtc atgcgcgtgg atggcggcga 1260

cgcccagacc aaggacatca ctggtctggc ttacctgaat tcgagcatca agggcaaggt 1320

cggcttcggc aacgacgtgc ataccgacgc gctcagccgc ggcttcgtcg gctcgctgag 1380

cgagatccgc ctggccgaaa cctccgcgaa cttcaccacc aacgaattca agctggtcta 1440

ctctcaggtc agctgcgaca cgtcgggcat caaggaggcg aataccttcg acgtggagcc 1500

cgccgagtgc gaggccgcgc ttaagaccaa gctgtccaag ctgcgtccga ccgaagggca 1560

ggccgactac atcgactggg gtcagatcgg attcctccat tacggcatca acacgtacta 1620

caaccaggag tggggtcacg gtaacgagga tccctcccgc atcaacccga ccggcctcga 1680

caccgaccag tgggcgaagt ccttcgccga cggtggcttc aagatgatca tggtgacggt 1740

caagcaccat gacggtttcg agctgtacga ctcgcggtac aacaccgagc acgactgggc 1800

aaacaccgcc gtcgccaagc gcacggggga gaaggacctg ttccgcaaga ttgtcgcctc 1860

ggcgaagaaa tacggcctga aggtcggcat ctactattcg ccggccgatt cctacatgga 1920

gaggaagggc gtctggggca acaactccgc acgcgtcgag cgcacgatcc ccacgctggt 1980

ggagaacgac gaccgcgccg gcaaggtggc ttccggcaaa ctgcccacgt tcaagtacaa 2040

ggccacggat tacggcgcct acatgctcaa ccagctctat gagctgctga ctgagtacgg 2100

cgacatctcc gaggtctggt tcgacggtgc ccaaggcaac accgcaggca ctgagcatta 2160

cgactatggc gtgttctacg agatgatccg ccggcttcag ccccaggcaa ttcaggccaa 2220

cgccgcatac gatgcccgat gggtgggcaa cgaggacggc tgggcccgtc agaccgagtg 2280

gagcccgcag gcggcataca acgacggcgt ggacaaggtg tcgctcaagc ctggccagat 2340

ggcccccgac ggtaagcttg gcagcatgtc gagcgtgctg tccgagatcc gcagcggcgc 2400

cgccaaccag ctgcactggt atccggccga agtcgacgcc aagaaccggc ccggatggtt 2460

ctaccgtgcc agccaatcgc cggcgtccgt agccgaagtc gtgaagtact acgagcagtc 2520

cacgggacgc aactcgcagt atctgctgaa cgtcccaccg tccgataccg gcaagctcgc 2580

cgatgcggat gccgcgggac ttaaggggct gggcgaggag ctcgcccgac gctacggcac 2640

cgatcttgcc ctgggcaaga gcgcgaccgt cgccgcgtcc gcgaacgaca ctgcggtagc 2700

ggccccgaag ctgaccgacg gttcgaagct ctcctccgac aaggccgtgg gcaatacgcc 2760

gacgtacacc atcgatctgg gcagcactgt cgccgtggat gcagtgaaga tctccgagga 2820

cgtgcgcaat gccggccagc agatcgaaag cgccactctg cagggacgag tcaatggaac 2880

atggacgaat ctggcgacta tgacgacggt cgggcagcag cgcgaccttc gcttcacgtc 2940

ccagaacatc gatgccatcc gtctggtggt caactcctcc cgcggtccgg tgcgtctgag 3000

ccgtcttgag gtgttccaca ccgaatccga gattcagacc ggcgcccgcg cctactacat 3060

cgatccgacg gcgcagaccg cgggagatgg attcacgaag gacaagccca tgacgtcgat 3120

cgagcagctg cacgatgtga ccgtcgcgcc aggctccgtg atcttcgtca aggcgggcac 3180

cgagctgacc ggggacttcg ccgtcttcgg ctacggcacc aaggacgagc ccatcaccgt 3240

gacgacatac ggcgaaagcg acaaagccac caccgcgagc ttcgacggca tgaccgccgg 3300

gctgacgctg aagcaggcgc tgaaggcgct cggcaaggac gacgccggct gggtcgtggc 3360

cgattccgcc actgcaccgg cctcccgcgt gtatgtcccg caggatgaga tcagcgtgca 3420

cgcccagtcg tcgcagaact ccggcgcaga ggcggcgagg gcgctcgacg gcgactcgtc 3480

gacgagctgg cactcccagt acagcccgac caccgcgtct gctccgcatt gggtgactct 3540

cgatctcggc aaatcgcgtg agaacgtcgc ctacttcgac tacctcgccc gtatcgacgg 3600

caacaataac ggtgccgcca aggattacga ggtgtatgtc tccgacgatc ccaacgattt 3660

tggagcccct gtggcctcgg gcacgttgaa gaacgtcgcc tacacgcagc gcatcaagct 3720

gacccccaag aacggacggt acgtcaagtt cgtcatcaag accgattatt ccggatcgaa 3780

cttcggctcc gcggcggaaa tgaatgtcga gttgctgccc acggccgtag aggaggacaa 3840

ggtcgccacc ccgcagaagc cgacagtgga cgatgatgcc gatacataca ccatccccga 3900

catcgaggga gtcgtgtaca aggtcgacgg caaggtgttg gccgctggtt ccgtagtgaa 3960

cgtgggcgat gaggacgtga ccgtcacggt caccgccgag cccgccgacg gataccgctt 4020

cccggatggt gtgacgtccc cagtcacgta tgagctgacg ttcaccaaga agggtggcga 4080

gaagcctccg accgaagtca acaaggacaa gctgcacgcc acgatcacca aggctcaggc 4140

gatcgaccgt tccgcctata cggacgagtc gctcaaggtg cttgatgaca agctcgccgc 4200

agcgctcaag gtctatgacg atgacaaggt gagccaggat gatgtcgatg ccgccgaggc 4260

ggctctgtct gcggcgatcg acgcgctgaa gaccaagccg acgacccccg gcggtgaagg 4320

tgagaagcct ggtgaaggtg aaaagcccgg tgacggcaac aagcccggtg acggcaagaa 4380

gcccggcgac gtgatcgcaa agaccggcgc ctccacaatg taactagcat aaccccttgg 4440

ggcctctaaa cgggtcttga ggggtttttt gctgaaacca atttgcctgg cggcagtagc 4500

gcggtggtcc cacctgaccc catgccgaac tcagaagtga aacgccgtag cgccgatggt 4560

agtgtggggt ctccccatgc gagagtaggg aactgccagg catcaaataa aacgaaaggc 4620

tcagtcgaaa gactgggcct ttcgggatcc aggccggcct gttaacgaat taatcttccg 4680

cggcggtatc gataagcttg atatcgaatt ccgaagttcc tattctctag acgccattca 4740

ggctgcgcaa ctgttgggaa gggcgatcgg tgcgggcctc ttcgctatta cgccagctgg 4800

cgaaaggggg atgtgctgca aggcgattaa gttgggtaac gccagggttt tcccagtcac 4860

gacgttgtaa aacgacggcc agtgaattcg agctcggtac ctaccgttcg tataatgtat 4920

gctatacgaa gttatcgagc tctagagaat gatcccctca ttaggccaca cgttcaagtg 4980

cagcgcacac cgtggaaacg gatgaaggca cgaacccagt tgacataagc ctgttcggtt 5040

cgtaaactgt aatgcaagta gcgtatgcgc tcacgcaact ggtccagaac cttgaccgaa 5100

cgcagcggtg gtaacggcgc agtggcggtt ttcatggctt gttatgactg tttttttgta 5160

cagtctatgc ctcgggcatc caagcagcaa gcgcgttacg ccgtgggtcg atgtttgatg 5220

ttatggagca gcaacgatgt tacgcagcag caacgatgtt acgcagcagg gcagtcgccc 5280

taaaacaaag ttaggtggct caagtatggg catcattcgc acatgtaggc tcggccctga 5340

ccaagtcaaa tccatgcggg ctgctcttga tcttttcggt cgtgagttcg gagacgtagc 5400

cacctactcc caacatcagc cggactccga ttacctcggg aacttgctcc gtagtaagac 5460

attcatcgcg cttgctgcct tcgaccaaga agcggttgtt ggcgctctcg cggcttacgt 5520

tctgcccaga tttgagcagc cgcgtagtga gatctatatc tatgatctcg cagtctccgg 5580

cgagcaccgg aggcagggca ttgccaccgc gctcatcaat ctcctcaagc atgaggccaa 5640

cgcgcttggt gcttatgtga tctacgtgca agcagattac ggtgacgatc ccgcagtggc 5700

tctctataca aagttgggca tacgggaaga agtgatgcac tttgatatcg acccaagtac 5760

cgccacctaa caattcgttc aagccgagat cgtagaattt cgacgacctg cagccaagca 5820

taacttcgta taatgtatgc tatacgaacg gtaggatcct ctagagtcga ccaggtggca 5880

cttttcgggc agaccgggga cttatcagcc aacctgt 5917

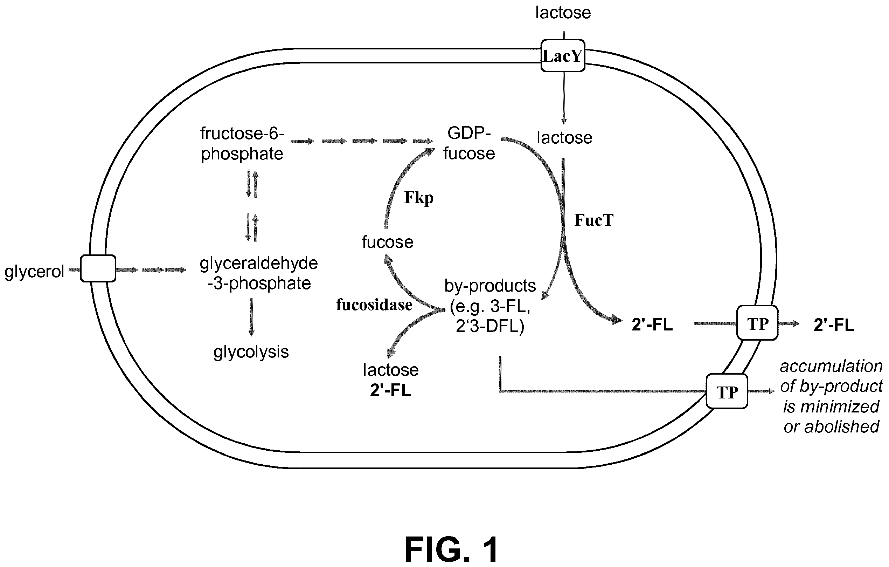

## Claims

1. A method for the production of a desired oligosaccharide using a genetically-engineered microbial host cell, the method comprises the steps of:
(i) providing a genetically-engineered microbial host cell that is able to produce the desired oligosaccharide, wherein the microbial host cell has been genetically engineered to express at least one heterologous glycosidase which is able to intracellularly degrade metabolic saccharide by-products that are generated during the intracellular biosynthesis of the desired oligosaccharide, and wherein the microbial host cell is able to recycle the degradation products resulting from the enzymatic activity of said glycosidase for the production of the desired oligosaccharide;
(ii) cultivating the genetically-engineered microbial host cell under conditions and in a medium permissive for the production of the desired oligosaccharide, thereby producing the desired oligosaccharide; and
(iii) optionally, recovering the desired oligosaccharide.

2. A genetically-engineered microbial host cell for producing a desired oligosaccharide, wherein said microbial host cell is
a) able to produce the desired oligosaccharide;
b) has been genetically engineered to express at least one heterologous glycosidase which is able to intracellularly degrade metabolic saccharide by-products that are generated during the intracellular biosynthesis of the desired oligosaccharide; and
c) is able to recycle degradation products resulting from the enzymatic activity of said glycosidase for the production of the desired oligosaccharide.

3. The method according to claim 1 or the genetically-engineered microbial host cell according to claim 2, wherein the heterologous glycosidase is selected from the group consisting of fucosidases, sialidases, hexosaminidases, galactosidases and glucosidases.

4. The method or the genetically-engineered microbial host cell according to any one of claims 1 to 3, wherein the heterologous glycosidase is selected from the group consisting of α-1,2-fucosidases, α-1,3-fucosidases, α-2,3-sialidases, α-2,6-sialidases, α-2,8-sialidases, β-1,3-galactosidases, β-1,4-galactosidases, β-1,6-galactosidases, β-*N*-acetylhexosaminidases and β-1,3-glucosidases.

5. The method or the genetically-engineered microbial host cell according to any one of claims 1 to 4, wherein the genetically-engineered microbial host cell has been genetically engineered to express a heterologous glycosyltransferase, preferably as glycosyltransferase selected from the group consisting of fucosyltransferases, sialyltransferases, galactosyltransferases, N-acetylglucosaminyltransferases and glucosyltransferases.

6. The method or the genetically-engineered microbial host cell according to any one of claims 1 to 5, wherein the microbial host cell has been genetically engineered to express a heterologous α-1,3-fucosyltransferase and a heterologous α-1,2-fucosidase.

7. The method or the genetically-engineered microbial host cell according to any one of claims 1 to 5, wherein the microbial host cell has been genetically engineered to express a heterologous α-1,2-fucosyltransferase and a heterologous α-1,3-fucosidase.

8. The method or the genetically-engineered microbial host cell according to any one of claims 1 to 5, wherein the microbial host cell has been genetically engineered to express a heterologous β-1,3-N-acetylglucosaminyltransferase, a heterologous α-1,2-fucosyltransferase, a heterologous β-1,3-galactosyltransferase and a heterologous α-1,3-fucosidase.

9. The method or the genetically-engineered microbial host cell according to any one of claims 1 to 5, wherein the microbial host cell has been genetically engineered to express a heterologous α-2,6-sialyltransferase and a heterologous α-2,3-sialidase.

10. The method or the genetically-engineered microbial host cell according to any one of claims 1 to 5, wherein the microbial host cell has been genetically engineered to express a heterologous β-1,3-*N*-acetylglucosaminyltransferase, a heterologous β-1,4-galactosyltransferase and a heterologous β-1,3-galactosidase and/or β-1,3-glucosidase and/or galactan-β-1,3-galactosidase.

11. The method or the genetically-engineered microbial host cell according to any one of claims 1 to 5, wherein the microbial host cell has been genetically engineered to express a heterologous β-1,3-*N*-acetylglucosaminyltransferase, a heterologous β-1,3-galactosyltransferase and a heterologous β-1,3-glucosidase and/or a galactan-β-1,3-galactosidase.

12. The method or the genetically-engineered microbial host cell according to any one of claims 1 to 11, wherein the desired oligosaccharide is a human milk oligosaccharide, preferably a human milk oligosaccharide selected from the group consisting of 2'-fucosyllactose (2'-FL), 3-fucosyllactose (3-FL), 2',3-difucosyllactose, lacto-*N*-triose II, lacto-*N*-tetraose, lacto-*N*-*neo*tetraose, lacto-*N*-fucopentaose I, lacto-*N*-*neo*fucopentaose I, lacto-*N*-fucopentaose II, lacto-*N*-fucopentaose III, lacto-*N*-fucopentaose V, lacto-*N*-*neo*fucopentaose V, lacto-*N*-difucohexaose I, lacto-*N*-difucosylhexaose II, *para*-Lacto-N-fucosylhexaose, fucosyl-lacto-*N*-sialylpentaose b, fucosyl-lacto-*N*-sialylpentaose c, fucosyl-lacto-*N*-sialylpentaose c, disialyl-lacto-*N*-fucopentaose, 3-fucosyl-3'-sialyllactose, 3-fucosyl-6'-sialyllactose, lacto-*N*-*neo*difucohexaose I, 3'-sialyllactose, 6'-sialyllactose, sialyllacto-*N*-tetraose a (LST-a), sialyllacto-*N-*tetraose b (LST-b), sialyllacto-*N*-tetraose c (LST-c), and disialyllacto-*N-*tetraose.

13. Use of the genetically-engineered microbial host cell according to any one of claims 2 to 12 for the production of a desired oligosaccharide, preferably an oligosaccharide selected from the group of HMOs.

14. An oligosaccharide, preferably an oligosaccharide selected from the group of HMOs, produced by the method or by the use of a genetically-engineered microbial host cell according to any one of claims 1 to 12 for the manufacture of a nutritional composition.

15. A nutritional composition containing at least one oligosaccharide that has been produced by the method or by the use of a genetically-engineered microbial host cell according to any one of claims 1 to 12, wherein the at least one oligosaccharide is preferably a HMO.
